# EUROPEAN PATENT SPECIFICATION

(11) **EP 1 957 538 B1**
(45) Date of publication and mention of the grant of the patent: **22.05.2013**
(21) Application number: 06836747.3
(22) Date of filing: 01.11.2006
(51) Int. Cl.: C07K 16/28

(54) **USES OF ANTI-CD40 ANTIBODIES**
ANWENDUNG VON ANTI-CD40-ANTIKÖRPERN
UTILISATIONS D'ANTICORPS ANTI-CD40

(30) Priority: 01.11.2005 US 732580 P
(43) Date of publication of application: 20.08.2008
(73) Proprietor: Novartis AG, 4056 Basel (CH); XOMA Technology Ltd., Berkeley CA 94701 (US)
(72) Inventor: AUKERMAN, Sharon, Lea, Emeryville, CA 94662-8097 (US); LUQMAN, Mohammad, Emeryville, CA 94662-8097 (US)
(74) Representative: Marshall, Cameron John
(86) International application number: PCT/US2006/042601
(87) International publication number: WO 2007/053661

(56) References cited:
- WO-A-03/035904
- LAW C-L ET AL: "Preclinical antitumor activity of a humanized anti-CD40 monoclonal antibody SGN-40" BIOSIS, 15 September 2005 (2005-09-15), XP002310999
- LONG LI ET AL: "Antagonist anti-CD40 monoclonal antibody, CHIR-12.12, inhibits growth of a rituximab-resistant NHL xenograft model and achieves synergistic activity when combined with ineffective rituximab" BIOSIS, 16 November 2004 (2004-11-16), XP002427479
- TAI YU-TZU ET AL: "Human anti-CD40 antagonist antibody triggers significant antitumor activity against human multiple myeloma" CANCER RESEARCH, AMERICAN ASSOCIATION FOR CANCER RESEARCH, BALTIMORE, MD, US, vol. 65, no. 13, July 2005 (2005-07), pages 5898-5906, XP002427470 ISSN: 0008-5472
- DALL'OZZO SEBASTIEN ET AL: "Rituximab-dependent cytotoxicity by natural killer cells: Influence of FCGR3A polymorphism on the concentration-effect relationship" CANCER RESEARCH, vol. 64, no. 13, 1 July 2004 (2004-07-01), pages 4664-4669, XP002433841 ISSN: 0008-5472 cited in the application
- CARTRON G ET AL: "Therapeutic activity of humanized anti-CD20 monoclonal antibody and polymorphism in IgG Fc receptor FcgammaRIIIa gene" BLOOD, W.B.SAUNDERS COMPANY, ORLANDO, FL, US, vol. 99, no. 3, 1 February 2002 (2002-02-01), pages 754-758, XP002404674 ISSN: 0006-4971
- SIBILIA J ET AL: "IMMUNOTHERAPY OF INFLAMMATORY DISEASES, 2005" EXPERT OPINION ON BIOLOGICAL THERAPY, ASHLEY, LONDON, GB, vol. 5, no. SUPPL 1, September 2005 (2005-09), pages S01-S13, XP009060332 ISSN: 1471-2598

## Description

This invention relates to new uses of anti-CD40 antibodies, in particular in the treatment of inflammatory diseases and autoimmune diseases that are associated with CD40-expressing cells.

### BACKGROUND OF THE INVENTION

Many members of the tumor necrosis factor (TNF) family of ligands and their corresponding receptors regulate growth of normal cells by inducing apoptosis or enhancing cell survival and proliferation. It is this balance between apoptotic signals and survival and proliferation signals that maintains normal cellular homeostasis. At least 26 TNF family receptors and 18 TNF family ligands have been identified to date. The biologically active forms of both the receptors and ligands are self-assembled protein trimers. Transmembrane and soluble forms of both the receptors and ligands have been identified. Though the intracellular domains of the receptors share no sequence homology, their extracellular domains comprise 40-amino-acid, cysteine-rich repeats. Their cytoplasmic tails signal by interacting with two major groups of intracellular proteins: TNF receptor-associated factors (TRAFs) and death domain (DD)-containing proteins. Interaction between at least six human TRAFs and TRAF-binding sites on the cytoplasmic tail of some of these receptors initiates several signaling pathways, including AKT (the serine/threonine kinase referred to as protein kinase B or PKB), nuclear factor-κB (NF-κB), and mitogen-activated protein kinases (MAPK). See, for example, the review by Younes and Kadin (2003) J. Clin. Oncol. 18:3526-3534.

The TNF family receptor member CD40 is a 50-55 kDa cell-surface antigen present on the surface of both normal and neoplastic human B cells, dendritic cells, monocytes, macrophages, CD8⁺ T cells, endothelial cells, and monocytic and epithelial cells. The CD40 antigen is also expressed on activated T cells, activated platelets, inflamed vascular smooth muscle cells, eosinophils, synovial membranes in rheumatoid arthritis, dermal fibroblasts, and other non-lymphoid cell types. Depending on the type of cell expressing CD40, ligation can induce intercellular adhesion, differentiation, activation, and proliferation. For example, binding of CD40 to its cognate ligand, CD40L (also designated CD154), stimulates B-cell proliferation and differentiation into plasma cells, antibody production, isotype switching, and B-cell memory generation. During B-cell differentiation, CD40 is expressed on pre-B cells but lost upon differentiation into plasma cells.

The CD40 ligand (CD40L), also known as CD 154, is a 32-33 kDa transmembrane protein that also exists in two smaller biologically active soluble forms, 18 kDa and 31 kDa, respectively (Graf et al. (1995) Eur. J. Immunol. 25:1749-1754; Mazzei et al. (1995) J. Biol. Chem. 270:7025-7028; Pietravalle et al. (1996) J. Biol. Chem. 271:5965-5967). CD40L is expressed on activated, but not resting, CD4⁺ T-helper cells (Lane et al. (1992) Eur. J. Immunol. 22:2573-2578; Spriggs et al. (1992) J. Exp. Med. 176:1543-1550; and Roy et al. (1993) J. Immunol. 151:1-14). Both CD40 and CD40L have been cloned and characterized (Stamenkovi et al. (1989) EMBO J. 8:1403-1410; Armitage et al. (1992) Nature 357:80-82; Lederman et al. (1992) J. Exp. Med 175:1091-1101; and Hollenbaugh et al. (1992) EMBO J. 11:4313-4321). See also U.S. Patent No. 5,945,513, describing human CD40L. Cells transfected with the CD40L gene and expressing the CD40L protein on their surface can trigger B-cell proliferation, and together with other stimulatory signals, can induce antibody production (Armitage *et al.* (1992) *supra*; and U.S. Patent No. 5,945,513). Patients with autoimmune disease have elevated serum levels of soluble CD40L (sCD40L) that are not seen in healthy subjects. Overexpression of CD40L causes autoimmune diseases similar to systemic lupus erythromatosus in rodent models (Higuchi et al. (2002) J. Immunol. 168:9-12). In contrast, absence of functional CD40L on activated T cells causes X-linked hyper-IgM syndrome (Allen et al. (1993) Science 259:990; and Korthauer et al. (1993) Nature 361:539). Further, blocking of CD40/CD40L interaction can prevent transplant rejection in non-human primate models. See, for example, Wee et al. (1992) Transplantation 53:501-7.

CD40 expression on APCs plays an important co-stimulatory role in the activation of these cells. For example, agonistic anti-CD40 monoclonal antibodies (mAbs) have been shown to mimic the effects of T helper cells in B-cell activation. When presented on adherent cells expressing FcγRII, these antibodies induce B-cell proliferation (Banchereau et al. (1989) Science 251:70). Moreover, agonistic anti-CD40 mAbs can replace the T helper signal for secretion of IgM, IgG, and IgE in the presence of IL-4 (Gascan et al. (1991) J. Immunol. 147:8). Furthermore, agonistic anti-CD40 mAbs can prevent programmed cell death (apoptosis) of B cells isolated from lymph nodes.

These and other observations support the current theory that the interaction of CD40 and CD40L plays a pivotal role in regulating both humoral and cell-mediated immune responses. More recent studies have revealed a much broader role of CD40/CD40L interaction in diverse physiological and pathological processes.

The CD40 signal transduction pathway depends on the coordinated regulation of many intracellular factors. Like other members of the TNF receptor family, CD40 activates TRAFs, including TRAF-2, -3, -5, and -6, which upregulate diverse signaling pathways following engagement of CD40 with CD40L (either membrane-bound CD40L or soluble CD40L), including extracellular signal-regulated kinase (ERK), c-jun amino terminal kinase (JNK), p38 MAPK, and NF-κB (see, for example, Younes and Carbone (1999) Int. J. Biol. Markers 14:135-143; van Kooten and Banchereau (2000) J. Leukoc. Biol. 67:2-17).

Signaling via CD40 has been shown to prevent cell death from apoptosis (Makus et al. (2002) J. Immunol. 14:973-982). Apoptotic signals are necessary to induce programmed cell death in a coordinated manner. Cell death signals can include intrinsic stimuli from within the cell such as endoplasmic reticulum stress or extrinsic stimuli such as receptor binding of FasL or TNFα. The signaling pathway is complex, involving activation of caspases such as Caspase-3 and Caspase-9, and of poly (ADP ribose) polymerase (PARP). During the cascade, anti-apoptotic signaling proteins, such as mcl-1 and bcl-x, and members of the IAP family of proteins, such as X-Linked Inhibitor of Apoptosis (XIAP), are down-regulated (Budihardjo et al. (1999) Annu. Rev. Cell Dev. Biol. 15:269-290). For example, in dendritic cells, CD40 cell signaling can block apoptosis signals transduced by FasL (Bjorck et al. (1997) Intl. Immunol. 9:365-372).

Thus, CD40 engagement by CD40L and subsequent activation of CD40 signaling are necessary steps for normal immune responses; however, dysregulation of CD40 signaling can lead to disease. The CD40 signaling pathway has been shown to be involved in autoimmune disease (Ichikawa et al. (2002) J. Immunol. 169:2781-2787 and Moore et al. (2002) J. Autoimmun. 19:139-145). Additionally, the CD40/CD40L interaction plays an important role in inflammatory processes. For example, both CD40 and CD40L are overexpressed in human and experimental atherosclerosis lesions. CD40 stimulation induces expression of matrix-degrading enzymes and tissue factor expression in atheroma-associated cell types, such as endothelial cells, smooth muscle cells, and macrophages. Further, CD40 stimulation induces production of proinflammatory cytokines such as IL-1, IL-6, and IL-8, and adhesion molecules such as ICAM-1, E-selectin, and VCAM. Inhibition of CD40/CD40L interaction prevents atherogenesis in animal models. In transplant models, blocking CD40/CD40L interaction prevents inflammation. It has been shown that CD40/CD40L binding acts synergistically with the Alzheimer amyloid-beta peptide to promote microglial activation, thus leading to neurotoxicity.

In patients with rheumatoid arthritis (RA), CD40 expression is increased on articular chondrocytes, thus, CD40 signaling likely contributes to production of damaging cytokines and matrix metalloproteinases. See, Gotoh et al. (2004) J. Rheumatol. 31:1506-1512. Further, it has been shown that amplification of the synovial inflammatory response occurs through activation of MAPKs and NF-κB via ligation of CD40 on CD14⁺ synovial cells from RA patients (Harigai et al. (2004) Arthritis. Rheum. 50:2167-2177). In an experimental model of RA, anti-CD40L antibody treatment prevented disease induction, joint inflammation, and anti-collagen antibody production (Durie et al. (1993) Science 261:1328-1330). Finally, in clinical trials, it has been shown that depleting CD20⁺ positive B cells of RA patients by administering Rituxan® (generally indicated for B cell lymphoma) improves symptoms (Shaw et al. (2003) Ann. Rheum. Dis. 62(Suppl. 2):ii55-ii59).

Blocking CD40/CD40L interactions during antigen presentation to T cells has Also been shown to induce T cell tolerance. Therefore, blocking CD40/CD40L interaction prevents initial T cell activation as well as induces long term tolerance to re-exposure to the antigen.

Human anti-CD40 monoclonal antibodies and a number of uses thereof are disclosed in co-owned patent applications published as WO 2005/044854, WO 2005/044304, WO 2005/044305, WO 2005/044306, WO 2005/044855, WO 2005/044307, and WO 2005/044294. Those applications specifically disclose a human IgG₁ anti-CD40 monoclonal antibody, designated as CHIR-12.12 therein, which was generated by immunization of transgenic mice bearing the human IgG₁ heavy chain locus and the human κ light chain locus (XenoMouse^{®} technology; Abgenix, California).

As shown by FACS analysis, CHIR-12.12 binds specifically to human CD40 and can prevent CD40-ligand (CD40L) binding. CHIR-12.12 can compete off CD40L pre-bound to cell surface CD40. The CHIR-12.12 monoclonal antibody is a strong antagonist and inhibits *in vitro* CD40L-mediated proliferation of normal and malignant B cells. The CHIR-12.12 monoclonal antibody directly inhibits survival and signaling pathways mediated by CD40L in normal human B-lymphocytes. *In vitro,* CHIR-12.12 kills primary cancer cells from NHL patients by ADCC. Dose-dependent anti-tumor activity was seen in a xenograft human lymphoma model. CHIR-12.12 is currently in Phase I trials for B-cell malignancies.

CD20 is a cell-surface antigen expressed early in B cell differentiation and remains on the cell surface throughout B cell development. CD20 is involved in B cell activation, is expressed at very high levels on neoplastic B cells, and is a clinically recognized therapeutic target (see, for example, Hooijberg et al. (1995) Cancer Research 55: 2627). Antibodies targeting CD20, such as rituximab (Rituxan®), have been approved by the U.S. Food and Drug Administration for the treatment of non-Hodgkin's lymphoma (see, for example, Boye et al. (2003) Ann. Oncol. 14:520*).* Rituxan has been shown to be an effective treatment for low-, intermediate-, and high-grade non-Hodgkin's lymphoma (NHL) (see for example, Maloney et al. (1994) Blood 84:2457-2466), McLaughlin et al. (1998) J. Clin. Oncol. 16:2825-2833, Maloney et al. (1997) Blood 90:2188-2195, Hainsworth et al. (2000) Blood 95:3052-3056, Colombat et al. (2001) Blood 97:101-106, Coiffer et al. (1998) Blood 92:1927-1932), Foran et al. (2000) J. Clin. Oncol.18:317-324, Anderson et al. (1997) Biochem. Soc. Trans. 25:705-708, or Vose et al. (1999) Ann. Oncol. 10:58a). Rituxan® also depletes normal B cells, which can play a role in inflammatory and autoimmune diseases. It is in clinical trials for autoimmune diseases.

Though the exact mechanism of action is not known, evidence indicates that the anti-lymphoma effects of Rituxan® are in part due to complement-mediated cytotoxicity (CMC), antibody-dependent cell-mediated cytotoxicity (ADCC), inhibition of cell proliferation, and finally direct induction of apoptosis. Some patients, however, become resistant to treatment with Rituxan® (see Witzig et al. (2002) J. Clin. Oncol. 20:3262, Grillo- Lopez et al. (1998) J. Clin. Oncol. 16:2825, or Jazirehi et al. (2003) Mol. Cancer Ther. 2:1183-1193). For example, some patients lose CD20 expression on malignant B cells after anti-CD20 antibody therapy (Davis et al. (1999) Clin. Cancer Res. 5:611). Furthermore, 30% to 50% of patients with low-grade NHL exhibit no clinical response to this monoclonal antibody (Hainsworth et al. (2000) Blood 95:3052-3056; Colombat et al. (2001) Blood 97:101-106). The clinical activity of rituximab in NHL has also been shown to be correlated with the patient's FcγRIIIa genotype. Patients with the FcγRIIIa 158aa polymorphism of V/V or V/F are more responsive to rituximab than those with F/F (for example, see Cartron et al. (2002) Blood 99(3):754-758 or Dall'Ozzo et al. (2004) Cancer Res. 64:4664-4669). For patients developing resistance to this monoclonal antibody, or having an inflammatory disease or autoimmune disease that is resistant to initial therapy with this antibody, alternative forms of therapeutic intervention are needed.

Further, Rituxan® depletes normal B cells in patients. Hence, it can be used to treat B cell dependent autoimmune and inflammatory diseases.

There is thus a continuing need for new therapeutic agents and new therapeutic strategies for inflammatory diseases and autoimmune diseases. In particular, there is a need for new therapeutic strategies for treatment of patients refractory to treatment with anti-CD20 antibodies, such as rituximab (Rituxan®). Law et al. (Cancer Res 2005; 65: 18) teaches the use of anti-CD40 antibodies for use in treatment of non-Hodgkin's lymphoma.

### SUMMARY OF THE INVENTION

The invention provides an anti-CD40 antibody, for use in the treatment of an inflammatory disease or autoimmune disease that is associated with CD40-expressing cells in a human patient heterozygous or homozygous for FcγRIIIa-158F (genotype V/F or F/F), wherein said antibody is selected from the group consisting of:
a) a monoclonal antibody that binds to an epitope capable of binding the monoclonal antibody CHIR-12.12 produced by the hybridoma cell line deposited with the ATCC as Patent Deposit No. PTA-5543;
b) a monoclonal antibody that binds to an epitope comprising residues 82-87 of the human CD40 sequence shown in SEQ ID NO:7 or SEQ ID NO:9; and
c) a monoclonal antibody that competes with the monoclonal antibody CHIR-12.12 produced by the hybridoma cell line deposited with the ATCC as Patent Deposit No. PTA-5543 in a competitive binding assay.

The invention also provides use of a therapeutically or prophylactically effective amount of an anti-CD40 antibody in the manufacture of a medicament for the treatment of an inflammatory disease or autoimmune disease that is associated with CD40-expressing cells in a human patient heterozygous or homozygous for FcγRIIIa-158F (genotype V/F or F/F), wherein said antibody is selected from the group consisting of:
a) a monoclonal antibody that binds to an epitope capable of binding the monoclonal antibody CHIR-12.12 produced by the hybridoma cell line deposited with the ATCC as Patent Deposit No. PTA-5543;
b) a monoclonal antibody that binds to an epitope comprising residues 82-87 of the human CD40 sequence shown in SEQ ID NO:7 or SEQ ID NO:9; and
c) a monoclonal antibody that competes with the monoclonal antibody CHIR-12.12 produced by the hybridoma cell line deposited with the ATCC as Patent Deposit No. PTA-5543 in a competitive binding assay.
The invention also provides a method for selecting an antibody therapy for treatment of a human patient having an inflammatory disease or autoimmune disease which is refractory to treatment with rituximab (Rituxan®), comprising determining the FcγRIIIa-158 genotype (V/V, V/F or F/F) of a human patient having an inflammatory disease or autoimmune disease that is associated with CD40-expressing cells and which is refractory to treatment with rituximab (Rituxan®) using a biological sample obtained from the human patient;
wherein if said human patient is heterozygous or homozygous for FcγRIIIa-158F (genotype V/F or F/F), an anti-CD40 antibody is selected for treatment of said inflammatory disease or autoimmune disease,
wherein said antibody is selected from the group consisting of:
a) a monoclonal antibody that binds to an epitope capable of binding the monoclonal antibody CHIR-12.12 produced by the hybridoma cell line deposited with the ATCC as Patent Deposit No. PTA-5543;
b) a monoclonal antibody that binds to an epitope comprising residues 82-87 of the human CD40 sequence shown in SEQ ID NO:7 or SEQ ID NO:9; and
c) a monoclonal antibody that competes with the monoclonal antibody CHIR-12.12 produced by the hybridoma cell line deposited with the ATCC as Patent Deposit No. PTA-5543 in a competitive binding assay.

### BRIEF SUMMARY OF THE DISCLOSURE

Methods for treating a human patient for an inflammatory disease or autoimmune disease that is associated with CD40-expressing cells are disclosed, where the human patient is heterozygous or homozygous for FcγRIIIa-158F (genotype V/F or F/F). The methods comprise administering to the human patient a therapeutically or prophylactically effective amount of an anti-CD40 antibody. The invention provides for the use of a therapeutically or prophylactically effective amount of an anti-CD40 antibody in the manufacture of a medicament for the treatment of an inflammatory disease or autoimmune disease that is associated with CD40-expressing cells in a human patient heterozygous or homozygous for FcγRIIIa-158F (genotype V/F or F/F).

Also disclosed are methods of inhibiting antibody production by B cells in a human patient heterozygous or homozygous for FcγRIIIa-158F (genotype V/F or F/F), comprising administering to the human patient an effective amount of an anti-CD40 antibody. Also disclosed is the use of an effective amount of an anti-CD40 antibody in the manufacture of a medicament for inhibiting antibody production by B cells in a human patient heterozygous or homozygous for FcγRIIIa-158F (V/F or F/F).

Methods and kits for identifying a human patient with an inflammatory disease or autoimmune disease that is treatable with an anti-CD40 antibody and which is refractory to treatment with rituximab (Rituxan®) are also disclosed. In some embodiments, the methods comprise: a) identifying a human patient with an inflammatory disease or autoimmune disease that is associated with CD40-expressing cells and which is refractory to treatment with rituximab (Rituxan®); and b) determining the human patient's FcγRIIIa-158 genotype (V/V, V/F or F/F); wherein the inflammatory disease or autoimmune disease is treatable with an anti-CD40 antibody if the human patient is heterozygous or homozygous for FcγRIIIa-158F (genotype V/F or F/F). The disclosure may further include the step of administering to a human patient identified using this method a therapeutically or prophylactically effective amount of an anti-CD40 antibody. Kits of the disclosure that provide for identification of a human patient with an inflammatory disease or autoimmune disease that is treatable with an anti-CD40 antibody comprise reagents for determining a human patient's FcγRIIIa-158 genotype.

Also disclsosed are methods and kits for selecting an antibody therapy for treatment of a human patient having an inflammatory disease or autoimmune disease that is refractory to treatment with rituximab (Rituxan®).In some embodiments, the methods comprise: a) identifying a human patient having an inflammatory or autoimmune disease that is associated with CD40-expressing cells and which is refractory to treatment with rituximab (Rituxan®); and b) determining the human patient's FcγRIIIa-158 genotype (V/V, V/F or F/F); wherein if the human patient is heterozygous or homozygous for FcγRIIIa-158F (genotype V/F or F/F), an anti-CD40 antibody is selected for treatment of the inflammatory disease or autoimmune disease. The disclosure may further include the step of administering to a human patient identified using this method a therapeutically or prophylactically effective amount of an anti-CD40 antibody. Kits of the disclosure that provide for selecting an antibody therapy for treatment of a human patient having an inflammatory or autoimmune disease associated with CD40-expressing cells comprise reagents for determining a human patient's FcγRIIIa-158 genotype.

Also disclosed are methods for treating a human patient for an inflammatory disease or autoimmune disease that is associated with CD40-expressing cells, where the methods comprise administering to the human patient a slow-internalizing antibody. In one such embodiment, a therapeutically or prophylactically effective amount of an anti-CD40 antibody is administered to the human patient such that the anti-CD40 antibody is not significantly internalized by CD40-expressing cells following its administration. In another such embodiment, a therapeutically or prophylactically effective amount of an anti-CD40 antibody is administered to the human patient such that the anti-CD40 antibody remains substantially uniformly distributed on the surface of CD40-expressing cells following its administration. In yet another such embodiment, an anti-CD40 antibody is administered to the human patient such that a therapeutically or prophylactically effective amount of the anti-CD40 antibody is present at the surface of CD40-expressing cells in the human patient following its administration.

Anti-CD40 antibodies for use in accordance with the present invention specifically bind the CD40 antigen. In some embodiments, anti-CD40 antibodies for use in the methods of the disclosure, in particular monoclonal antibodies, exhibit a strong binding affinity for human FcγRIIIa-158V, a strong binding affinity for human FcγRIIIa-158F, or a strong binding affinity for both human FcγRIIIa-158V and FcγIIIa-158F. In some of these embodiments, the anti-CD40 antibodies can bind to either of the two FcγRIIIa amino acid 158 allotypes (V or F) on a human patient's natural killer (NK) cells with binding characteristics that are adequate to cause potent antibody-dependent cellular cytotoxicity (ADCC). Suitable anti-CD40 antibodies include, but are not limited to, anti-CD40 antibodies that are free of significant agonist activity, including, for example, anti-CD40 antibodies that are an antagonist of CD40-CD40L signaling on CD40-expressing cells. In some embodiments, the anti-CD40 antibody is selected from the group consisting of: a) the monoclonal antibody CHIR-12.12; b) the monoclonal antibody produced by the hybridoma cell line 12.12; c) a monoclonal antibody comprising an amino acid sequence selected from the group consisting of the sequence shown in SEQ ID NO:2, the sequence shown in SEQ ID NO:4, the sequence shown in SEQ ID NO:5, both the sequences shown in SEQ ID NO:2 and SEQ ID NO:4, and both the sequences shown in SEQ ID NO:2 and SEQ ID NO:5; d) a monoclonal antibody having an amino acid sequence encoded by a nucleic acid molecule comprising a nucleotide sequence selected from the group consisting of the sequence shown in SEQ ID NO:1, the sequence shown in SEQ ID NO:3, and both the sequences shown in SEQ ID NO:1 and SEQ ID NO:3; e) a monoclonal antibody that binds to an epitope capable of binding the monoclonal antibody produced by the hybridoma cell line 12.12; f) a monoclonal antibody that binds to an epitope comprising residues 82-87 of the human CD40 sequence shown in SEQ ID NO:7 or SEQ ID NO:9; g) a monoclonal antibody that binds to an epitope comprising residues 82-89 of the human CD40 sequence shown in SEQ ID NO:7 or SEQ ID NO:9; h) a monoclonal antibody that competes with the monoclonal antibody CHIR-12.12 in a competitive binding assay; i) the monoclonal antibody of preceding item a) or a monoclonal antibody of any one of preceding items c)-h), wherein the antibody is recombinantly produced; and j) a monoclonal antibody that is an antigen-binding fragment of a monoclonal antibody of any one of preceding items a)-i), wherein the fragment retains the capability of specifically binding to human CD40 antigen.

The methods of the disclosure find use in treatment of inflammatory diseases or autoimmune diseases that are associated with CD40-expressing cells. Examples include, but are not limited to, systemic lupus erythematosus (SLE), discoid lupus, lupus nephritis, sarcoidosis, inflammatory arthritis, including juvenile arthritis, rheumatoid arthritis, psoriatic arthritis, Reiter's syndrome, ankylosing spondylitis, and gouty arthritis, rejection of an organ or tissue transplant, hyperacute, acute, or chronic rejection and/or graft versus host disease, multiple sclerosis, hyper IgE syndrome, polyarteritis nodosa, primary biliary cirrhosis, inflammatory bowel disease, Crohn's disease, celiac's disease (gluten-sensitive enteropathy), autoimmune hepatitis, pernicious anemia, autoimmune hemolytic anemia, psoriasis, scleroderma, myasthenia gravis, autoimmune thrombocytopenic purpura, autoimmune thyroiditis, Grave's disease, Hasimoto's thyroiditis, immune complex disease, chronic fatigue immune dysfunction syndrome (CFIDS), polymyositis and dermatomyositis, cryoglobulinemia, thrombolysis, cardiomyopathy, pemphigus vulgaris, pulmonary interstitial fibrosis, Type I and Type II diabetes mellitus, type 1, 2, 3, and 4 delayed-type hypersensitivity, allergy or allergic disorders, unwanted/unintended immune responses to therapeutic proteins, asthma, Churg-Strauss syndrome (allergic granulomatosis), atopic dermatitis, allergic and irritant contact dermatitis, urtecaria, IgE-mediated allergy, atherosclerosis, vasculitis, idiopathic inflammatory myopathies, hemolytic disease, Alzheimer's disease, and chronic inflammatory demyelinating polyneuropathy, pulmonary inflammation including but not limited to lung graft rejection, asthma, sarcoidosis, emphysema, cystic fibrosis, idiopathic pulmonary fibrosis, chronic bronchitis, allergic rhinitis and allergic diseases of the lung such as hypersensitivity pneumonitis, eosinophilic pneumonia, bronchiolitis obliterans due to bone marrow and/or lung transplantation or other causes, graft atherosclerosis/graft phlebosclerosis, pulmonary fibrosis resulting from collagen, vascular, and autoimmune diseases such as rheumatoid arthritis and lupus erythematosus, as well as inflammatory diseases or autoimmune diseases associated with CD20-expressing cells. The methods of the disclosure are particularly advantageous with respect to inflammatory diseases and autoimmune diseases that are associated with cells expressing both CD40 and CD20. In this manner, the disclosure enables the treatment of patients having an inflammatory or autoimmune disease that is non-responsive or refractory to therapy with other therapeutic agents, including anti-CD20 antibodies for patients who are homozygous or heterozygous for the FcγRIIIa-158F (genotype V/F or F/F).

### BRIEF DESCRIPTION OF THE DRAWINGS

Figure 1A-1F show results of an analysis of antibody-dependent cellular cytotoxicity (ADCC) in six cell lines.
Figure 2A-2D show results of an analysis of antibody-dependent cellular cytotoxicity (ADCC) in CLL patient cells (n = 8).
Figure 3 summarizes results of an analysis of ADCC in CLL patient cells (n=9).
Figure 4 shows results of an analysis of ADCC in CLL patient cells, using NK effector cells from two different donors.
Figure 5 shows results of quantitation of CD40 and CD20 cell-surface expression on CLL patient cells and normal B cells.
Figure 6 summarizes ADCC activity for cells with quantitated CD40 and CD20 cell-surface expression.
Figure 7 is a bar chart showing levels of cell-surface bound CHIR-12.12 on Daudi and ARH77 cell lines.
Figure 8 shows results of investigation of internalization of CHIR-12.12 and rituximab in CLL patient cells by FACS analysis.
Figure 9 shows results of investigation of internalization of CHIR-12.12 and rituximab in normal B cells by confocal microscopy of FITC-labelled antibodies.
Figure 10 shows results of investigation of internalization of CHIR-12.12 and rituximab in CLL patient cells by confocal microscopy of Alexa488-labelled antibodies.
Figure 11 summarizes the relationship between ADCC activity and internalization.
Figure 12 is a bar chart showing maximum percentage specific lysis of Daudi cells by CHIR-12.12 or rituximab by purified NK effector cells from donors with different FcγRIIIa genotypes.
Figure 13 is a bar chart showing ADCC potency (ED₅₀) of CHIR-12.12 or rituximab on Daudi cells by in purified NK effector cells from donors with different FcγRIIIa genotypes.
Figure 14 summarizes comparative ADCC of CHIR-12.12 and rituximab against CLL patient cells (n=9) by human NK cells from multiple genotyped human donors.

### DETAILED DESCRIPTION

The inventors have made the surprising finding that anti-CD40 antibodies, such as CHIR-12.12, are able to mediate potent antibody-dependent cellular cytotoxicity (ADCC) of CD40-expressing target cells under conditions where other ADCC mediating antibodies are less effective or relatively ineffective. Contrary to other antibodies, such as rituximab (Rituxan®), anti-CD40 antibodies used according to the invention can bind to either of the two FcγRIIIa amino acid 158 allotypes (V or F) on a human patient's natural killer (NK) cells with binding characteristics that are adequate to cause potent ADCC. This finding is unexpected and represents an advance in our ability to treat inflammatory diseases and autoimmune diseases across an entire patient cross-section.

Accordingly, anti-CD40 antibodies, such as CHIR-12.12, can be used in the treatment of inflammatory diseases and autoimmune diseases associated with CD40-expressing cells in human patients heterozygous or homozygous for FcγRIIIa-158F (genotype V/F or F/F), in addition to human patients homozygous for FcγRIIIa-158V (genotype V/V).

Disclosed herein is a method for treating a human patient for an inflammatory disease or autoimmune disease that is associated with CD40-expressing cells, wherein said human patient is heterozygous or homozygous for FcγRIIIa-158F (genotype V/F or F/F), the method comprising administering to said human patient a therapeutically or prophylactically effective amount of an anti-CD40 antibody. The invention provides the use of a therapeutically or prophylactically effective amount of an anti-CD40 antibody in the manufacture of a medicament for the treatment of an inflammatory disease or autoimmune disease that is associated with CD40-expressing cells in a human patient heterozygous or homozygous for FcγRIIIa-158F (genotype V/F or F/F).

As noted above, the clinical activity of rituximab in NHL has been shown to be correlated with the patient's FcγRIIIa genotype. Patients with the FcγRIIIa 158aa polymorphism of F/F are less responsive to rituximab than those with V/V or V/F (for example, see Cartron et al. (2002) Blood 99(3):754-758 or Dall'Ozzo et al. (2004) Cancer Res. 64:4664-4669. As noted above, Rituxan® is in clinical trials for autoimmune diseases. Accordingly, the present invention is advantageous for the treatment of inflammatory diseases and autoimmune diseases that are not responsive to treatment with an anti-CD20 antibody such as rituximab (Rituxan®).Morover, such potent killing of target cells without the need to use an antibody-toxin conjugate will result in a drug that is cheaper to make and having fewer side effects.

Anti-CD40 antibodies, such as CHIR-12.12, can be used in methods for inhibiting antibody production by B cells in a human patient heterozygous or homozygous for FcγRIIIa-158F (genotype V/F or F/F), in addition to human patients homozygous for FcγRIIIa-158V (genotype V/V).

Disclosed herein is a method of inhibiting antibody production by B cells in a human patient heterozygous or homozygous for FcγRIIIa-158F (genotype V/F or F/F), comprising administering to said human patient an effective amount of an anti-CD40 antibody, such as CHIR-12.12. Also disclosed herein is the use of an effective amount of an anti-CD40 antibody in the manufacture of a medicament for inhibiting antibody production by B cells in a human patient heterozygous or homozygous for FcγRIIIa-158F (V/F or F/F).

It would not have been expected by a person skilled in the art that one could inhibit antibody production by B cells in a human patient heterozygous or homozygous for FcγRIIIa-158F (genotype V/F or F/F).

The disclosure allows the treatment regimen selected for an individual human patient to be based on that patient's FcγRIIIa-158 genotype by administering an ADCC-mediating anti-CD40 antibody.

Disclosed herein is a method for identifying a human patient with an inflammatory disease or autoimmune disease treatable with an anti-CD40 antibody and which is refractory to treatment with rituximab (Rituxan®), comprising:
a) identifying a human patient with an inflammatory disease or autoimmune disease that is associated with CD40-expressing cells and which is refractory to treatment with rituximab (Rituxan®); and
b) determining said human patient's FcγRIIIa-158 genotype (V/V, V/F or F/F);
wherein said inflammatory disease or autoimmune disease is treatable with an anti-CD40 antibody if said human patient is heterozygous or homozygous for FcγRIIIa-158F (genotype V/F or F/F). The disclosure may further include the step of administering to a human patient identified using this method a therapeutically or prophylactically effective amount of an anti-CD40 antibody.

This method of identifying a human patient with an inflammatory disease or autoimmune disease treatable with an anti-CD40 antibody can readily be performed by a person skilled in the art using a suitable diagnostic kit. The kit should comprise reagents suitable for determining a human patient's FcγRIIIa-158 genotype. Thus, also disclosed is a kit for identifying a human patient with an inflammatory disease or autoimmune disease treatable with an anti-CD40 antibody, comprising reagents for determining a human patient's FcγRIIIa-158 genotype. Suitable kits are described in more detail elsewhere herein.

Also disclosed herein is a method for selecting an antibody therapy for treatment of a human patient having an inflammatory disease or autoimmune disease which is refractory to treatment with rituximab (Rituxan®), comprising:
a) identifying a human patient having an inflammatory disease or autoimmune disease that is associated with CD40-expressing cells and which is refractory to treatment with rituximab (Rituxan®); and
b) determining said human patient's FcγRIIIa-158 genotype (V/V, V/F or F/F);
wherein if said human patient is heterozygous or homozygous for FcγRIIIa-158F (genotype V/F or F/F), an anti-CD40 antibody is selected for treatment of said inflammatory disease or autoimmune disease. In particular, an anti-CD40 antibody may be selected in preference to treatment with rituximab (Rituxan®). The disclosure further includes the step of administering to a human patient identified using this method a therapeutically or prophylactically effective amount of an anti-CD40 antibody.

This method of selecting an antibody therapy for treatment of a human patient having an inflammatory disease or autoimmune disease can readily be performed by a person skilled in the art using a suitable diagnostic kit. The kit should comprise reagents suitable for determining a human patient's FcγRIIIa-158 genotype. Thus, also disclosed herein is a kit for selecting an antibody therapy for treatment of a human patient having an inflammatory disease or autoimmune disease associated with CD40-expressing cells, comprising reagents for determining a human patient's FcγRIIIa-158 genotype.

The inventors have also made the surprising finding that anti-CD40 antibodies, such as CHIR-12.12, are not significantly internalized by CD40-expressing cells following administration. Instead, and-CD40 antibodies, such as CHIR-12.12, are substantially uniformly distributed on the surface of CD40-expressing cells for a significant period of time following administration. This is in contrast to other antibodies, in particular anti-CD20 antibodies, such as rituximab (Rituxan®).

The duration of CD40 binding at the surface of CD40-expressing cells and the uniform distribution of the anti-CD40 antibody on the surface of CD40-expressing cells enables the anti-CD40 antibodies to mediate potent antibody-dependent cellular cytotoxicity (ADCC) of CD40-expressing target cells, via binding to an FcR, such as the FcγRIIIa on natural killer (NK) cells.

Thus, disclosed herein is a method for treating a human patient for an inflammatory disease or autoimmune disease that is associated with CD40-expressing cells, the method comprising administering to said human patient a therapeutically or prophylactically effective amount of an anti-CD40 antibody, such that the anti-CD40 antibody is not significantly internalized by CD40-expressing cells following administration.

Also disclosed herein is a method for treating a human patient for an inflammatory disease or autoimmune disease that is associated with CD40-expressing cells, the method comprising administering to said human patient a therapeutically or prophylactically effective amount of an anti-CD40 antibody, such that the anti-CD40 antibody remains substantially uniformly distributed on the surface of CD40-expressing cells following administration.

Also disclosed herein is a method for treating a human patient for an inflammatory disease or autoimmune disease that is associated with CD40-expressing cells, the method comprising administering to said human patient an anti-CD40 antibody, such that a therapeutically or prophylactically effective amount of the anti-CD40 antibody is present at the surface of CD40-expressing cells in said human patient following administration.

These aspects of the disclosure thus involve administering to a patient a slow-internalizing antibody. By "slow-intemalizing antibody" we mean an antibody that remains disposed on the cell surface for a significant period of time. As the skilled person will be aware, this property contrasts with properties deemed advantageous for many therapeutic applications that actually require internalization of antibody-receptor complex in order for the therapy to be efficacious. In this context, a significant period of time generally exceeds 3 hours, preferably 6 hours, more preferably 12 hours, more preferably 24 hours, 36 hours, 48 hours, 72 hours, 96 hours, 120 hours, 144 hours, 168 hours or more.

Preferably, at least 5%, at least 10%, at least 20%, at least 30%, at least 40%, at least 50%, at least 60%, at least 70%, at least 80%, at least 90%, or more of the antibody initially disposed on the surface of a CD40-expressing cell remains disposed on the surface of the cell after the above significant period of time.

Internalization of antibodies can be assessed by various assays. For example, cell lines such as the Daudi lymphoma cell line, or ARH77 MM cell line, can be used to evaluate the effect of a candidate antibody binding on internalization. Cells are incubated with human IgG1 (control antibody) or the candidate antibody on ice (with 0.1 % sodium azide to block internalization) or 37°C (without sodium azide) for a period of time, suitably 3 hours. After a wash with cold staining buffer (e.g. PBS+1%BSA+0.1% sodium azide), cells are stained, for example with goat anti-human IgG-FITC for 30 minutes on ice. The degree of staining can then be assessed; in this example, geometric mean fluorescent intensity (MFI) could be recorded, such as by FACS Calibur. Other suitable assays will be known to those of skill in the art (see, for example http://www.abgenix.com/documents/SBS2003%20poster.pdf).

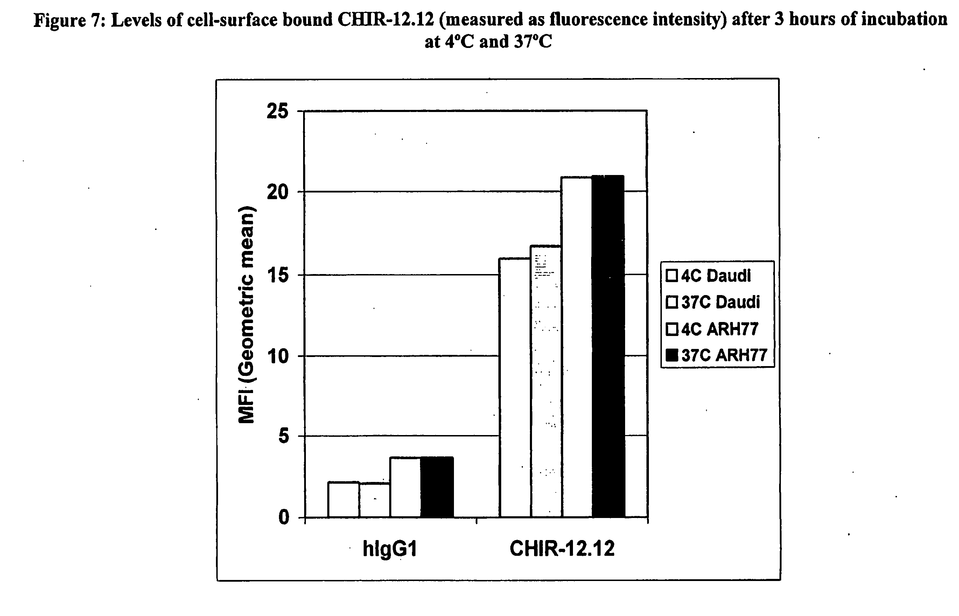

In experiments set out in Examples 4 and 5 herein, no difference in MFI was observed between cells incubated with CH12.12 on ice in the presence of sodium azide or at 37°C in the absence of sodium azide (see Figures 7-10). These data show that CH12.12, upon binding to CD40, is not internalized and continues to be displayed on the cell surface.

A summary of standard techniques and procedures which may be employed in order to utilise the invention is given below. It will be understood that this invention is not limited to the particular methodology, protocols, cell lines, vectors and reagents described. It is also to be understood that the terminology used herein is for the purpose of describing particular embodiments only and it is not intended that this terminology should limit the scope of the present invention. The extent of the invention is limited only by the terms of the appended claims.

Standard abbreviations for nucleotides and amino acids are used in this specification.

The practice of the present invention will employ, unless otherwise indicated, conventional techniques of molecular biology, microbiology, recombinant DNA technology and immunology, which are within the skill of those working in the art.

Such techniques are explained fully in the literature. Examples of particularly suitable texts for consultation include the following: Sambrook et al. (1989) Molecular Cloning; A Laboratory Manual (2d ed.); D.N Glover, ed. (1985) DNA Cloning, Volumes I and II; M.J. Gait, ed. (1984) Oligonucleotide Synthesis*;* B.D. Hames & S.J. Higgins, eds. (1984) Nucleic Acid Hybridization*;* B.D. Hames & S.J. Higgins, eds. (1984) Transcription and Translation*;* R.I. Freshney, ed. (1986) Animal Cell Culture; Immobilized Cells and Enzymes (IRL Press, 1986); B. Perbal (1984) A Practical Guide to Molecular Cloning; the Methods in Enzymology series (Academic Press, Inc.), especially volumes 154 & 155; J.H. Miller and M.P. Calos, eds. (1987) Gene Transfer Vectors for Mammalian Cells (Cold Spring Harbor Laboratory); Mayer and Walker, eds. (1987) Immunochemical Methods in Cell and Molecular Biology (Academic Press, London); Scopes (1987) Protein Purification: Principles and Practice (2d ed.; Springer Verlag, N.Y.); and D.M. Weir and C. C. Blackwell, eds. (1986) Handbook of Experimental Immunology, Volumes I-IV.

The methods disclosed herein involve the use of anti-CD40 antibodies in the treatment of inflammatory diseases and autoimmune diseases associated with CD40-expressing cells.

By "CD40", "CD40 antigen", or "CD40 receptor" is intended the 50-55 kDa transmembrane glycoprotein of the tumor necrosis factor (TNF) receptor family (see, for example, U.S. Patent Nos. 5,674,492 and 4,708,871; Stamenkovic et al. (1989) EMBO 8:1403; Clark (1990) Tissue Antigens 36:33; Barclay et al. (1997) The Leucocyte Antigen Facts Book (2d ed.; Academic Press, San Diego)). Two isoforms of human CD40, encoded by alternatively spliced transcript variants of this gene, have been identified. The first isoform (also known as the "long isoform" or "isoform 1") is expressed as a 277-amino-acid precursor polypeptide (SEQ ID NO:9; first reported as GenBank Accession No. CAA43045, and identified as isoform 1 in GenBank Accession No. NP_001241), encoded by SEQ ID NO:8 (see GenBank Accession Nos. X60592 and NM_001250), which has a signal sequence represented by the first 19 residues. The second isoform (also known as the "short isoform" or "isoform 2") is expressed as a 203-amino-acid precursor polypeptide (SEQ ID NO:7; GenBank Accession No. NP_690593), encoded by SEQ ID NO:6 (GenBank Accession No. NM_152854), which also has a signal sequence represented by the first 19 residues. The precursor polypeptides of these two isoforms of human CD40 share in common their first 165 residues (i.e., residues 1-165 of SEQ ID NO:7 and SEQ ID NO:9). The precursor polypeptide of the short isoform (shown in SEQ ID NO:7) is encoded by a transcript variant (SEQ ID NO:6) that lacks a coding segment, which leads to a translation frame shift; the resulting CD40 isoform contains a shorter and distinct C-terminus (residues 166-203 of SEQ ID NO:7) from that contained in the long isoform of CD40 (C-terminus shown in residues 166-277 of SEQ ID NO:9). For purposes of the present invention, the term "CD40," or "CD40 antigen," "CD40 cell surface antigen," or "CD40 receptor" encompasses both the short and long isoforms of CD40. The CD40 antigen may be fully or partially glycosylated.

As noted elsewhere herein, CD40 is found on the surface of both normal and neoplastic human B cells, dendritic cells, monocytes, macrophages, CD8⁺ T cells, endothelial cells, monocytic and epithelial cells, activated T cells, activated platelets, inflamed vascular smooth muscle cells, eosinophils, synovial membranes in rheumatoid arthritis, dermal fibroblasts, and other non-lymphoid cell types.

By "CD40-expressing cells" herein is intended any normal or malignant cells that express detectable levels of the CD40 antigen. Preferably, the CD40-expresing cells are cells that express detectable levels of cell-surface CD40 antigen. Methods for detecting CD40 expression in cells are well known in the art and include, but are not limited to, PCR techniques, immunohistochemistry, flow cytometry, Western blot, ELISA, and the like. These methods allow for the detection of CD40 mRNA, CD40 antigen and cell-surface CD40 antigen. Detection of cell-surface CD40 expression can be performed as described in Example 3 herein, or by other suitable methods.

By "CD40 ligand" or "CD40L" is intended primarily the 32-33 kDa transmembrane protein that also exists in two smaller biologically active soluble forms, 18 kDa and 31 kDa, respectively (Graf et al. (1995) Eur. J. Immunol. 25:1749-1754; Mazzei et al. (1995) J. Biol. Chem. 270:7025-7028; Pietravalle et al. (1996) J. Biol. Chem. 271:5965-5967). Human CD40L is also known as CD154 or gp39. By "CD40 ligand" or "CD40L" is also intended to any other peptide, polypeptide, or protein that can bind to and activate one or more CD40 signaling pathways. Thus, "CD40 ligands" include, but are not limited to, full-length CD40 ligand proteins and variants and fragments thereof that retain sufficient activity to carry out the function of binding to and stimulating CD40 signaling on CD40-expressing cells. Modifications to a native CD40 ligand, for example, human CD40L, include, but are not limited to, substitutions, deletions, truncations, extensions, fusion proteins, fragments, peptidomimetics, and the like.

By "CD40 signaling" is intended any of the biological activities that result from interaction of cell-surface CD40 with a CD40 ligand or other agonist, such as an agonist antibody. Examples of CD40 signaling are signals that lead to proliferation and survival of CD40-expressing cells, and stimulation of one or more CD40-signaling pathways within CD40-expressing cells. A CD40 "signaling pathway" or "signal transduction pathway" is intended to mean at least one biochemical reaction, or a group of biochemical reactions, that results from interaction of the CD40 receptor with a CD40 ligand, for example, CD40L, and which generates a signal that, when transmitted through the signal pathway, leads to activation of one or more downstream molecules in the signaling cascade. Signal transduction pathways involve a number of signal transduction molecules that lead to transmission of a signal from the cell-surface CD40 receptor across the plasma membrane of a cell, and through one or more in a series of signal transduction molecules, through the cytoplasm of the cell, and in some instances, into the cell's nucleus. Of particular interest to the present invention are CD40 signal transduction pathways, including the AKT signaling pathway, which leads to activation of AKT, and ultimately activation of NF-κB via the NF-κB signaling pathway; and mitogen-activated protein kinase (MAPK) signaling pathways, including the MEK/ERK signaling pathway and the MEK/p38 signaling pathway, which lead to activation of ERK and p38, respectively.

As noted above, disclosed herein is a method for treating a human patient for an inflammatory disease or autoimmune disease that is associated with CD40-expressing cells, wherein said human patient is heterozygous or homozygous for FcγRIIIa-158F (genotype V/F or F/F), the method comprising administering to said human patient a therapeutically or prophylactically effective amount of an anti-CD40 antibody.

By "human patient" is intended a human patient who is afflicted with, at risk of developing or relapsing with, any inflammatory disease or autoimmune disease that is associated with CD40-expressing cells.

By "inflammatory disease or autoimmune disease associated with CD40-expressing cells" is intended any inflammatory disease or autoimmune disease associated with CD40-expressing cells. The inflammatory disease or autoimmune disease associated with CD40-expressing cells may be an inflammatory disease or autoimmune disease associated with an undesirable level of CD40 signaling on CD40-expressing cells, or the inflammatory disease or autoimmune disease might be only indirectly associated with CD40-expressing cells. By "an inflammatory disease or autoimmune disease associated with an undesirable level of CD40 signaling" is intended an inflammatory disease or autoimmune disease whose development or progression is associated with an undesirable level of CD40 signaling.

By "an undesirable level of CD40 signaling" is intended any physiologically undesirable level of CD40 signaling that might occur in CD40-expressing cells in a human patient having an inflammatory disease or autoimmune disease.

Inflammatory diseases are characterized by inflammation and tissue destruction, or a combination thereof. "Inflammatory disease" includes any inflammatory immune-mediated process where the initiating event or target of the immune response involves non-self antigen(s), including, for example, alloantigens, xenoantigens, viral antigens, bacterial antigens, unknown antigens, or allergens.

As used herein, the term "autoimmunity" is generally understood to encompass inflammatory immune-mediated processes involving "self" antigens. In autoimmune diseases, self antigen(s) trigger host immune responses.

The present disclosure can be used in the treatment of inflammation associated with tissue transplant rejection. "Transplant rejection" or "graft rejection" refers to any host-mounted immune response against a graft including but not limited to HLA antigens, blood group antigens, and the like.

The disclosure can also be used to treat graft versus host disease, such as that associated with bone marrow transplantation, for example. In such graft versus host disease, the donor bone marrow includes lymphocytes and cells that mature into lymphocytes. The donor's lymphocytes recognize the recipient's antigens as non-self and mount an inflammatory immune response. Hence, as used herein, "graft versus host disease" or "graft versus host reaction" refers to any T cell mediated immune response in which donor lymphocytes react to the host's antigens.

Inflammatory diseases and autoimmune diseases that can be treated in accordance with the methods disclosed herein include, but not limited to, systemic lupus erythematosus (SLE), discoid lupus, lupus nephritis, sarcoidosis, inflammatory arthritis, including juvenile arthritis, rheumatoid arthritis, psoriatic arthritis, Reiter's syndrome, ankylosing spondylitis, and gouty arthritis, rejection of an organ or tissue transplant, hyperacute, acute, or chronic rejection and/or graft versus host disease, multiple sclerosis, hyper IgE syndrome, polyarteritis nodosa, primary biliary cirrhosis, inflammatory bowel disease, Crohn's disease, celiac's disease (gluten-sensitive enteropathy), autoimmune hepatitis, pernicious anemia, autoimmune hemolytic anemia, psoriasis, scleroderma, myasthenia gravis, autoimmune thrombocytopenic purpura, autoimmune thyroiditis, Grave's disease, Hasimoto's thyroiditis, immune complex disease, chronic fatigue immune dysfunction syndrome (CFIDS), polymyositis and dermatomyositis, cryoglobulinemia, thrombolysis, cardiomyopathy, pemphigus vulgaris, pulmonary interstitial fibrosis, Type I and Type II diabetes mellitus, type 1, 2, 3, and 4 delayed-type hypersensitivity, allergy or allergic disorders, unwanted/unintended immune responses to therapeutic proteins (see for example, U.S. Patent Application No. US 2002/0119151 and Koren, et al. (2002) Curr. Pharm. Biotechnol. 3:349-60), asthma, Churg-Strauss syndrome (allergic granulomatosis), atopic dermatitis, allergic and irritant contact dermatitis, urtecaria, IgE-mediated allergy, atherosclerosis, vasculitis, idiopathic inflammatory myopathies, hemolytic disease, Alzheimer's disease, chronic inflammatory demyelinating polyneuropathy, and the like. The methods of the disclosure are also useful in the treatment of pulmonary inflammation including but not limited to lung graft rejection, asthma, sarcoidosis, emphysema, cystic fibrosis, idiopathic pulmonary fibrosis, chronic bronchitis, allergic rhinitis and allergic diseases of the lung such as hypersensitivity pneumonitis, eosinophilic pneumonia, bronchiolitis obliterans due to bone marrow and/or lung transplantation or other causes, graft atherosclerosis/graft phlebosclerosis, as well as pulmonary fibrosis resulting from collagen, vascular, and autoimmune diseases such as rheumatoid arthritis and lupus erythematosus.

The inflammatory disease or autoimmune disease may be an inflammatory disease or autoimmune disease associated with CD40-expressing cells. Examples of antibody dependent autoimmune diseases include Rheumatoid Arthritis, Psoriasis, Systemic Lupus Erythematosus, Crohn's disease, Myasthenia gravis, Idiopathic Thrombocytopenia purpura, or Sjogren's syndrome.

Further, depletion of B cells and other CD40 bearing cells could limit T cell activation by signaling through CD40 ligand binding. Hence, depleting B cells and other CD40 bearing cells could be used to treat T-cell mediated autoimmune and inflammatory diseases, such as Multiple Sclerosis, graft rejection, graft versus host disease, Alzheimer's disease, or Diabetes. It also could be useful for bone marrow transplants.

The present invention is particularly advantageous in respect of inflammatory diseases and autoimmune diseases that are associated with cells expressing CD40. CHIR-12.12, disclosed herein, can be used to treat patients having an inflammatory disease or autoimmune disease that is refractory to therapy with other therapeutic agents, including anti-CD20 antibodies, such as Rituxan®, as described in more detail elsewhere herein.

"Treatment" is herein defined as the application or administration of an anti-CD40 antibody to a subject, or application or administration of an anti-CD40 antibody to an isolated tissue or cell line from a subject, where the subject has an autoimmune disease and/or inflammatory disease, a symptom associated with an autoimmune disease and/or inflammatory disease, or a predisposition toward development of an autoimmune disease and/or inflammatory disease, where the purpose is to cure, heal, alleviate, relieve, alter, remedy, ameliorate, improve, or affect the autoimmune disease and/or inflammatory disease, any associated symptoms of the autoimmune disease and/or inflammatory disease, or the predisposition toward the development of the autoimmune disease and/or inflammatory disease. By "treatment" is also intended the application or administration of a pharmaceutical composition comprising an anti-CD40 antibody to a subject, or application or administration of a pharmaceutical composition comprising an anti-CD40 antibody to an isolated tissue or cell line from a subject, where the subject has an autoimmune disease and/or inflammatory disease, a symptom associated with an autoimmune disease and/or inflammatory disease, or a predisposition toward development of an autoimmune disease and/or inflammatory disease, where the purpose is to cure, heal, alleviate, relieve, alter, remedy, ameliorate, improve, or affect the autoimmune disease and/or inflammatory disease, any associated symptoms of the autoimmune disease and/or inflammatory disease, or the predisposition toward the development of the autoimmune disease and/or inflammatory disease.

By "anti-inflammatory activity" is intended a reduction or prevention of inflammation. Therapy with an anti-CD40 antibody as defined elsewhere herein causes a physiological response that is beneficial with respect to treatment of an autoimmune disease and/or inflammatory disease, where the disease involves cells expressing the CD40 antigen. It is recognized that the methods disclosed herein may be useful in preventing phenotypic change in cells such as proliferation, activation, and the like.

In the therapeutic methods of the present invention, at least one anti-CD40 antibody as defined elsewhere herein is used to promote a positive therapeutic response with respect to an inflammatory disease or autoimmune disease.

By "positive therapeutic response" with respect to an inflammatory disease and/or autoimmune disease is intended an improvement in the disease in association with the anti-inflammatory activity of the antibody, and/or an improvement in the symptoms associated with the disease. That is, an anti-proliferative effect, the prevention of further proliferation of the CD40-expressing cell, a reduction in the inflammatory response including but not limited to reduced secretion of inflammatory cytokines, adhesion molecules, proteases, immunoglobulins (in instances where the CD40 bearing cell is a B cell), combinations thereof, and the like, increased production of anti-inflammatory proteins, a reduction in the number of autoreactive cells, an increase in immune tolerance, inhibition of autoreactive cell survival, and/or a decrease in one or more symptoms mediated by stimulation of CD40-expressing cells can be observed. Such positive therapeutic responses are not limited to the route of administration and may comprise administration to the donor, the donor tissue (such as for example organ perfusion), the host, any combination thereof, and the like.

Clinical response can be assessed using screening techniques such as magnetic resonance imaging (MRI) scan, x-radiographic imaging, computed tomographic (CT) scan, flow cytometry or fluorescence-activated cell sorter (FACS) analysis, histology, gross pathology, and blood chemistry, including but not limited to changes detectable by ELISA, RIA, chromatography, and the like. In addition to these positive therapeutic responses, the subject undergoing therapy with the anti-CD40 antibody may experience the beneficial effect of an improvement in the symptoms associated with the disease.

By "therapeutically or prophylactically effective dose" or "therapeutically or prophylactically effective amount" is intended an amount of anti-CD40 antibody that, when administered brings about a positive therapeutic response with respect to treatment of a patient with an inflammatory disease or autoimmune disease associated with CD40-expressing cells. Suitable dosages are described in more detail elsewhere herein. The method of treatment may comprise a single administration of a therapeutically effective dose or multiple administrations of a therapeutically effective dose of the anti-CD40 antibody, as described in more detail elsewhere herein.

The methods disclosed herein are particularly useful for treating inflammatory diseases or autoimmune diseases, including those listed above, that are refractory to one or more known therapies for inflammatory or autoimmune diseases. Such therapies include, but are not limited to, surgery or surgical procedures (e.g. splenectomy, lymphadenectomy, thyroidectomy, plasmaphoresis, leukophoresis, cell, tissue, or organ transplantation, intestinal procedures, organ perfusion, and the like), radiation therapy, therapy such as steroid therapy and non-steroidal therapy, hormone therapy, cytokine therapy, therapy with dermatological agents (for example, topical agents used to treat skin conditions such as allergies, contact dermatitis, and psoriasis), immunosuppressive therapy, and other anti-inflammatory monoclonal antibody therapy, and the like, as descibed in more detail elsewhere herein. By "refractory" is intended the particular inflammatory disease or autoimmune disease is resistant to, or non-responsive to a particular therapy. An inflammatory disease or autoimmune disease can be refractory to a particular therapy either from the onset of treatment with the particular therapy (i.e., non-responsive to initial exposure to the therapy), or as a result of developing resistance to the therapy, either over the course of a first treatment period with the therapy or during a subsequent treatment period with the therapy. Thus, the present disclosure is useful for treating a human patient who is refractory to a therapy for inflammatory or autoimmune diseases, when that human patient is either resistant to or non-responsive to that therapy.

The methods of the present invention involve the use of anti-CD40 antibodies. "Antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy-chain variable domains. The term "variable" refers to the fact that certain portions of the variable domains differ extensively in sequence among antibodies. The variable regions confer antigen-binding specificity. The constant domains are not involved directly in binding an antibody to an antigen, but exhibit various effector functions, such as Fc receptor (FcR) binding, participation of the antibody in antibody-dependent cellular toxicity, initiation of complement dependent cytotoxicity, and mast cell degranulation.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their "heavy chains", immunoglobulins can be assigned to different classes. There are five major classes of human immunoglobulins: IgA, IgD, IgE, IgG, and IgM, and several of these may be further divided into subclasses (isotypes), e.g., IgG1, IgG2, IgG3, IgG4, IgA1, and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known. Different isotypes have different effector functions. For example, human IgG1 and IgG3 isotypes have ADCC (antibody dependent cell-mediated cytotoxicity) activity. IgG1 antibodies, in particular human IgG1 antibodies, are particularly useful in the methods of the present invention.

"Human effector cells" are leukocytes that express one or more FcRs and perform effector functions. Preferably, the cells express at least FcγRIII and carry out antigen-dependent cell-mediated cyotoxicity (ADCC) effector function. Examples of human leukocytes that mediate ADCC include peripheral blood mononuclear cells (PBMC), natural killer (NK) cells, monocytes, macrophages, eosinophils, and neutrophils, with PBMCs and NK cells being preferred. Antibodies that have ADCC activity are typically of the IgG1 or IgG3 isotype. Note that in addition to isolating IgG1 and IgG3 antibodies, such ADCC-mediating antibodies can be made by engineering a variable region from a non-ADCC antibody or variable region fragment to an IgG1 or IgG3 isotype constant region.

The terms "Fc receptor" or "FcR" are used to describe a receptor that binds to the Fc region of an antibody. The preferred FcR is a native-sequence human FcR. Moreover, a preferred FcR is one that binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor Fcγ(RIIB contains an immunoreceptor tyrosine-based inhibition motif (ITIM) in its cytoplasmic domain (see Daeron (1997) Annu. Rev. Immunol. 15:203-234). FcRs are reviewed in Ravetch and Kinet (1991) Annu. Rev. Immunol. 9:457-492 (1991); Capel et al. (1994) Immunomethods 4:25-34; and de Haas et al. (1995) J. Lab. Clin. Med 126:330-341. Other FcRs, including those to be identified in the future, are encompassed by the term "FcR" herein. The term also includes the neonatal receptor, FcRn, which is responsible for the transfer of maternal IgGs to the fetus (Guyer et al. (1976) J. Immunol. 117:587 and Kim et al. (1994) J. Immunol. 24:249 (1994)).

The term "antibody" is used herein in the broadest sense and covers fully assembled antibodies, antibody fragments which retain the ability to specifically bind to the CD40 antigen (*e.g*., Fab, F(ab')₂, Fv, and other fragments), single chain antibodies, diabodies, antibody chimeras, hybrid antibodies, bispecific antibodies, humanized antibodies, and the like), and recombinant peptides comprising the forgoing. The term "antibody" covers both polyclonal and monoclonal antibodies.

As used herein "anti-CD40 antibody" encompasses any antibody that specifically recognizes the CD40 antigen. In some embodiments, anti-CD40 antibodies for use in the methods of the present invention, in particular monoclonal anti-CD40 antibodies, exhibit a strong single-site binding affinity for the CD40 antigen. Such monoclonal antibodies exhibit an affinity for CD40 (K_{D}) of at least 10⁻⁵ M, at least 3 x 10⁻⁵ M, preferably at least 10⁻⁶ M, or at least to 10⁻⁷ M, more preferably at least 10⁻⁸ M, or at least 10⁻¹² M, when measured using a standard assay such as Biacore™. Biacore analysis is known in the art and details are provided in the "BIAapplications handbook". Methods described in WO 01/27160 can be used to modulate the binding affinity.

By "specifically recognizes" or "specifically binds to" is intended that the anti-CD40 antibody does not bind to unrelated antigens, such as the CD20 antigen.

In some embodiments, anti-CD40 antibodies for use in the methods of the present invention, in particular monoclonal antibodies, exhibit a strong binding affinity for human FcγRIIIa-158V. Preferably, an anti-CD40 antibody for use in the methods of the invention binds to human FcγRIIIa-158V with an affinity (K_{D}) of at least about 0.5 µM when measured using a standard assay such as Biacore™. As disclosed in Example 6 herein, the CHIR-12.12 antibody binds to human FcγRIIIa-158V with an affinity (K_{D}) of 492 nM.

In some embodiments, anti-CD40 antibodies for use in the methods of the present invention, in particular monoclonal antibodies, exhibit a strong binding affinity for human FcγRIIIa-158F. Preferably, an anti-CD40 antibody for use in the methods of the invention binds to human FcγRIIIa-158F with an affinity (K_{D}) of at least about 12 µM when measured using a standard assay such as Biacore™. Preferably, the anti-CD40 antibody for use in the methods of the invention binds to human FcγRIIIa-158F with an affinity (K_{D}) of at least about 10 µM, at least about 8 µM, at least about 6 µM, at least about 5 µM, at least about 4 µM, or at least about 3 µM. As disclosed in Example 6 herein, the CHIR-12.12 antibody binds to human FcγRIIIa-158F with an affinity (K_{D}) of 2.8 µM.

In some embodiments, anti-CD40 antibodies for use in the methods disclosed herein, in particular monoclonal antibodies, exhibit a strong binding affinity for both human FcγRIIIa-158V and FcγRIIIa-158F. Preferably, an anti-CD40 antibody for use in the methods of the invention binds to human FcγRIIIa-158V with an affinity (K_{D}) of at least about 0.5 µM and binds to human FcγRIIIa-158F with an affinity (K_{D}) of at least about 12 µM, when measured using a standard assay such as Biacore™_{.}

The antibodies for use in the methods of the present invention can be produced using any suitable antibody production method known to those of skill in the art.

The anti-CD40 antibody used in the methods of the present invention may be a polyclonal antibody. Thus, polyclonal sera may be prepared by conventional methods. In general, a solution containing the antigen of interest (in this case, the CD40 antigen) is first used to immunize a suitable animal, preferably a mouse, rat, rabbit, or goat. Rabbits or goats are preferred for the preparation of polyclonal sera due to the volume of serum obtainable, and the availability of labeled anti-rabbit and anti-goat antibodies.

Sera from immunized animals may be screened for antibody reactivity against the initial antigen. Lymphocytes may be isolated from lymph nodes or spleen cells and may further be selected for B cells by selecting for CD 138-negative and CD 19-positive cells. In one aspect, such B cell cultures (BCCs) may be fused to myeloma cells to generate hybridomas as detailed herein.

Polyclonal sera can also be prepared in a transgenic animal, preferably a mouse bearing human immunoglobulin loci. In a preferred embodiment, Sf9 cells expressing the protein of interest (in this case, the CD40 antigen), are used as the immunogen. Immunization can also be performed by mixing or emulsifying the antigen-containing solution in saline, preferably in an adjuvant such as Freund's complete adjuvant, and injecting the mixture or emulsion parenterally (generally subcutaneously or intramuscularly). A dose of 50-200 µg/injection is typically sufficient. Immunization is generally boosted 2-6 weeks later with one or more injections of the protein in saline, preferably using Freund's incomplete adjuvant. One may alternatively generate antibodies by *in vitro* immunization using methods known in the art, which for the purposes of this invention is considered equivalent to *in vivo* immunization. Polyclonal antisera are obtained by bleeding the immunized animal into a glass or plastic container, incubating the blood at 25°C for one hour, followed by incubating at 4°C for 2-18 hours. The serum is recovered by centrifugation (e.g., 1,000 x g for 10 minutes). About 20-50 ml per bleed may be obtained from rabbits.

Production of the Sf 9 (*Spodoptera frugiperda*) cells is disclosed in U.S. Patent No. 6,004,552. In the case of CD40, briefly, sequences encoding human CD40 were recombined into a baculovirus using transfer vectors. The plasmids were co-transfected with wild-type baculovirus DNA into Sf 9 cells. Recombinant baculovirus- infected Sf 9 cells were identified and clonally purified.

The anti-CD40 antibody used in the methods of the present invention may be a monoclonal antibody. The term "monoclonal antibody" (and "mAb") as used herein refers to an antibody obtained from a substantially homogeneous population of antibodies, i.e., the individual antibodies comprising the population are identical except for possible naturally occurring mutations that may be present in minor amounts. The term is not limited regarding the species of the antibody and does not require production of the antibody by any particular method.

In contrast to polyclonal antibody preparations, which typically include different antibodies directed against different antigenic determinants (epitopes), each monoclonal antibody is directed against a single determinant (epitope) on the antigen.

By "epitope" is intended the part of an antigenic molecule to which an antibody is produced and to which the antibody will bind. Epitopes can comprise linear amino acid residues (i.e., residues within the epitope are arranged sequentially one after another in a linear fashion), non-linear amino acid residues (referred to herein as "non-linear epitopes"; these epitopes are not arranged sequentially), or both linear and non-linear amino acid residues. An anti-CD40 monoclonal antibody suitable for use in the methods of the present invention will be capable of specifically binding to an epitope on human CD40 antigen expressed on the surface of a human cell, *i*.e. an epitope that is exposed to the exterior of the cell.

The monoclonal antibodies to be used in accordance with the present invention may be made by the hybridoma method first described by Kohler et al. (1975) Nature 256:495, or may be made by recombinant DNA methods (see, *e.g.,* U.S. Patent No. 4,816,567). Monoclonal antibodies may also be isolated from antibody phage libraries generated using the techniques described in, for example, McCafferty et al. (1990) Nature 348:552-554 (1990) and U.S. Patent No. 5,514,548. Clackson et al. (1991) Nature 352:624-628 and Marks et al. (1991) J. Mol. Biol. 222:581-597 describe the isolation of murine and human antibodies, respectively, using phage libraries. Subsequent publications describe the production of high affinity (nM range) human antibodies by chain shuffling (Marks et al. (1992) Bio/Technology 10:779-783), as well as combinatorial infection and *in vivo* recombination as a strategy for constructing very large phage libraries (Waterhouse et al. (1993) Nucleic. Acids Res. 21:2265-2266). Thus, these techniques are viable alternatives to traditional monoclonal antibody hybridoma techniques for isolation of monoclonal antibodies.

In the traditional method of Kohler et al. (1975) Nature 256:495-496, typically a mouse is immunized with a solution containing an antigen. Immunization can be performed by mixing or emulsifying the antigen-containing solution in saline, preferably in an adjuvant such as Freund's complete adjuvant, and injecting the mixture or emulsion parenterally. Any method of immunization known in the art may be used to obtain the monoclonal antibodies of the invention. After immunization of the animal, the spleen (and optionally, several large lymph nodes) are removed and dissociated into single cells. The spleen cells may be screened by applying a cell suspension to a plate or well coated with the antigen of interest. The B cells expressing membrane bound immunoglobulin specific for the antigen bind to the plate and are not rinsed away. Resulting B cells, or all dissociated spleen cells, are then induced to fuse with myeloma cells to form hybridomas, and are cultured in a selective medium. The resulting cells are plated by serial dilution and are assayed for the production of antibodies that specifically bind the antigen of interest (and that do not bind to unrelated antigens). The selected monoclonal antibody (mAb)-secreting hybridomas are then cultured either *in vitro* (e.g., in tissue culture bottles or hollow fiber reactors), or *in vivo* (as ascites in mice).

In another aspect, B cell cultures may be screened further for reactivity against the initial antigen, preferably. Such screening includes enzyme-linked immunosorbent assay (ELISA) with the target/antigen protein, a competition assay with known antibodies that bind the antigen of interest, and *in vitro* binding to transiently transfected CHO or other cells that express the target antigen.

Where anti-CD40 antibodies for use in the methods of the invention are to be prepared using recombinant DNA methods, the DNA encoding the monoclonal antibodies is readily isolated and sequenced using conventional procedures (*e.g.,* by using oligonucleotide probes that are capable of binding specifically to genes encoding the heavy and light chains of murine antibodies). The hybridoma cells described herein serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al. (1993) Curr. Opinion in Immunol. 5:256 and Phickthun (1992) Immunol. Revs. 130:151*.* Alternatively, antibody can be produced in a cell line such as a CHO cell line, as disclosed in U.S. Patent Nos. 5,545,403; 5,545,405; and 5,998,144 . Briefly the cell line is transfected with vectors capable of expressing a light chain and a heavy chain, respectively. By transfecting the two proteins on separate vectors, chimeric antibodies can be produced. Another advantage is the correct glycosylation of the antibody.

A "host cell," as used herein, refers to a microorganism or a eukaryotic cell or cell line cultured as a unicellular entity that can be, or has been, used as a recipient for a recombinant vector or other transfer polynucleotides, and include the progeny of the original cell that has been transfected. It is understood that the progeny of a single cell may not necessarily be completely identical in morphology or in genomic or total DNA complement as the original parent, due to natural, accidental, or deliberate mutation.

In some embodiments, the anti-CD40 antibody, such as CHIR-12.12, is produced in CHO cells using the GS gene expression system (Lonza Biologics, Portsmouth, New Hampshire), which uses glutamine synthetase as a marker. See, also U.S. Patent Nos. 5,122,464; 5,591,639; 5,658,759; 5,770,359; 5,827,739; 5,879,936; 5,891,693; and 5,981,216 .

Monoclonal antibodies to CD40 are known in the art. See, for example, the sections dedicated to B-cell antigen in McMichael, ed. (1987; 1989) Leukocyte Typing III and IV (Oxford University Press, New York); U.S. Patent Nos. 5,674,492; 5,874,082; 5,677,165; 6,056,959; WO 00/63395; International Publication Nos. WO 02/28905 and WO 02/28904; Gordon et al. (1988) J. Immunol. 140:1425; Valle et al. (1989) Eur. J. Immunol. 19:1463; Clark et al. (1986) PNAS 83:4494; Paulie et al. (1989) J. Immunol. 142:590; Gordon et al. (1987) Eur. J. Immunol. 17:1535; Jabara et al. (1990) J. Exp. Med. 172:1861; Zhang et al. (1991) J. Immunol. 146:1836; Gascan et al. (1991) J. Immunol. 147:8; Banchereau et al. (1991) Clin. Immunol. Spectrum 3:8; and Banchereau et al. (1991) Science 251:70 .

As noted above, the term "antibody" as used herein encompasses chimeric antibodies. By "chimeric" antibodies is intended antibodies that are most preferably derived using recombinant deoxyribonucleic acid techniques and which comprise both human (including immunologically "related" species, e.g., chimpanzee) and non-human components. Thus, the constant region of the chimeric antibody is most preferably substantially identical to the constant region of a natural human antibody; the variable region of the chimeric antibody is most preferably derived from a non-human source and has the desired antigenic specificity to the antigen of interest (CD40). The non-human source can be any vertebrate source that can be used to generate antibodies to CD40 antigen. Such non-human sources include, but are not limited to, rodents (e.g., rabbit, rat, mouse, etc.; see, for example, U.S. Patent No. 4,816,567) and non-human primates (e.g., Old World Monkey, Ape, etc.; see, for example, U.S. Patent Nos. 5,750,105 and 5,756,096).

As noted above, the term "antibody" as used herein encompasses humanized antibodies. By "humanized" is intended forms of antibodies that contain minimal sequence derived from non-human immunoglobulin sequences. For the most part, humanized antibodies are human immunoglobulins (recipient antibody) in which residues from a hypervariable region (also known as complementarity determining region or CDR) of the recipient are replaced by residues from a hypervariable region of a non-human species (donor antibody) such as mouse, rat, rabbit, or nonhuman primate having the desired specificity, affinity, and capacity. The phrase "complementarity determining region" refers to amino acid sequences which together define the binding affinity and specificity of the natural Fv region of a native immunoglobulin binding site. See, e.g., Chothia et al (1987) J. Mol. Biol. 196:901-917; Kabat *et al* (1991) U. S. Dept. of Health and Human Services, NIH Publication No. 91-3242). The phrase "constant region" refers to the portion of the antibody molecule that confers effector functions. In previous work directed towards producing non-immunogenic antibodies for use in therapy of human disease, mouse constant regions were substituted by human constant regions. The constant regions of the subject humanized antibodies were derived from human immunoglobulins. However, these humanized antibodies can elicit an unwanted and potentially dangerous immune response in humans and there was a loss of affinity.

Humanization can be performed following the method of Winter and coworkers (Jones et al. (1986) Nature 321:522-525; Riechmann et al. (1988) Nature 332:323-327; Verhoeyen et al. (1988) Science 239:1534-1536), by substituting rodent or mutant rodent CDRs or CDR sequences for the corresponding sequences of a human antibody. See also U.S. Patent Nos. 5,225,539; 5,585,089; 5,693,761; 5,693,762; 5,859,205. In some instances, residues within the framework regions of one or more variable regions of the human immunoglobulin are replaced by corresponding non-human residues (see, for example, U.S. Patent Nos. 5,585,089; 5,693,761; 5,693,762; and 6,180,370). Furthermore, humanized antibodies may comprise residues that are not found in the recipient antibody or in the donor antibody. These modifications are made to further refine antibody performance (e.g., to obtain desired affinity). In general, the humanized antibody will comprise substantially all of at least one, and typically two, variable domains, in which all or substantially all of the hypervariable regions correspond to those of a non-human immunoglobulin and all or substantially all of the framework regions are those of a human immunoglobulin sequence. The humanized antibody optionally also will comprise at least a portion of an immunoglobulin constant region (Fc), typically that of a human immunoglobulin. For further details see Jones et al. (1986) Nature 331:522-525; Riechmann et al. (1988) Nature 332:323-329; and Presta (1992) Curr. Op. Struct. Biol. 2:593-596 . Accordingly, such "humanized" antibodies may include antibodies wherein substantially less than an intact human variable domain has been substituted by the corresponding sequence from a non-human species. In practice, humanized antibodies are typically human antibodies in which some CDR residues and possibly some framework residues are substituted by residues from analogous sites in rodent antibodies. See, for example, U.S. Patent Nos. 5,225,539; 5,585,089; 5,693,761; 5,693,762; 5,859,205. See also U.S. Patent No. 6,180,370, and International Publication No. WO 01/27160, where humanized antibodies and techniques for producing humanized antibodies having improved affinity for a predetermined antigen are disclosed.

Humanized anti-CD40 antibodies can also be produced using the Human Engineering™ technology (Xoma Ltd., Berkeley, California).

Humanized anti-CD40 monoclonal antibodies include antibodies such as SGN-40 (Tai et al. (2004) Cancer Res. 64:2846-52; U.S. Patent No. 6,838,261), which is the humanized form of the murine anti-CD40 antibody SGN-14 (Francisco et al. (2000) Cancer Res. 60:3225-31), and the antibodies disclosed in U.S. Patent Application Publication No. 2004/0120948 .

The present invention can also be practiced using xenogeneic or modified antibodies produced in a non-human mammalian host, more particularly a transgenic mouse, characterized by inactivated endogenous immunoglobulin (Ig) loci. In such transgenic animals, competent endogenous genes for the expression of light and heavy subunits of host immunoglobulins are rendered non-functional and substituted with the analogous human immunoglobulin loci. These transgenic animals produce human antibodies in the substantial absence of light or heavy host immunoglobulin subunits. See, for example, U.S. Patent Nos. 5,877,397 and 5,939,598 .

Thus, in some embodiments, fully human antibodies to CD40, for example, are obtained by immunizing transgenic mice. One such mouse is obtained using XenoMouse^{®} technology (Abgenix; Fremont, California), and is disclosed in U.S. Patent Nos. 6,075,181, 6,091,001, and 6,114,598 . For example, to produce the CHIR-12.12 antibody, mice transgenic for the human Ig G₁ heavy chain locus and the human κ light chain locus were immunized with Sf 9 cells expressing human CD40. Mice can also be transgenic for other isotypes. Fully human anti-CD40 antibodies useful in the methods of the present invention are characterized by binding properties similar to those exhibited by the CHIR-12.12 monoclonal antibody.

As noted above, the term "antibody" as used herein also encompasses antibody fragments that can bind antigen. "Antibody fragments" comprise a portion of an intact antibody, preferably the antigen-binding or variable region of the intact antibody. Examples of antibody fragments include Fab, Fab, F(ab')2, and Fv fragments; diabodies; linear antibodies (Zapata et al. (1995) Protein Eng. 10:1057-1062); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen.

"Fv" is the minimum antibody fragment that contains a complete antigen recognition and binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (C_{H}1) of the heavy chain. Fab fragments differ from Fab' fragments by the addition of a few residues at the carboxy terminus of the heavy chain C_{H}1 domain including one or more cysteines from the antibody hinge region. Fab'-SH is the designation herein for Fab' in which the cysteine residue(s) of the constant domains bear a free thiol group. Fab' fragments are produced by reducing the F(ab')2 fragment's heavy chain disulfide bridge. Other chemical couplings of antibody fragments are also known.

Fragments of an anti-CD40 antibody are suitable for use in the methods of the invention so long as they retain the desired affinity of the full-length antibody. Thus, for example, a fragment of an anti-CD40 antibody will retain the ability to bind to the CD40 antigen. Such fragments are characterized by properties similar to the corresponding full-length antibody. Thus, for example, a fragment of a full-length antagonist anti-CD40 antibody will preferably be capable of specifically binding a human CD40 antigen expressed on the surface of a human cell, and is free of significant agonist activity but exhibits antagonist activity when bound to a CD40 antigen on a human CD40-expressing cell. Such fragments are referred to herein as "antigen-binding" fragments. Fragments of an anti-CD40 antibody for use in the methods of the invention will also preferably retain the ability to bind to the relevant FcR or FcRs. Thus, for example, a fragment of an anti-CD40 antibody may retain the ability to bind to FcγRIIIa. Thus, for example, a fragment of a full-length anti-CD40 antibody may be capable of binding specifically to a cell-surface CD40 antigen, and also capable of binding to FcγRIIIa on human effector cells, such as natural killer (NK) cells. Such fragments are referred to herein as "FcR-binding" fragments. Such fragments will generally include at least part of the constant domain of the heavy chain.

Various techniques have been developed for the production of antibody fragments. Traditionally, these fragments were derived *via* proteolytic digestion of intact antibodies (see, e.g., Morimoto et al. (1992) Journal of Biochemical and Biophysical Methods 24:107-117 (1992) and Brennan et al. (1985) Science 229:81). However, these fragments can now be produced directly by recombinant host cells. For example, the antibody fragments can be isolated from the antibody phage libraries discussed above. Alternatively, Fab'-SH fragments can be directly recovered from *E*. *coli* and chemically coupled to form F(ab')₂ fragments (Carter et al. (1992) Bio/Technology 10:163-167). According to another approach, F(ab')₂ fragments can be isolated directly from recombinant host cell culture. Other techniques for the production of antibody fragments will be apparent to the skilled practitioner.

Suitable antigen-binding fragments of an antibody comprise a portion of a full-length antibody, generally the antigen-binding or variable region thereof. Examples of antibody fragments include, but are not limited to, Fab, F(ab')₂, and Fv fragments and single-chain antibody molecules. By "Fab" is intended a monovalent antigen-binding fragment of an immunoglobulin that is composed of the light chain and part of the heavy chain. By F(ab')₂ is intended a bivalent antigen-binding fragment of an immunoglobulin that contains both light chains and part of both heavy chains. By "single-chain Fv" or "sFv" antibody fragments is intended fragments comprising the V_{H} and V_{L} domains of an antibody, wherein these domains are present in a single polypeptide chain. See, for example, U.S. Patent Nos. 4,946,778, 5,260,203, 5,455,030, and 5,856,456. Generally, the Fv polypeptide further comprises a polypeptide linker between the V_{H} and V_{L} domains that enables the sFv to form the desired structure for antigen binding. For a review of sFv see Pluckthun (1994) in The Pharmacology of Monoclonal Antibodies, Vol. 113, ed. Rosenburg and Moore (Springer-Verlag, New York), pp. 269-315. Antigen-binding fragments of the antagonist anti-CD40 antibodies disclosed herein can also be conjugated to a cytotoxin to effect killing of the target cells, as described herein below.

In some embodiments of the invention, the anti-CD40 antibody is an antagonist anti-CD40 antibody. When such antibodies bind CD40 displayed on the surface of human cells, such as human B cells, they do not cause significant agonist activity. In some embodiments, their binding to CD40 displayed on the surface of human cells results in inhibition of proliferation and differentiation of these human cells. The anti-CD40 antibodies suitable for use in the methods of the invention include those antibodies that can exhibit antagonist activity toward normal and malignant human cells expressing the cell-surface CD40 antigen.

By "agonist activity" is intended that a substance functions as an agonist. An agonist combines with a receptor on a cell and initiates a reaction or activity that is similar to or the same as that initiated by the receptor's natural ligand. An agonist of CD40 induces any or all of, but not limited to, the following responses: B cell proliferation and/or differentiation; upregulation of intercellular adhesion via such molecules as ICAM-1, E-selectin, VCAM, and the like; secretion of pro-inflammatory cytokines such as IL-1, IL-6, IL-8, IL-12, TNF, and the like; signal transduction through the CD40 receptor by such pathways as TRAF (e.g., TRAF2 and/or TRAF3), MAP kinases such as NIK (NF-κB inducing kinase), I-kappa B kinases (IKK α/β), transcription factor NF-κB, Ras and the MEK/ERK pathway, the PI3K/AKT pathway, the P38 MAPK pathway, and the like; transduction of an anti-apoptotic signal by such molecules as XIAP, mcl-1, bcl-x, and the like; B and/or T cell memory generation; B cell antibody production; B cell isotype switching, up-regulation of cell-surface expression of MHC Class II and CD80/86, and the like.

By "significant" agonist activity is intended an agonist activity of at least 30%, 35%, 40%, 45%, 50%, 60%, 70%, 75%, 80%, 85%, 90%, 95%, or 100% greater than the agonist activity induced by a neutral substance or negative control as measured in an assay of a B cell response. Preferably, "significant" agonist activity is an agonist activity that is at least 2-fold greater or at least 3-fold greater than the agonist activity induced by a neutral substance or negative control as measured in an assay of a B cell response. Thus, for example, where the B cell response of interest is B cell proliferation, "significant" agonist activity would be induction of a level of B cell proliferation that is at least 2-fold greater or at least 3-fold greater than the level of B cell proliferation induced by a neutral substance or negative control. In one embodiment, a non-specific immunoglobulin, for example IgG1, that does not bind to CD40 serves as the negative control. A substance "free of significant agonist activity" would exhibit an agonist activity of not more than about 25% greater than the agonist activity induced by a neutral substance or negative control, preferably not more than about 20% greater, 15% greater, 10% greater, 5% greater, 1% greater, 0.5% greater, or even not more than about 0.1% greater than the agonist activity induced by a neutral substance or negative control as measured in an assay of a B cell response.

By "antagonist activity" is intended that the substance functions as an antagonist. An antagonist of CD40 prevents or reduces induction of any of the responses induced by binding of the CD40 receptor to an agonist ligand, particularly CD40L. The antagonist may reduce induction of any one or more of the responses to agonist binding by 5%, 10%, 15%, 20%, 25%, 30%, 35%, preferably 40%, 45%, 50%, 55%, 60%, more preferably 70%, 80%, 85%, and most preferably 90%, 95%, 99%, or 100%. Methods for measuring CD40 ligand binding specificity and antagonist activity of an anti-CD40 therapeutic agent, for example, an anti-CD40 antibody, are known in the art and include, but are not limited to, standard competitive binding assays, assays for monitoring immunoglobulin secretion by B cells, B cell proliferation assays, Banchereau-Like-B cell proliferation assays, T cell helper assays for antibody production, co-stimulation of B cell proliferation assays, and assays for up-regulation of B cell activation markers. See, for example, such assays disclosed in WO 00/75348 and U.S. Patent No. 6,087,329 . Also see WO 2005/044854, WO 2005/044304, WO 2005/044305, WO 2005/044306, WO 2005/044855, WO 2005/044307, and WO 2005/044294WO.

Antagonist/lack of agonist activity can be evaluated by assays showing that CHIR-12.12 lacks agonist activity. Suitable assays are shown in the assays described in US 5677165 (Chiron Corporation).

In one embodiment of the invention, the antagonist anti-CD40 antibody is free of significant agonist activity in one cellular response. In another embodiment of the invention, the antagonist anti-CD40 antibody is free of significant agonist activity in assays of more than one cellular response (e.g., proliferation and differentiation, or proliferation, differentiation, and, for B cells, antibody production).

Of particular interest are antagonist anti-CD40 antibodies that are free of significant agonist activity as defined herein but exhibit antagonist activity when bound to CD40 antigen on human B cells. In one embodiment of the invention, the antagonist anti-CD40 antibody is free of significant agonist activity in one B cell response. In another embodiment of the invention, the antagonist anti-CD40 antibody is free of significant agonist activity in assays of more than one B cell response (e.g., proliferation and differentiation, or proliferation, differentiation, and antibody production).

Any of the assays known in the art can be used to determine whether an anti-CD40 antibody acts as an antagonist of one or more B cell responses. In some embodiments, the anti-CD40 antibody acts as an antagonist of at least one B cell response selected from the group consisting of B cell proliferation, B cell differentiation, antibody production, intercellular adhesion, B cell memory generation, isotype switching, up-regulation of cell-surface expression of MHC Class II and CD80/86, and secretion of pro-inflammatory cytokines such as IL-8, IL-12, and TNF. Of particular interest are antagonist anti-CD40 antibodies that free of significant agonist activity with respect to B cell proliferation when bound to the human CD40 antigen on the surface of a human B cell.

In one such embodiment, the anti-CD40 antibody is an antagonist of B cell proliferation induced by soluble or cell-surface CD40L, as measured in a B cell proliferation assay. Suitable B cell proliferation assays are known in the art. Preferably, the antagonist anti-CD40 antibody stimulates B cell proliferation at a level that is not more than about 25% greater than the B cell proliferation induced by a neutral substance or negative control, preferably not more than about 20% greater, 15% greater, 10% greater, 5% greater, 1% greater, 0.5% greater, or even not more than about 0.1 % greater than the B cell proliferation induced by a neutral substance or negative control.

In other embodiments, the anti-CD40 antibody is an antagonist of B cell proliferation that is induced by another anti-CD40 antibody, for example, the S2C6 anti-CD40 antibody, as measured in a B cell proliferation, and the level of B cell proliferation stimulated by the other anti-CD40 antibody in the presence of the antagonist anti-CD40 antibody is not more than about 25% of the B cell proliferation induced by the other anti-CD40 antibody in the absence of the antagonist anti-CD40 antibody (i.e., at least 75% inhibition), preferably not more than about 20%, 15%, 10%, 5%, 1%, 0.5%, or even not more than about 0.1% of the B cell proliferation induced by the other anti-CD40 antibody in the absence of the antagonist anti-CD40 antibody.

In yet other embodiments, the anti-CD40 antibody is an antagonist of B cell proliferation that is induced by the cell line EL4B5 as measured in a B cell activation assay, and the level of B cell proliferation stimulated by the EL4B5 cell line in the presence of the antagonist anti-CD40 antibody is not more than about 25% of the B cell proliferation induced by this cell line in the absence of the antagonist anti-CD40 antibody (i.e., at least 75% inhibition), preferably not more than about 20%, 15%, 10%, 5%, 1%, 0.5%, or even not more than about 0.1 % of the B cell proliferation induced by this cell line in the absence of the antagonist anti-CD40 antibody.

In still other embodiments, the anti-CD40 antibody is an antagonist of human T-cell-induced antibody production by human B cells as measured in the human T-cell helper assay for antibody production by B cells. In this manner, the level of IgG antibody production, IgM antibody production, or both IgG and IgM antibody production by B cells stimulated by T cells in the presence of the antagonist anti-CD40 antibody is not more than about 50% of the respective antibody production by B cells stimulated by T cells in the absence of the antagonist anti-CD40 antibody (i.e., at least 75% inhibition), preferably not more than about 25%, 20%, 15%, 10%, 5%, 1%, 0.5%, or even not more than about 0.1% of the respective antibody production by B cells stimulated by T cells in the absence of the antagonist anti-CD40 antibody. Additional antagonist anti-CD40 antibodies include the monoclonal antibodies referred to as 5D12, 3A8 and 3C6, which are secreted by a hybridoma having ATCC accession numbers HB 11339, HB 12024 and HB 11340, respectively. See, for example, U.S. Patent No. 6,315,998.

Antagonist anti-CD40 antibodies are known in the art. See, for example, the human anti-CD40 antibody produced by the hybridoma designated F4-465 disclosed in U.S. Patent Application Publication Nos. 20020142358 and 20030059427 . F4-465 was obtained from the HAC mouse (Kuroiwa et al. (2000) Nature Biotech. 10:1086 (2000)) and therefore expresses the human lambda light chain. Also see WO 2005/044854, WO 2005/044304, WO 2005/044305, WO 2005/044306, WO 2005/044855, WO 2005/044307, and WO 2005/044294WO .

In addition to antagonist activity, the anti-CD40 antibody for use in the methods of the present invention will preferably have another mechanism of action against a target cell. For example, the anti-CD40 antibody will preferably have ADCC activity. Alternatively, the variable regions of the anti-CD40 antibody can be expressed on another antibody isotype that has ADCC activity. It is also possible to conjugate native forms, recombinant forms, or antigen-binding fragments of anti-CD40 antibodies to a cytotoxin, a therapeutic agent, or a radioactive metal ion or radioisotope, as described further elsewhere herein.

As explained elsewhere herein, the inventors have made the surprising finding that, contrary to other antibodies, anti-CD40 antibodies, such as CHIR-12.12, are able to mediate potent antibody-dependent cellular cytotoxicity (ADCC) of CD40-expressing target cells via binding to either of the two FcγRIIIa amino acid 158 allotypes (V or F) on a human patient's natural killer (NK) cells. Accordingly, anti-CD40 antibodies, such as CHIR-12.12, can be used in the treatment of inflammatory diseases and autoimmune diseases associated with CD40-expressing cells in human patients heterozygous or homozygous for FcγRIIIa-158F (genotype V/F or F/F), in addition to human patients homozygous for FcγRIIIa-158V (genotype V/V). The present disclosure is especially advantageous for the treatment of inflammatory diseases and autoimmune diseases that are not responsive to treatment with rituximab (Rituxan®), because the clinical activity of rituximab in NHL has been shown to be correlated with the patient's FcγRIIIa genotype.

Thus, particularly preferred anti-CD40 antibodies for use in the methods of the present invention are those which, in addition to antagonist activity, are capable of mediating ADCC of CD40-expressing cells by human effector cells, such as natural killer cells (NK cells) expressing FcγRIIIa. Most preferred are those anti-CD40 antibodies that are capable of binding both FcγRIIIa-158F and FcγRIIIa-158V with high affinity, as described further elsewhere herein.

Particularly preferred anti-CD40 antibodies are those disclosed in WO 2005/044854, WO 2005/044304, WO 2005/044305, WO 2005/044306, WO 2005/044855, WO 2005/044307, and WO 2005/044294WO .

Of particular interest to the present invention are antagonist anti-CD40 antibodies that share the binding characteristics of the CHIR-12.12 monoclonal antibody described in WO 2005/044854, WO 2005/044304, WO 2005/044305, WO 2005/044306, WO 2005/044855, WO 2005/044307, and WO 2005/044294. Such antibodies include, but are not limited to the following:
a) the monoclonal antibody CHIR-12.12;
b) the monoclonal antibody produced by the hybridoma cell line 12.12;
c) a monoclonal antibody comprising an amino acid sequence selected from the group consisting of the sequence shown in SEQ ID NO:2, the sequence shown in SEQ ID NO:4, the sequence shown in SEQ ID NO:5, both the sequences shown in SEQ ID NO:2 and SEQ ID NO:4, and both the sequences shown in SEQ ID NO:2 and SEQ ID NO:5;
d) a monoclonal antibody having an amino acid sequence encoded by a nucleic acid molecule comprising a nucleotide sequence selected from the group consisting of the sequence shown in SEQ ID NO:1, the sequence shown in SEQ ID NO:3, and both the sequences shown in SEQ ID NO:1 and SEQ ID NO:3;
e) a monoclonal antibody that binds to an epitope capable of binding the monoclonal antibody produced by the hybridoma cell line 12.12;
f) a monoclonal antibody that binds to an epitope comprising residues 82-87 of the human CD40 sequence shown in SEQ ID NO:7 or SEQ ID NO:9;
g) a monoclonal antibody that binds to an epitope comprising residues 82-89 of the human CD40 sequence shown in SEQ ID NO:7 or SEQ ID NO:9;
h) a monoclonal antibody that competes with the monoclonal antibody CHIR-12.12 in a competitive binding assay;
i) the monoclonal antibody of preceding item a) or a monoclonal antibody of any one of preceding items c)-h), wherein said antibody is recombinantly produced; and
j) a monoclonal antibody that is an antigen-binding fragment of a monoclonal antibody of any one of preceding items a)-i), wherein said fragment retains the capability of specifically binding to said human CD40 antigen.

The monoclonal antibody CHIR-12.12 is particularly preferred for use in the methods of the present invention.

The monoclonal antibody CHIR-12.12 was described in detail in WO 2005/044854, WO 2005/044304, WO 2005/044305, WO 2005/044306, WO 2005/044855, WO 2005/044307, and WO 2005/044294. The CHIR-12.12 antibody is a fully human anti-CD40 monoclonal antibody of the IgG₁ isotype produced from the hybridoma cell line 153.8E2.D10.D6.12.12 (referred to as the cell line 12.12). The cell line was created using splenocytes from immunized xenotypic mice containing the human IgG₁ heavy chain locus and the human κ chain locus (XenoMouse^{®} technology; Abgenix; Fremont, California). The spleen cells were fused with the mouse myeloma SP2/0 cells (Sierra BioSource). The resulting hybridomas were sub-cloned several times to create the stable monoclonal cell line 12.12. Other antibodies suitable for use in the methods of the invention may be prepared similarly using mice transgenic for human immunoglobulin loci, as described elsewhere herein.

The CHIR-12.12 monoclonal antibody binds soluble CD40 in ELISA-type assays, prevents the binding of CD40-ligand to cell-surface CD40, and displaces the pre-bound CD40-ligand, as determined by flow cytometric assays. Antibodies CHIR-5.9 and CHIR-12.12 compete with each other for binding to CD40 but not with 15B8, the anti-CD40 monoclonal antibody described in U.S. Provisional Application Serial No. 60/237,556, titled "*Human Anti-CD40 Antibodies*," filed October 2, 2000, and PCT International Application No. PCT/US01/30857, also titled "*Human Anti-CD40 Antibodies*," filed October 2, 2001 (Attorney Docket No. PP16092.003) and published as WO 2002/028904 . When tested *in vitro* for effects on proliferation of B cells from normal human subjects, CHIR-12.12 acts as antagonist anti-CD40 antibody. Furthermore, CHIR-12.12 does not induce strong proliferation of human lymphocytes from normal subjects. The antibody is able to kill CD40-expressing target cells by antibody dependent cellular cytotoxicity (ADCC). The binding affinity of CHIR-12.12 for human CD40 is 5x10⁻¹⁰ M, as determined by the Biacore™ assay.

The nucleotide and amino acid sequences of the variable regions of the CHIR-12.12 antibody are provided herein. More particularly, the amino acid sequences for the leader, variable, and constant regions for the light chain and heavy chain for mAb CHIR-12.12 are set forth in SEQ ID NO:2 (complete sequence for the light chain of mAb CHIR-12.12), SEQ ID NO:4 (complete sequence for the heavy chain for mAb CHIR-12.12), and SEQ ID NO:5 (complete sequence for a variant of the heavy chain for mAb CHIR-12.12 set forth in SEQ ID NO:4, where the variant comprises a serine substitution for the alanine residue at position 153 of SEQ ID NO:4). The nucleotide sequences encoding the light chain and heavy chain for mAb CHIR-12.12 are set forth in SEQ ID NO:1 (coding sequence for the light chain for mAb CHIR-12.12) and SEQ ID NO:3 (coding sequence for the heavy chain for mAb CHIR-12.12). Hybridomas expressing the CHIR-12.12 antibody have been deposited with the ATCC with a patent deposit designation of PTA-5543.

Anti-CD40 antibodies for use in the methods of the present invention include antibodies differing from the CHIR-12.12 monoclonal antibody but retaining the CDRs, and antibodies with one or more amino acid addition(s), deletion(s), or substitution(s). The anti-CD40 antibodies for use in the methods of the present invention may also be de-immunized antibodies, particularly de-immunized antagonist anti-CD40 antibodies, which can be produced as described in, for example, International Publication Nos. WO 98/52976 and WO 0034317 . In this manner, residues within the antagonist anti-CD40 antibodies of the invention are modified so as to render the antibodies non- or less immunogenic to humans while retaining their antagonist activity toward human CD40-expressing cells, wherein such activity is measured by assays noted elsewhere herein. Also included within the scope of the present invention are fusion proteins comprising an antibody of interest, for example, an antagonist anti-CD40 antibody or an antagonist anti-CD40L antibody, or a fragment thereof, which fusion proteins can be synthesized or expressed from corresponding polynucleotide vectors, as is known in the art. Such fusion proteins are described with reference to conjugation of antibodies as noted elsewhere herein.

Any known antibody having the binding specificity of interest can have sequence variations produced using methods described in, for example, Patent Publication Nos. EP 0983303 A1, WO 00/34317, and WO 98/52976 . For example, it has been shown that sequences within the CDR can cause an antibody to bind to MHC Class II and trigger an unwanted helper T-cell response. A conservative substitution can allow the antibody to retain binding activity yet lose its ability to trigger an unwanted T-cell response. Any such conservative or non-conservative substitutions can be made using art-recognized methods, such as those noted elsewhere herein, and the resulting antibodies can also be used in the methods of the present invention. The variant antibodies can be routinely tested for the particular activity, for example, antagonist activity, affinity, and specificity using methods described herein.

For example, amino acid sequence variants of an antagonist anti-CD40 antibody, for example, the CHIR-12.12 monoclonal antibody, can be prepared by mutations in the cloned DNA sequence encoding the antibody of interest. Methods for mutagenesis and nucleotide sequence alterations are well known in the art. See, for example, Walker and Gaastra, eds. (1983) Techniques in Molecular Biology (MacMillan Publishing Company, New York); Kunkel (1985) Proc. Natl. Acad Sci. USA 82:488-492; Kunkel et al. (1987) Methods Enzymol. 154:367-382; Sambrook et al. (1989) Molecular Cloning: A Laboratory Manual (Cold Spring Harbor, New York); U.S. Patent No. 4,873,192; and the references cited therein . Guidance as to appropriate amino acid substitutions that do not affect biological activity of the polypeptide of interest may be found in the model of Dayhoff et al. (1978) in Atlas of Protein Sequence and Structure (Natl. Biomed. Res. Found., Washington, D.C.). Conservative substitutions, such as exchanging one amino acid with another having similar properties, may be preferred. Examples of conservative substitutions include, but are not limited to, Gly⇔Ala, Val⇔Ile⇔Leu, Asp⇔Glu, Lys⇔Arg, Asn⇔Gln, and Phe⇔Trp⇔Tyr.

In constructing variants of an antibody of interest, for example, an antagonist anti-CD40 antibody polypeptide of interest, modifications are made such that variants continue to possess the desired activity, i.e., similar binding affinity and, in the case of antagonist anti-CD40 antibodies, are capable of specifically binding to a human CD40 antigen expressed on the surface of a human cell, and being free of significant agonist activity but exhibiting antagonist activity when bound to a CD40 antigen on a human CD40-expressing cell. Obviously, any mutations made in the DNA encoding the variant polypeptide must not place the sequence out of reading frame and preferably will not create complementary regions that could produce secondary mRNA structure. See EP patent application Publication No. 75,444.

In addition, the constant region of an antibody, for example, an antagonist anti-CD40 antibody, can be mutated to alter effector function in a number of ways. For example, see U.S. Patent No. 6,737,056B1 and U.S. Patent Application Publication No. 2004/0132101A1, which disclose Fc mutations that optimize antibody binding to Fc receptors.

Preferably, variants of a reference antibody, for example, an antagonist anti-CD40 antibody, have amino acid sequences that have at least 70% or 75% sequence identity, preferably at least 80% or 85% sequence identity, more preferably at least 90%, 91%, 92%, 93%, 94% or 95% sequence identity to the amino acid sequence for the reference antibody, for example, an antagonist anti-CD40 antibody molecule, for example, the CHIR-12.12 monoclonal antibody described herein, or to a shorter portion of the reference antibody molecule. More preferably, the molecules share at least 96%, 97%, 98% or 99% sequence identity. For purposes of the present invention, percent sequence identity is determined using the Smith-Waterman homology search algorithm using an affine gap search with a gap open penalty of 12 and a gap extension penalty of 2, BLOSUM matrix of 62. The Smith-Waterman homology search algorithm is taught in Smith and Waterman (1981) Adv. Appl. Math. 2:482-489. A variant may, for example, differ from the reference antibody, for example, an antagonist anti-CD40 antibody, by as few as 1 to 15 amino acid residues, as few as 1 to 10 amino acid residues, such as 6-10, as few as 5, as few as 4, 3, 2, or even 1 amino acid residue.

With respect to optimal alignment of two amino acid sequences, the contiguous segment of the variant amino acid sequence may have additional amino acid residues or deleted amino acid residues with respect to the reference amino acid sequence. The contiguous segment used for comparison to the reference amino acid sequence will include at least 20 contiguous amino acid residues, and may be 30, 40, 50, or more amino acid residues. Corrections for sequence identity associated with conservative residue substitutions or gaps can be made (see Smith-Waterman homology search algorithm).

The precise chemical structure of a polypeptide capable of specifically binding CD40 and retaining antagonist activity, particularly when bound to CD40 antigen on target cells, depends on a number of factors. As ionizable amino and carboxyl groups are present in the molecule, a particular polypeptide may be obtained as an acidic or basic salt, or in neutral form. All such preparations that retain their biological activity when placed in suitable environmental conditions are included in the definition of antagonist anti-CD40 antibodies as used herein. Further, the primary amino acid sequence of the polypeptide may be augmented by derivatization using sugar moieties (glycosylation) or by other supplementary molecules such as lipids, phosphate, acetyl groups and the like. It may also be augmented by conjugation with saccharides. Certain aspects of such augmentation are accomplished through post-translational processing systems of the producing host; other such modifications may be introduced *in vitro.* In any event, such modifications are included in the definition of an anti-CD40 antibody used herein so long as the antagonist properties of the anti-CD40 antibody are not destroyed. It is expected that such modifications may quantitatively or qualitatively affect the activity, either by enhancing or diminishing the activity of the polypeptide, in the various assays. Further, individual amino acid residues in the chain may be modified by oxidation, reduction, or other derivatization, and the polypeptide may be cleaved to obtain fragments that retain activity. Such alterations that do not destroy antagonist activity do not remove the polypeptide sequence from the definition of anti-CD40 antibodies of interest as used herein.

The art provides substantial guidance regarding the preparation and use of polypeptide variants. In preparing the anti-CD40 antibody variants, one of skill in the art can readily determine which modifications to the native protein nucleotide or amino acid sequence will result in a variant that is suitable for use as a therapeutically active component of a pharmaceutical composition used in the methods of the present invention.

The anti-CD40 antibody for use in the methods of the invention preferably possesses at least one of the following biological activities *in vitro* and/or *in vivo:* inhibition of immunoglobulin secretion by normal human peripheral B cells stimulated by T cells; inhibition of survival and/or proliferation of normal human peripheral B cells stimulated by CD40L-expressing cells or soluble CD40 ligand (sCD40L); inhibition of survival and/or proliferation of normal human peripheral B cells stimulated by Jurkat T cells; inhibition of "survival" anti-apoptotic intracellular signals in any cell stimulated by sCD40L or solid-phase CD40L; and, inhibition of CD40 signal transduction in any cell upon ligation with sCD40L or solid-phase CD40L, deletion, anergy and/or tolerance induction of CD40-bearing target cells or cells bearing cognate ligands to CD40 including, but not limited to, T cells and B cells, induction of expansion or activation of CD4⁺CD25⁺ regulatory T cells (see for example, donor alloantigen-specific tissue rejection via CD40-CD40L interference, van Maurik et al. (2002) J. Immunol. 169:5401-5404), cytotoxicity via any mechanism (including, but not limited to, antibody-dependent cell-mediated cytotoxicity (ADCC), complement-dependent cytotoxicity (CDC), down-regulation of proliferation, and/or apoptosis in target cells), modulation of target cell cytokine secretion and/or cell surface molecule expression, and combinations thereof.

Assays for such biological activities can be performed as described herein and in provisional applications entitled *"Antagonist Anti-CD40 Monoclonal Antibodies and Methods for Their Use,"* filed November 4, 2003, November 26, 2003, and April 27, 2004, and assigned U.S. Patent Application Nos. 60/517,337 (Attorney Docket No. PP20107.001 (035784/258442)), 60/525,579 (Attorney Docket No. PP20107.002 (035784/271525)), and 60/565,710 (Attorney Docket No. PP20107.003 (035784/277214)), respectively; and International Patent Application No. PCT/US2004/037152 (Attorney Docket No. PP20107.004 (035784/282916)), published as WO 2005/044854, also entitled *"Antagonist Anti-CD40 Monoclonal Antibodies and Methods for Their Use,"* filed November 4, 2004 . See also the assays described in Schultze et al. (1998) Proc. Natl. Acad. Sci. USA 92:8200-8204; Denton et al. (1998) Pediatr. Transplant. 2:6-15; Evans et al. (2000) J. Immunol. 164:688-697; Noelle (1998) Agents Actions Suppl. 49:17-22; Lederman et al. (1996) Curr. Opin. Hematol. 3:77-86; Coligan et al. (1991) Current Protocols in Immunology 13:12; Kwekkeboom et al. (1993) Immunology 79:439-444; and U.S. Patent Nos. 5,674,492 and 5,847,082.

A representative assay to detect antagonist anti-CD40 antibodies specific to the CD40-antigen epitopes identified herein is a "competitive binding assay." Competitive binding assays are serological assays in which unknowns are detected and quantitated by their ability to inhibit the binding of a labeled known ligand to its specific antibody. This is also referred to as a competitive inhibition assay. In a representative competitive binding assay, labeled CD40 polypeptide is precipitated by candidate antibodies in a sample, for example, in combination with monoclonal antibodies raised against one or more epitopes of the monoclonal antibodies of the invention. Anti-CD40 antibodies that specifically react with an epitope of interest can be identified by screening a series of antibodies prepared against a CD40 protein or fragment of the protein comprising the particular epitope of the CD40 protein of interest. For example, for human CD40, epitopes of interest include epitopes comprising linear and/or nonlinear amino acid residues of the short isoform of human CD40 (see GenBank Accession No. NP_690593) set forth in SEQ ID NO:10, encoded by the sequence set forth SEQ ID NO:9; see also GenBank Accession No. NM_152854), or of the long isoform of human CD40 (see GenBank Accession Nos. CAA43045 and NP_001241, set forth in SEQ ID NO:12, encoded by the sequence set forth in SEQ ID NO:11; see GenBank Accession Nos. X60592 and NM_001250). Alternatively, competitive binding assays with previously identified suitable antagonist anti-CD40 antibodies could be used to select monoclonal antibodies comparable to the previously identified antibodies.

Antibodies employed in such immunoassays may be labeled or unlabeled. Unlabeled antibodies may be employed in agglutination; labeled antibodies may be employed in a wide variety of assays, employing a wide variety of labels. Detection of the formation of an antibody-antigen complex between an anti-CD40 antibody and an epitope of interest can be facilitated by attaching a detectable substance to the antibody. Suitable detection means include the use of labels such as radionuclides, enzymes, coenzymes, fluorescers, chemiluminescers, chromogens, enzyme substrates or co-factors, enzyme inhibitors, prosthetic group complexes, free radicals, particles, dyes, and the like. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material is luminol; examples of bioluminescent materials include luciferase, luciferin, and aequorin; and examples of suitable radioactive material include ¹²⁵I, ¹³¹I, ³⁵S, or ³H. Such labeled reagents may be used in a variety of well-known assays, such as radioimmunoassays, enzyme immunoassays, e.g., ELISA, fluorescent immunoassays, and the like. See for example, U.S. Patent Nos. 3,766,162; 3,791,932; 3,817,837; and 4,233,402.

It is also possible to engineer an antibody to have increased ADCC activity. In particular, the carboxy-terminal half of the CH2 domain is critical to ADCC mediated through the FcRIII receptor. Since the CH2 and hinge regions have an important role in effector functions, a series of multiple-domain antibodies that contain extra CH2 and/or hinge regions may be created and investigated for any changes in effector potency (see Greenwood, J., Gorman, S. D., Routledge, E.G., Lloyd, I.S. & Waldmann , H., Ther Immunol. 1994 Oct;1 1(5):247-55). An alternative approach may be to engineer extra domains in parallel, for example, through creation of dimers by engineering a cysteine into the H-chain of a chimeric Ig (see Shopes B. (1992) J. Immunol. 1992 1; 148(9): 2918-22). Furthermore, changes to increase ADCC activity may be engineered by introducing mutations into the Fc region (see, for example, US 6,737,056 B1), expressing cells in fucosyl transferase deficient cell lines (see, for example, US2003/0115614), or effecting other changes to antibody glycosylation (see, for example, US 6,602,684).

The present invention is especially advantageous for the treatment of inflammatory diseases and autoimmune diseases that are associated with CD20-expressing cells, particularly in Rituxan® resistant patients, more particularly in those that are heterozygous or homozygous for FcγRIIIa-158F (genotype V/F or F/F).

As used herein, "anti-CD20 antibody" encompasses any antibody that specifically recognizes the CD20 cell surface antigen, including polyclonal antibodies, monoclonal antibodies, single-chain antibodies, and fragments thereof such as Fab, F(ab')₂, Fᵥ, and other fragments that retain the antigen-binding function of the parent anti-CD20 antibody. Of particular interest in connection with the methods of the present invention are anti-CD20 antibodies or antigen-binding fragments thereof that have the binding properties exhibited by the IDEC-C2B8 monoclonal antibody (Biogen IDEC Inc., Cambridge, MA).

In some embodiments, the anti-CD40 antibodies used in the methods of the invention exhibit more potent therapeutic activity than the chimeric anti-CD20 monoclonal antibody IDEC-C2B8, where therapeutic activity is assayed with equivalent amounts of these antibodies in an appropriate experimental model. IDEC-C2B8 (IDEC Pharmaceuticals Corp., San Diego, California; commercially available under the tradename Rituxan®, also referred to as rituximab) is a chimeric anti-CD20 monoclonal antibody containing human IgG1 and kappa constant regions with murine variable regions isolated from a murine anti-CD20 monoclonal antibody, IDEC-2B8 (Reff et al. (1994) Blood 83:435-445). Rituximab is licensed for treatment of relapsed B cell low-grade or follicular non-Hodgkin's lymphoma (NHL), and is in clinical trials for autoimmune diseases. The discovery of antibodies with superior therapeutic activity compared to rituximab could drastically improve methods of therapy for inflammatory diseases and autoimmune diseases.

Suitable models exist to test for activity in systemic lupus erythematosus (SLE), multiple sclerosis, inflammation and atherosclerosis, transplantation, and Alzheimer's disease as described below.

For example, to test for efficacy in human systemic lupus erythematosus (SLE) in which peripheral blood mononuclear cells (PMBCs) from SLE patients are engrafted into SCID mice. See, for example, the model described in Duchosal et al. J. Exp. Med. 172:985-8. After transfer of PBMCs from SLE patients into SCID mice, it is determined whether or not treatment influences the T lymphocyte response to auto-antigen and auto-antibody production and disease manifestations such as glomerulonephritis.

Marmoset monkey experimental autoimmune encephalitis (EAE) is a model for human multiple sclerosis. See, for example, the model described in Raine et al. (1999) Ann. Neurol. 46:144-60 and Hart et al. (2004) Lancet Neurol. 3:588-97.

The antibodies may be tested in vitro for their ability to inhibit CD40L-induced production of matrix-degrading enzymes, tissue factor expression, proinflammatory cytokines, and upregulation of adhesion molecules. Subsequent studies test the ability of the antibodies to show anti-inflammatory activities in vivo using transgenic mice expressing the human CD40 and/or CD20 molecules. See, for example, the model described in Yasui (2002) Int. Immunol. 14:319-29.

The antibodies may be tested for their ability to prevent transplant rejection in non-human primate models. Cynomolgus monkey renal allograft recipients are treated with antibody to demonstrate the effect on graft acceptance with or without additional immunosuppressive drugs such as cyclosporine, FK506, rapamycin, corticosteroids, CTLA4-Ig, and anti-B Lymphocyte Stimulator antibody, and the like. See, for example, the model described in Wee et al. (1992) Transplantation 53:501-7.

For Alzherimer's disease, the antibodies may be tested first in vitro for their ability to block microglial activation. In vivo efficacy studies with the antibodies may be conducted in double-transgenic mice expressing human CD40 and/or CD20 and overproducing amyloid-beta peptide. See, for example, the model described in Tan et al. (2002) Nat. Neurosci. 5:1288-93.

By "equivalent amount" of the anti-CD40 antibody of the invention and Rituxan® is intended the same or less mg dose is administered on a per weight basis. Thus, where the anti-CD40 antibody is dosed at 0.01 mg/kg body weight of the mouse used in the model, Rituxan® is also dosed at 0.01 mg/kg body weight of the mouse. Similarly, where the anti-CD40 antibody is dosed at 0.1, 1, or 10 mg/kg body weight of the mouse used in the model, the Rituxan® is also dosed at 0.1, 1, or 10 mg/kg, respectively, of the body weight of the mouse.

Another difference in antibody efficacy is to measure *in vitro* the concentration of antibody needed to obtain the maximum lysis of target cells *in vitro* in the presence of NK cells. For example, the anti-CD40 antibodies of the invention reach maximum lysis ofDaudi cells at an EC50 of less than ½, and preferably ¼, and most preferably, 1/10 the concentration of Rituxan®. This type of measurement is also described in the Examples herein.

Anti-CD40 antibodies that benefit from having significantly greater efficacy than equivalent amounts of Rituxan® in the assays described above may include:
a) the monoclonal antibody CHIR-12.12;
b) the monoclonal antibody produced by the hybridoma cell line 12.12;
c) a monoclonal antibody comprising an amino acid sequence selected from the group consisting of the sequence shown in SEQ ID NO:2, the sequence shown in SEQ ID NO:4, the sequence shown in SEQ ID NO:5, both the sequences shown in SEQ ID NO:2 and SEQ ID NO:4, and both the sequences shown in SEQ ID NO:2 and SEQ ID NO:5;
d) a monoclonal antibody having an amino acid sequence encoded by a nucleic acid molecule comprising a nucleotide sequence selected from the group consisting of the sequence shown in SEQ ID NO:1, the sequence shown in SEQ ID NO:3, and both the sequences shown in SEQ ID NO:1 and SEQ ID NO:3;
e) a monoclonal antibody that binds to an epitope capable of binding the monoclonal antibody produced by the hybridoma cell line 12.12;
f) a monoclonal antibody that binds to an epitope comprising residues 82-87. of the human CD40 sequence shown in SEQ ID NO:7 or SEQ ID NO:9;
g) a monoclonal antibody that binds to an epitope comprising residues 82-89 of the human CD40 sequence shown in SEQ ID NO:7 or SEQ ID NO:9;
h) a monoclonal antibody that competes with the monoclonal antibody CHIR-12.12 in a competitive binding assay;
i) the monoclonal antibody of preceding item a) or a monoclonal antibody of any one of preceding items c)-h), wherein said antibody is recombinantly produced; and
j) a monoclonal antibody that is an antigen-binding fragment of a monoclonal antibody of any one of preceding items a)-i), wherein said fragment retains the capability of specifically binding to said human CD40 antigen.

Diclosed herein is a method for identifying a human patient with an inflammatory disease or autoimmune disease treatable with an anti-CD40 antibody, comprising:
a) identifying a human patient with an inflammatory disease or autoimmune disease that is associated with CD40-expressing cells; and
b) determining said human patient's FcγRIIIa-158 genotype (V/V, V/F or F/F);
wherein said inflammatory disease or autoimmune disease is treatable with an anti-CD40 antibody if said human patient is heterozygous or homozygous for FcyRIIIa-158F (genotype V/F or F/F). The inflammatory disease or autoimmune disease may be refractory to treatment with rituximab (Rituxan®).

Once a human patient with an inflammatory disease or autoimmune disease treatable with an anti-CD40 antibody has been identified, that human patient can then be treated with an anti-CD40 antibody. Thus, the method may include the further step of (c) administering to a human patient identified as heterozygous or homozygous for FcγRIIIa-158F (genotype V/F or F/F) a therapeutically or prophylactically effective amount of an anti-CD40 antibody.

This method of identifying a human patient with an inflammatory disease or autoimmune disease treatable with an anti-CD40 antibody can readily be performed by a person skilled in the art using a suitable diagnostic kit. The kit should comprise reagents suitable for determining a human patient's FcγRIIIa-158 genotype. Also disclosed herein is a kit for identifying a human patient with an inflammatory disease or autoimmune disease treatable with an anti-CD40 antibody, comprising reagents for determining a human patient's FcγRIIIa-158 genotype. Suitable kits are described in more detail elsewhere herein.

Also disclosed herein is a method for selecting an antibody therapy for treatment of a human patient having an inflammatory disease or autoimmune disease, comprising:
(a) identifying a human patient having an inflammatory disease or autoimmune disease that is associated with CD40-expressing cells; and
(b) determining said human patient's FcγRIIIa-158 genotype (V/V, V/F or F/F),
wherein if said human patient is heterozygous or homozygous for FcγRIIIa-158F (genotype V/F or F/F), an anti-CD40 antibody is selected for treatment of said inflammatory disease or autoimmune disease. The inflammatory disease or autoimmune disease may be refractory to treatment with rituximab (Rituxan®).

Once an anti-CD40 antibody therapy for treatment of a human patient having an inflammatory disease or autoimmune disease has been selected, that human patient can then be treated with an anti-CD40 antibody. Thus, the method may include the further step of (c) administering to a human patient identified as heterozygous or homozygous for FcγRIIIa-158F (genotype V/F or F/F) a therapeutically or prophylactically effective amount of an anti-CD40 antibody.

This method of selecting an antibody therapy for treatment of a human patient having an inflammatory disease or autoimmune disease can also readily be performed by a person skilled in the art using a suitable diagnostic kit. The kit should comprise reagents suitable for determining a human patient's FcγRIIIa-158 genotype. Also disclosed herein is a kit for selecting an antibody therapy for treatment of a human patient having an inflammatory disease or autoimmune disease associated with CD40-expressing cells, comprising reagents for determining a human patient's FcγRIIIa-158 genotype.

By "treatable with an anti-CD40 antibody" is intended the human patient (i.e., an individual with an inflammatory disease or autoimmune disease), when treated with the anti-CD40 antibody, would benefit from a "positive therapeutic response" (as defined elsewhere herin) with respect to the inflammatory disease or autoimmune disease for which treatment is sought.

Any method for determining a human patient's FcγRIIIa-158 genotype using a biological sample obtained from the human patient is contemplated.

For example, disclosed herein is a kit for use in determining a human patient's FcγRIIIa-158 genotype, which includes a microarray comprising at least one probe of 10 or more nucleotides in length and of a sequence suitable for determining a human patient's FcγRIIIa-158 genotype. Labeled RNA or DNA is hybridized to complementary probes on the array and then detected by laser scanning. Hybridization intensities for each probe on the array are determined and converted to a quantitative value representing relative gene expression levels. The selection of probe sequences and lengths can readily be performed by the skilled person. The nucleotide sequence of the human gene and mRNA encoding the FcγRIIIa-158 F and V allotypes is known. Thus, the skilled person can select probe(s) that, under the appropriate experimental conditions, allow a determination of the FcγRIIIa-158 genotype of the target sequences.

Techniques for the synthesis of these arrays using mechanical synthesis methods are described in, e.g., U.S. Patent No. 5,384,261 . Although a planar array surface is preferred, the array may be fabricated on a surface of virtually any shape or even a multiplicity of surfaces. Arrays may be peptides or nucleic acids on beads, gels, polymeric surfaces, fibers such as fiber optics, glass or any other appropriate substrate, see U.S. Patent Nos. 5,770,358, 5,789,162, 5,708,153, 6,040,193 and 5,800,992 . Arrays may be packaged in such a manner as to allow for diagnostics or other manipulation of an all-inclusive device. See, for example, U.S. Patent Nos. 5,856,174 and 5,922,591.

For example, also disclosed herein is a kit for use in determining a human patient's FcγRIIIa-158 genotype, comprising oligonucleotides suitable for use as primers in polymerase-catalysed amplification of the region of the gene or mRNA encoding amino acid 158 of FcγRIIIa. The selection of primer sequences and lengths can readily be performed by the skilled person. The nucleotide sequence of the human gene and mRNA encoding the FcγRIIIa-158 F and V allotypes is known. Thus, the skilled person can select primers which, under the appropriate experimental conditions, will allow amplification of the region of the gene or mRNA encoding amino acid 158 of FcγRIIIa. The amplified sequence can then be sequenced using known methods to determine the patient's FcγRIIIa-158 genotype.

Another method for determining a human patient's FcγRIIIa-158 genotype is to use a nucleic acid-based method that detects DNA fragmentation that is characteristic of the human patient's FcγRIIIa-158 genotype. When resolved using electrophoresis on agarose gels, DNA of each FcγRIIIa-158 genotype has a characteristic pattern. Also disclosed herein is a kit for use in determining a human patient's FcγRIIIa-158 genotype, comprising one or more restriction enzymes suitable for determining a human patient's FcγRIIIa-158 genotype. Suitable restriction enzymes are known in the art (for example, see Koene et al., Blood, 1997, Vol. 90, No. 3, p. 1109-1114).

The kits disclosed herein may also include instructions which indicate how to use the kit to determine a human patient's FcγRIIIa-158 genotype. The kit can also comprise, e.g., a buffering agent, a preservative, or a protein stabilizing agent. Each component of the kit is usually enclosed within an individual container, and all of the various containers are within a single package along with instructions which indicate how to use the kit to determine a human patient's FcγRIIIa-158 genotype.

The invention provides the use of anti-CD40 antibodies in the manufacture of medicaments for treating an inflammatory disease or autoimmune disease associated with CD40-expressing cells, as described elsewhere herein.

The anti-CD40 antibodies of this invention are administered at a concentration that is therapeutically effective to prevent or treat an inflammatory disease or autoimmune disease associated with CD40-expressing cells. To accomplish this goal, the antibodies may be formulated using a variety of acceptable carrier and/or excipients known in the art. The anti-CD40 antibody may be administered by a parenteral route of administration. Typically, the antibodies are administered by injection, either intravenously or subcutaneously. Methods to accomplish this administration are known to those of ordinary skill in the art.

Intravenous administration occurs preferably by infusion over a period of about half hour to 1 hour to about 10 hours (less than 1, 2, 3, 4, 5, 6, 7, 8, 9, or 10 hours). Subsequent infusions may be administered over a period of about less than 1 to about 6 hours, including, for example, about 1 to about 4 hours, about 1 to about 3 hours, or about 1 to about 2 hours or less than an hour. Alternatively, an anti-CD40 antibody such as CHIR-12.12 can be administered subcutaneously.

A pharmaceutical composition of the invention is formulated to be compatible with its intended route of administration. Solutions or suspensions used for parenteral application can include the following components: a sterile diluent such as water for injection, saline solution; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

The anti-CD40 antibodies are typically provided by standard technique within a pharmaceutically acceptable buffer, for example, sterile saline, sterile buffered water, combinations of the foregoing, etc. Methods for preparing parenterally administrable agents are described in Remington's Pharmaceutical Sciences (18th ed.; Mack Publishing Company, Eaton, Pennsylvania, 1990). See also, for example, WO 98/56418, which describes stabilized antibody pharmaceutical formulations suitable for use in the methods of the present invention.

The amount of at least one anti-CD40 antibody to be administered is readily determined by one of ordinary skill in the art without undue experimentation. Factors influencing the mode of administration and the respective amount of at least one anti-CD40 antibody include, but are not limited to, the the severity of the disease, the history of the disease, and the age, height, weight, health, and physical condition of the individual undergoing therapy. Similarly, the amount of anti-CD40 antibody to be administered will be dependent upon the mode of administration and whether the subject will undergo a single dose or multiple doses of this anti-tumor agent. Generally, a higher dosage of anti-CD40 antibody is preferred with increasing weight of the subject undergoing therapy.

The dose of anti-CD40 antibody to be administered is in the range from about 0.003 mg/kg to about 50 mg/kg, from about 0.01 mg/kg to about 40 mg/kg, from about 0.01 mg/kg to about 30 mg/kg, from about 0.1 mg/kg to about 30 mg/kg, from about 0.5 mg/kg to about 30 mg/kg, from about 1 mg/kg to about 30 mg/kg, from about 3 mg/kg to about 30 mg/kg, from about 3 mg/kg to about 25 mg/kg, from about 3 mg/kg to about 20 mg/kg, from about 5 mg/kg to about 15 mg/kg, or from about 7 mg/kg to about 12 mg/kg.

Thus, for example, the dose can be 0.3 mg/kg, 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, or 50 mg/kg, or other such doses falling within the range of about 0.3 mg/kg to about 50 mg/kg.

Treatment of a subject with a therapeutically effective amount of an antibody can include a single treatment or, preferably, can include a series of treatments. Thus, in another embodiment of the invention, the method comprises administration of multiple doses of anti-CD40 antibody. The method may comprise administration of 1, 2, 3, 4, 5, 6, 7, 8, 9, 10, 15, 20, 25, 30, 35, 40, or more therapeutically effective doses of a pharmaceutical composition comprising an anti-CD40 antibody. The frequency and duration of administration of multiple doses of the pharmaceutical compositions comprising anti-CD40 antibody can be readily determined by one of skill in the art without undue experimentation. The same therapeutically effective dose of an anti-CD40 antibody can be administered over the course of a treatment period. Alternatively, different therapeutically effective doses of an anti-CD40 antibody can be used over the course of a treatment period.

In a preferred example, a subject is treated with anti-CD40 antibody in the range of between about 0.1 to 20 mg/kg body weight, once per week for between about 1 to 10 weeks, preferably between about 2 to 8 weeks, more preferably between about 3 to 7 weeks, and even more preferably for about 4, 5, or 6 weeks. Treatment may occur bi-annually or annually to prevent relapse or upon indication of relapse. It will also be appreciated that the effective dosage of antibody used for treatment may increase or decrease over the course of a particular treatment. Changes in dosage may result and become apparent from the results of diagnostic assays as described herein.

Thus, in one embodiment, the dosing regimen includes a first administration of a therapeutically effective dose of at least one anti-CD40 antibody on days 1, 8, 15, and 22 of a treatment period. In another embodiment, the dosing regimen includes a dosing regimen having a first administration of a therapeutically effective dose of at least one anti-CD40 antibody daily, or on days 1, 3, 5, and 7 of a week in a treatment period; a dosing regimen including a first administration of a therapeutically effective dose of at least one anti-CD40 antibody on days 1 and 3-4 of a week in a treatment period; and a preferred dosing regimen including a first administration of a therapeutically effective dose of at least one anti-CD40 antibody on day 1 of a week in a treatment period. The treatment period may comprise 1 week, 2 weeks, 3 weeks, a month, 2 months, 3 months; 6 months, or a year. Treatment periods may be subsequent or separated from each other by a week, 2 weeks, a month, 3 months, 6 months, or a year.

In other embodiments, the initial therapeutically effective dose of an anti-CD40 antibody as defined elsewhere herein can be in the lower dosing range (i.e., about 0.003 mg/kg to about 20 mg/kg) with subsequent doses falling within the higher dosing range (i.e., from about 20 mg/kg to about 50 mg/kg).

In alternative embodiments, the initial therapeutically effective dose of an anti-CD40 antibody as defined elsewhere herein can be in the upper dosing range (i.e., about 20 mg/kg to about 50 mg/kg) with subsequent doses falling within the lower dosing range (i.e., 0.003 mg/kg to about 20 mg/kg). Thus, in some embodiments of the invention, anti-CD40 antibody therapy may be initiated by administering a "loading dose" of the antibody to the subject in need therapy. By "loading dose" is intended an initial dose of the anti-CD40 antibody that is administered to the subject, where the dose of the antibody administered falls within the higher dosing range (i.e., from about 20 mg/kg to about 50 mg/kg). The "loading dose" can be administered as a single administration, for example, a single infusion where the antibody is administered IV, or as multiple administrations, for example, multiple infusions where the antibody is administered IV, so long as the complete "loading dose" is administered within about a 24-hour period. Following administration of the "loading dose," the subject is then administered one or more additional therapeutically effective doses of the anti-CD40 antibody. Subsequent therapeutically effective doses can be administered, for example, according to a weekly dosing schedule, or once every two weeks, once every three weeks, or once every four weeks. In such embodiments, the subsequent therapeutically effective doses generally fall within the lower dosing range (i.e., 0.003 mg/kg to about 20 mg/kg).

Alternatively, in some embodiments, following the "loading dose", the subsequent therapeutically effective doses of the anti-CD40 antibody are administered according to a "maintenance schedule," wherein the therapeutically effective dose of the antibody is administered once a month, once every 6 weeks, once every two months, once every 10 weeks, once every three months, once every 14 weeks, once every four months, once every 18 weeks, once every five months, once every 22 weeks, once every six months, once every 7 months, once every 8 months, once every 9 months, once every 10 months, once every 11 months, or once every 12 months. In such embodiments, the therapeutically effective doses of the anti-CD40 antibody fall within the lower dosing range (i.e., 0.003 mg/kg to about 20 mg/kg), particularly when the subsequent doses are administered at more frequent intervals, for example, once every two weeks to once every month, or within the higher dosing range (i.e., from about 20 mg/kg to about 50 mg/kg), particularly when the subsequent doses are administered at less frequent intervals, for example, where subsequent doses are administered about one month to about 12 months apart.

The anti-CD40 antibodies present in the pharmaceutical compositions described herein for use in the methods of the invention may be native or obtained by recombinant techniques, and may be from any source, including mammalian sources such as, e.g., mouse, rat, rabbit, primate, pig, and human. Preferably such polypeptides are derived from a human source, and more preferably are recombinant, human proteins from hybridoma cell lines.

The pharmaceutical compositions useful in the methods of the invention may comprise biologically active variants of the antagonist anti-CD40 antibodies of the invention, as described elsewhere herein.

Any pharmaceutical composition comprising an anti-CD40 antibody having the binding properties described herein as the therapeutically active component can be used in the methods of the invention. Thus liquid, lyophilized, or spray-dried compositions comprising one or more of the anti-CD40 antibodies may be prepared as an aqueous or nonaqueous solution or suspension for subsequent administration to a subject in accordance with the methods of the invention. Each of these compositions will comprise at least one anti-CD40 antibody as a therapeutically or prophylactically active component By "therapeutically or prophylactically active component" is intended the anti-CD40 antibody is specifically incorporated into the composition to bring about a desired therapeutic or prophylactic response with regard to treatment, prevention, or diagnosis of a disease or condition within a subject when the pharmaceutical composition is administered to that subject. Preferably the pharmaceutical compositions comprise appropriate stabilizing agents, bulking agents, or both to minimize problems associated with loss of protein stability and biological activity during preparation and storage.

Formulants may be added to pharmaceutical compositions comprising an anti-CD40 antibody of the invention. These formulants may include, but are not limited to, oils, polymers, vitamins, carbohydrates, amine acids, salts, buffers, albumin, surfactants, or bulking agents. Preferably carbohydrates include sugar or sugar alcohols such as mono-, di-, or polysaccharides, or water soluble glucans. The saccharides or glucans can include fructose, glucose, mannose, sorbose, xylose, maltose, sucrose, dextran, pullulan, dextrin, α and β cyclodextrin, soluble starch, hydroxyethyl starch, and carboxymethylcellulose, or mixtures thereof. "Sugar alcohol" is defined as a C₄ to C₈ hydrocarbon having a hydroxyl group and includes galactitol, inositol, mannitol, xylitol, sorbitol, glycerol, and arabitol. These sugars or sugar alcohols may be used individually or in combination. The sugar or sugar alcohol concentration is between 1.0% and 7% w/v., more preferably between 2.0% and 6.0% w/v. Preferably amino acids include levorotary (L) forms of carnitine, arginine, and betaine; however, other amino acids may be added. Preferred polymers include polyvinylpyrrolidone (PVP) with an average molecular weight between 2,000 and 3,000, or polyethylene glycol (PEG) with an average molecular weight between 3,000 and 5,000. Surfactants that can be added to the formulation are shown in EP Nos. 270,799 and 268,110.

Additionally, antibodies can be chemically modified by covalent conjugation to a polymer to increase their circulating half-life, for example. Preferred polymers, and methods to attach them to peptides, are shown in U.S. Patent Nos. 4,766,106; 4,179,337; 4,495,285; and 4,609,546. Preferred polymers are polyoxyethylated polyols and polyethylene glycol (PEG). PEG is soluble in water at room temperature and has the general formula: R(O--CH₂ --CH₂)ₙ O--R where R can be hydrogen, or a protective group such as an alkyl or alkanol group. Preferably, the protective group has between 1 and 8 carbons, more preferably it is methyl. The symbol n is a positive integer, preferably between 1 and 1,000, more preferably between 2 and 500. The PEG has a preferred average molecular weight between 1,000 and 40,000, more preferably between 2,000 and 20,000, most preferably between 3,000 and 12,000. Preferably, PEG has at least one hydroxy group, more preferably it is a terminal hydroxy group. It is this hydroxy group which is preferably activated to react with a free amino group on the inhibitor. However, it will be understood that the type and amount of the reactive groups may be varied to achieve a covalently conjugated PEG/antibody of the present invention.

Water-soluble polyoxyethylated polyols are also useful in the present invention. They include polyoxyethylated sorbitol, polyoxyethylated glucose, polyoxyethylated glycerol (POG), and the like. POG is preferred. One reason is because the glycerol backbone of polyoxyethylated glycerol is the same backbone occurring naturally in, for example, animals and humans in mono-, di-, triglycerides. Therefore, this branching would not necessarily be seen as a foreign agent in the body. The POG has a preferred molecular weight in the same range as PEG The structure for POG is shown in Knauf et al. (1988) J. Bio. Chem. 263:15064-15070, and a discussion of POG/IL-2 conjugates is found in U.S. Patent No. 4,766,106.

Another drug delivery system for increasing circulatory half-life is the liposome. Methods of preparing liposome delivery systems are discussed in Gabizon et al. (1982) Cancer Research 42:4734; Cafiso (1981) Biochem Biophys Acta 649:129; and Szoka (1980) Ann. Rev. Biophys. Eng. 9:467. Other drug delivery systems are known in the art and are described in, *e.g.,* Poznansky et al. (1980) Drug Delivery Systems (R.L. Juliano, ed., Oxford, N.Y.) pp. 253-315; Poznansky (1984) Pharm Revs 36:277.

The formulants to be incorporated into a pharmaceutical composition should provide for the stability of the anti-CD40 antibody. That is, the anti-CD40 antibody should retain its physical and/or chemical stability and have the desired biological activity, i.e., one or more of the antagonist activities defined herein above, including, but not limited to, inhibition of immunoglobulin secretion by normal human peripheral B cells stimulated by T cells; inhibition of survival and/or proliferation of normal human peripheral B cells stimulated by Jurkat T cells; inhibition of survival and/or proliferation of normal human peripheral B cells stimulated by CD40L-expressing cells or soluble CD40 ligand (sCD40L); inhibition of "survival" anti-apoptotic intracellular signals in any cell stimulated by sCD40L or solid-phase CD40L; inhibition of CD40 signal transduction in any cell upon ligation with sCD40L or solid-phase CD40L; and inhibition of proliferation of human malignant B cells as noted elsewhere herein.

Methods for monitoring protein stability are well known in the art. See, for example, Jones (1993) Adv. Drug Delivery Rev. 10:29-90; Lee, ed. (1991) Peptide and Protein Drug Delivery (Marcel Dekker, Inc., New York, New York); and the stability assays disclosed herein below. Generally, protein stability is measured at a chosen temperature for a specified period of time. In preferred embodiments, a stable antibody pharmaceutical formulation provides for stability of the anti-CD40 antibody when stored at room temperature (about 25°C) for at least 1 month, at least 3 months, or at least 6 months, and/or is stable at about 2-8°C for at least 6 months, at least 9 months, at least 12 months, at least 18 months, at least 24 months.

A protein such as an antibody, when formulated in a pharmaceutical composition, is considered to retain its physical stability at a given point in time if it shows no visual signs (i.e., discoloration or loss of clarity) or measurable signs (for example, using size-exclusion chromatography (SEC) or UV light scattering) of precipitation, aggregation, and/or denaturation in that pharmaceutical composition. With respect to chemical stability, a protein such as an antibody, when formulated in a pharmaceutical composition, is considered to retain its chemical stability at a given point in time if measurements of chemical stability are indicative that the protein (i.e., antibody) retains the biological activity of interest in that pharmaceutical composition. Methods for monitoring changes in chemical stability are well known in the art and include, but are not limited to, methods to detect chemically altered forms of the protein such as result from clipping, using, for example, SDS-PAGE, SEC, and/or matrix-assisted laser desorption ionization/time of flight mass spectrometry; and degradation associated with changes in molecular charge (for example, associated with deamidation), using, for example, ion-exchange chromatography. See, for example, the methods disclosed herein below.

An anti-CD40 antibody, when formulated in a pharmaceutical composition, is considered to retain a desired biological activity at a given point in time if the desired biological activity at that time is within about 30%, preferably within about 20% of the desired biological activity exhibited at the time the pharmaceutical composition was prepared as determined in a suitable assay for the desired biological activity. Assays for measuring the desired biological activity of the anti-CD40 antibodies can be performed as described in the Examples herein. See also the assays described in Schultze et al. (1998) Proc. Natl. Acad Sci. USA 92:8200-8204; Denton et al. (1998) Pediatr. Transplant. 2:6-15; Evans et al. (2000) J. Immunol. 164:688-697; Noelle (1998) Agents Actions Suppl. 49:17-22; Lederman et al. (1996) Curr. Opin. Hematol. 3:77-86; Coligan et al. (1991) Current Protocols in Immunology 13:12; Kwekkeboom et al. (1993) Immunology 79:439-444; and U.S. Patent Nos. 5,674,492 and 5,847,082.

In some embodiments, the anti-CD40 antibody is formulated in a liquid pharmaceutical formulation. The anti-CD40 antibody can be prepared using any method known in the art, including those methods disclosed herein above. In one embodiment, the anti-CD40 antibody is recombinantly produced in a CHO cell line.

Where the anti-CD40 antibody is to be stored prior to its formulation, it can be frozen, for example, at ≤ -20°C, and then thawed at room temperature for further formulation. The liquid pharmaceutical formulation comprises a therapeutically effective amount of the anti-CD40 antibody. The amount of antibody thereof present in the formulation takes into consideration the route of administration and desired dose volume.

In this manner, the liquid pharmaceutical composition comprises the anti-CD40 antibody at a concentration of about 0.1 mg/ml to about 50.0 mg/ml, about 0.5 mg/ml to about 40.0 mg/ml, about 1.0 mg/ml to about 30.0 mg/ml, about 5.0 mg/ml to about 25.0 mg/ml, about 5.0 mg/ml to about 20.0 mg/ml, or about 15.0 mg/ml to about 25.0 mg/ml. In some embodiments, the liquid pharmaceutical composition comprises the anti-CD40 antibody at a concentration of about 0.1 mg/ml to about 5.0 mg/ml, about 5.0 mg/ml to about 10.0 mg/ml, about 10.0 mg/ml to about 15.0 mg/ml, about 15.0 mg/ml to about 20.0 mg/ml, about 20.0 mg/ml to about 25.0 mg/ml, about 25.0 mg/ml to about 30.0 mg/ml, about 30.0 mg/ml to about 35.0 mg/ml, about 35.0 mg/ml to about 40.0 mg/ml, about 40.0 mg/ml to about 45.0 mg/ml, or about 45.0 mg/ml to about 50.0 mg/ml. In other embodiments, the liquid pharmaceutical composition comprises the anti-CD40 antibody at a concentration of about 15.0 mg/ml, about 16.0 mg/ml, about 17.0 mg/ml, about 18.0 mg/ml, about 19.0 mg/ml, about 20.0 mg/ml, about 21.0 mg/ml, about 22.0 mg/ml, about 23.0 mg/ml, about 24.0 mg/ml, or about 25.0 mg/ml. The liquid pharmaceutical composition comprises the anti-CD40 antibody and a buffer that maintains the pH of the formulation in the range of about pH 5.0 to about pH 7.0, including about pH 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, and other such values within the range of about pH 5.0 to about pH 7.0. In some embodiments, the buffer maintains the pH of the formulation in the range of about pH 5.0 to about pH 6.5, about pH 5.0 to about pH 6.0, about pH 5.0 to about pH 5.5, about pH 5.5 to about 7.0, about pH 5.5 to about pH 6.5, or about pH 5.5 to about pH 6.0.

Any suitable buffer that maintains the pH of the liquid anti-CD40 antibody formulation in the range of about pH 5.0 to about pH 7.0 can be used in the formulation, so long as the physicochemical stability and desired biological activity of the antibody are retained as noted herein above. Suitable buffers include, but are not limited to, conventional acids and salts thereof, where the counter ion can be, for example, sodium, potassium, ammonium, calcium, or magnesium. Examples of conventional acids and salts thereof that can be used to buffer the pharmaceutical liquid formulation include, but are not limited to, succinic acid or succinate, citric acid or citrate, acetic acid or acetate, tartaric acid or tartarate, phosphoric acid or phosphate, gluconic acid or gluconate, glutamic acid or glutamate, aspartic acid or aspartate, maleic acid or maleate, and malic acid or malate buffers. The buffer concentration within the formulation can be from about 1 mM to about 50 mM, including about 1 mM, 2 mM, 5 mM, 8 mM, 10 mM, 15 mM, 20 mM, 25 mM, 30 mM, 35 mM, 40 mM, 45 mM, 50 mM, or other such values within the range of about 1 mM to about 50 mM. In some embodiments, the buffer concentration within the formulation is from about 5 mM to about 15 mM, including about 5 mM, 6 mM, 7 mM, 8 mM, 9 mM, 10 mM, 11 mM, 12 mM, 13 mM, 14 mM, 15 mM, or other such values within the range of about 5 mM to about 15 mM.

In some embodiments, the liquid pharmaceutical formulation comprises a therapeutically effective amount of the anti-CD40 antibody and succinate buffer or citrate buffer at a concentration that maintains the pH of the formulation in the range of about pH 5.0 to about pH 7.0, preferably about pH 5.0 to about pH 6.5. By "succinate buffer" or "citrate buffer" is intended a buffer comprising a salt of succinic acid or a salt of citric acid, respectively. In a preferred embodiment, the succinate or citrate counterion is the sodium cation, and thus the buffer is sodium succinate or sodium citrate, respectively. However, any cation is expected to be effective. Other possible succinate or citrate cations include, but are not limited to, potassium, ammonium, calcium, and magnesium. As noted above, the succinate or citrate buffer concentration within the formulation can be from about 1 mM to about 50 mM, including about 1 mM, 2 mM, 5 mM, 8 mM, 10 mM, 15 mM, 20 mM, 25 mM, 30 mM, 35 mM, 40 mM, 45 mM, 50 mM, or other such values within the range of about 1 mM to about 50 mM. In some embodiments, the buffer concentration within the formulation is from about 5 mM to about 15 mM, including about 5 mM, 6 mM, 7 mM, 8 mM, 9 mM, 10 mM, 11 mM, 12 mM, 13 mM, 14 mM, or about 15 mM. In other embodiments, the liquid pharmaceutical formulation comprises the anti-CD40 antibody at a concentration of about 0.1 mg/ml to about 50.0 mg/ml, or about 5.0 mg/ml to about 25.0 mg/ml, and succinate or citrate buffer, for example, sodium succinate or sodium citrate buffer, at a concentration of about 1 mM to about 20 mM, about 5 mM to about 15 mM, preferably about 10 mM.

Where it is desirable for the liquid pharmaceutical formulation to be near isotonic, the liquid pharmaceutical formulation comprising the anti-CD40 antibody and a buffer can further comprise an amount of an isotonizing agent sufficient to render the formulation near isotonic. By "near isotonic" is intended the aqueous formulation has an osmolarity of about 240 mmol/kg to about 360 mmol/kg, preferably about 240 to about 340 mmol/kg, more preferably about 250 to about 330 mmol/kg, even more preferably about 260 to about 320 mmol/kg, still more preferably about 270 to about 310 mmol/kg. Methods of determining the isotonicity of a solution are known to those skilled in the art. See, for example, Setnikar et al. (1959) J. Am. Pharm. Assoc. 48:628.

Those skilled in the art are familiar with a variety of pharmaceutically acceptable solutes useful in providing isotonicity in pharmaceutical compositions. The isotonizing agent can be any reagent capable of adjusting the osmotic pressure of the liquid pharmaceutical formulation of the present invention to a value nearly equal to that of a body fluid. It is desirable to use a physiologically acceptable isotonizing agent. Thus, the liquid pharmaceutical formulation comprising a therapeutically effective amount of the anti-CD40 antibody and a buffer can further comprise components that can be used to provide isotonicity, for example, sodium chloride; amino acids such as alanine, valine, and glycine; sugars and sugar alcohols (polyols), including, but not limited to, glucose, dextrose, fructose, sucrose, maltose, mannitol, trehalose, glycerol, sorbitol, and xylitol; acetic acid, other organic acids or their salts, and relatively minor amounts of citrates or phosphates. The ordinary skilled person would know of additional agents that are suitable for providing optimal tonicity of the liquid formulation.

In some preferred embodiments, the liquid pharmaceutical formulation comprising an anti-CD40 antibody and a buffer further comprises sodium chloride as the isotonizing agent. The concentration of sodium chloride in the formulation will depend upon the contribution of other components to tonicity. In some embodiments, the concentration of sodium chloride is about 50 mM to about 300 mM, about 50 mM to about 250 mM, about 50 mM to about 200 mM, about 50 mM to about 175 mM, about 50 mM to about 150 mM, about 75 mM to about 175 mM, about 75 mM to about 150 mM, about 100 mM to about 175 mM, about 100 mM to about 200 mM, about 100 mM to about 150 mM, about 125 mM to about 175 mM, about 125 mM to about 150 mM, about 130 mM to about 170 mM, about 130 mM to about 160 mM, about 135 mM to about 155 mM, about 140 mM to about 155 mM, or about 145 mM to about 155 mM. In one such embodiment, the concentration of sodium chloride is about 150 mM. In other such embodiments, the concentration of sodium chloride is about 150 mM, the buffer is sodium succinate or sodium citrate buffer at a concentration of about 5 mM to about 15 mM, the liquid pharmaceutical formulation comprises a therapeutically effective amount of the anti-CD40 antibody and the formulation has a pH of about pH 5.0 to about pH 7.0, about pH 5.0 to about pH 6.0, or about pH 5.5 to about pH 6.5. In other embodiments, the liquid pharmaceutical formulation comprises the anti-CD40 antibody at a concentration of about 0.1 mg/ml to about 50.0 mg/ml or about 5.0 mg/ml to about 25.0 mg/ml, about 150 mM sodium chloride, and about 10 mM sodium succinate or sodium citrate, at a pH of about pH 5.5.

Protein degradation due to freeze thawing or mechanical shearing during processing of a liquid pharmaceutical formulations of the present invention can be inhibited by incorporation of surfactants into the formulation in order to lower the surface tension at the solution-air interface. Thus, in some embodiments, the liquid pharmaceutical formulation comprises a therapeutically effective amount of the anti-CD40 antibody, a buffer, and further comprises a surfactant. In other embodiments, the liquid pharmaceutical formulation comprises an anti-CD40 antibody, a buffer, an isotonizing agent, and further comprises a surfactant

Typical surfactants employed are nonionic surfactants, including polyoxyethylene sorbitol esters such as polysorbate 80 (Tween 80) and polysorbate 20 (Tween 20); polyoxypropylene-polyoxyethylene esters such as Pluronic F68; polyoxyethylene alcohols such as Brij 35; simethicone; polyethylene glycol such as PEG400; lysophosphatidylcholine; and polyoxyethylene-p-t-octylphenol such as Triton X-100. Classic stabilization of pharmaceuticals by surfactants or emulsifiers is described, for example, in Levine et al. (1991) J. Parenteral Sci. Technol. 45(3):160-165. A preferred surfactant employed in the practice of the present invention is polysorbate 80. Where a surfactant is included, it is typically added in an amount from about 0.001 % to about 1.0% (w/v), about 0.001% to about 0.5%, about 0.001% to about 0.4%, about 0.001% to about 0.3%, about 0.001% to about 0.2%, about 0.005% to about 0.5%, about 0.005% to about 0.2%, about 0.01% to about 0.5%, about 0.01 % to about 0.2%, about 0.03% to about 0.5%, about 0.03% to about 0.3%, about 0.05% to about 0.5%, or about 0.05% to about 0.2%.

Thus, in some embodiments, the liquid pharmaceutical formulation comprises a therapeutically effective amount of the anti-CD40 antibody, the buffer is sodium succinate or sodium citrate buffer at a concentration of about 1 mM to about 50 mM, about 5 mM to about 25 mM, or about 5 mM to about 15 mM; the formulation has a pH of about pH 5.0 to about pH 7.0, about pH 5.0 to about pH 6.0, or about pH 5.5 to about pH 6.5; and the formulation further comprises a surfactant, for example, polysorbate 80, in an amount from about 0.001% to about 1.0% or about 0.001% to about 0.5%. Such formulations can optionally comprise an isotonizing agent, such as sodium chloride at a concentration of about 50 mM to about 300 mM, about 50 mM to about 200 mM, or about 50 mM to about 150 mM. In other embodiments, the liquid pharmaceutical formulation comprises the anti-CD40 antibody at a concentration of about 0.1 mg/ml to about 50.0 mg/ml or about 5.0 mg/ml to about 25.0 mg/ml, including about 20.0 mg/ml; about 50 mM to about 200 mM sodium chloride, including about 150 mM sodium chloride; sodium succinate or sodium citrate at about 5 mM to about 20 mM, including about 10 mM sodium succinate or sodium citrate; sodium chloride at a concentration of about 50 mM to about 200 mM, including about 150 mM; and optionally a surfactant, for example, polysorbate 80, in an amount from about 0.001 % to about 1.0%, including about 0.001% to about 0.5%; where the liquid pharmaceutical formulation has a pH of about pH 5.0 to about pH 7.0, about pH 5.0 to about pH 6.0, about pH 5.0 to about pH 5.5, about pH 5.5 to about pH 6.5, or about pH 5.5 to about pH 6.0.

The liquid pharmaceutical formulation can be essentially free of any preservatives and other carriers, excipients, or stabilizers noted herein above. Alternatively, the formulation can include one or more preservatives, for example, antibacterial agents, pharmaceutically acceptable carriers, excipients, or stabilizers described herein above provided they do not adversely affect the physicochemical stability of the anti-CD40 antibody. Examples of acceptable carriers, excipients, and stabilizers include, but are not limited to, additional buffering agents, co-solvents, surfactants, antioxidants including ascorbic acid and methionine, chelating agents such as EDTA, metal complexes (for example, Zn-protein complexes), and biodegradable polymers such as polyesters. A thorough discussion of formulation and selection of pharmaceutically acceptable carriers, stabilizers, and isomolytes can be found in Remington's Pharmaceutical Sciences (18th ed.; Mack Publishing Company, Eaton, Pennsylvania, 1990).

"Carriers" as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, succinate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN, polyethylene glycol (PEG), and Pluronics.

Administration "in combination with" one or more further therapeutic agents includes simultaneous (concurrent) and consecutive administration in any order.

After the liquid pharmaceutical formulation or other pharmaceutical composition described herein is prepared, it can be lyophilized to prevent degradation. Methods for lyophilizing liquid compositions are known to those of ordinary skill in the art. Just prior to use, the composition may be reconstituted with a sterile diluent (Ringer's solution, distilled water, or sterile saline, for example) that may include additional ingredients. Upon reconstitution, the composition is preferably administered to subjects using those methods that are known to those skilled in the art.

In some embodiments, the anti-CD40 antibodies may be administered in combination with at least one other known therapy for inflammatory and/or autoimmune diseases. Such therapies include, but are not limited to, surgery or surgical procedures (e.g. splenectomy, lymphadenectomy, thyroidectomy, plasmaphoresis, leukophoresis, cell, tissue, or organ transplantation, intestinal procedures, organ perfusion, and the like), radiation therapy, therapy such as steroid therapy and non-steroidal therapy, hormone therapy, cytokine therapy, therapy with dermatological agents (for example, topical agents used to treat skin conditions such as allergies, contact dermatitis, and psoriasis), immunosuppressive therapy, and other anti-inflammatory monoclonal antibody therapy, and the like. Where the methods of disclosed herein comprise combined therapeutic regimens, these therapies can be given simultaneously, i.e., the antagonist anti-CD40 antibody or antigen-binding fragment thereof is administered concurrently or within the same time frame as the other therapy (i.e., the therapies are going on concurrently, but the anti-CD40 antibody or antigen-binding fragment thereof is not administered precisely at the same time as the other therapy). Alternatively, the antagonist anti-CD40 antibody of the present invention or antigen-binding fragment thereof may also be administered prior to or subsequent to the other therapy. Sequential administration of the different therapies may be performed regardless of whether the treated subject responds to the first course of therapy to decrease the possibility of remission or relapse.

In this manner, the anti-CD40 antibodies are administered in combination with at least one other therapy, including, but not limited to, surgery, organ perfusion, radiation therapy, steroid therapy, non-steroidal therapy, antibiotic therapy, antifungal therapy, hormone therapy, cytokine therapy, therapy with dermatological agents (for example, topical agents used to treat skin conditions such as allergies, contact dermatitis, and psoriasis), immunosuppressive therapy, other anti-inflammatory monoclonal antibody therapy, combinations thereof, and the like. Thus, where the combined therapies comprise administration of an anti-CD40 antibody in combination with administration of another therapeutic agent, as with steroids as one example, the methods of the invention encompass coadministration, using separate formulations or a single pharmaceutical formulation, and consecutive administration in either order.

Examples of immunosuppressive drugs that can be administered in combination with anti-CD40 antibodies include, but are not limited to, methotrexate, cyclophosphamide, mizoribine, chlorambucil, cyclosporine, such as, for example, aerosolized cyclosporine (see, U.S. Patent Application Publication No. US20020006901), tacrolimus (FK506; ProGraf™), mycophenolate mofetil, and azathioprine (6-mercaptopurine), sirolimus (rapamycin), deoxyspergualin, leflunomide and its malononitriloamide analogs; and immunosuppressive proteins, including,

Examples of suitable anti-inflammatory agents that can be administered in combination with anti-CD40 antibodies include, but are not limited to, corticosteroids such as, for example, clobetasol, halobetasol, hydrocortisone, triamcinolone, betamethasone, fluocinole, fluocinonide, prednisone, prednisolone, methylprednisolone; non-steroidal anti-inflammatory drugs (NSAIDs) such as, for example, sulfasalazine, medications containing mesalamine (known as 5-ASA agents), celecoxib, diclofenac, etodolac, fenprofen, flurbiprofen, ibuprofen, ketoprofen, meclofamate, meloxicam , nabumetone, naproxen, oxaprozin, piroxicam, rofecoxib, salicylates, sulindac, and tolmetin; Enbrel ® (a soluble TNF receptor), anti-inflammatory antibodies such as adalimumab (HUMIRA^{®}, a TNF-α antagonist) and infliximab (Remicade^{®}, a TNF-α antagonist), and the like.

Transplant rejection and graft versus host disease can be hyperacute (humoral), acute (T cell mediated), or chronic (unknown etiology), or a combination thereof. Thus, the anti-CD40 antibodies of the invention are used in some embodiments to prevent and/or ameliorate rejection and/or symptoms associated with hyperacute, acute, and/or chronic transplant rejection of any tissue, including, but not limited to, liver, kidney, pancreas, pancreatic islet cells, small intestine, lung, heart, corneas, skin, blood vessels, bone, heterologous or autologous bone marrow, and the like. Graft tissues may be obtained from any donor and transplanted into any recipient host, and thus the transplant procedure may comprise transplanting animal tissue to humans (e.g., xenografts), transplanting tissue from one human to another human (e.g., allografts), and/or transplanting tissue from one part of a human's body to another (e.g., autografts). Treatment with the antibodies of the invention may also reduce transplantation sequelae such as fever, anorexia, hemodynamic abnormalities, leukopenia, white cell infiltration of the transplanted organ/tissue, as well as opportunistic infections.

In embodiments where the anti-CD40 antibodies of the invention are used to treat graft rejection, rheumatoid arthritis, or multiple sclerosis, the antibodies may used in combination with suitable immunosuppressive drugs as described herein.

Thus, the invention provides the use of a therapeutically or prophylactically effective amount of an anti-CD40 antibody in the manufacture of a medicament for the treatment of an inflammatory disease or autoimmune disease that is associated with CD40-expressing cells in a human patient heterozygous or homozygous for FcγRIIIa-158F (genotype V/F or F/F), wherein the medicament is coordinated with treatment with at least one other therapy.

By "coordinated" is intended the medicament comprising the anti-CD40 antibody is to be used either prior to, during, or after treatment of the subject with at least one other therapy.

The invention also provides for the use of an anti-CD40 antibody in the manufacture of a medicament for treating a human patient for an inflammatory disease or autoimmune disease that is associated with CD40-expressing cells, wherein said human patient is heterozygous or homozygous for FcγRIIIa-158F (genotype V/F or F/F) and has been pretreated with at least one other therapeutic agent.

By "pretreated" or "pretreatment" is intended the subject has received one or more other therapies (i.e., been treated.with at least one other therapy) prior to receiving the medicament comprising the anti-CD40 antibody. "Pretreated" or "pretreatment" includes subjects that have been treated with at least one other therapy within 2 years, within 18 months, within 1 year, within 6 months, within 2 months, within 6 weeks, within 1 month, within 4 weeks, within 3 weeks, within 2 weeks, within 1 week, within 6 days, within 5 days, within 4 days, within 3 days, within 2 days, or even within 1 day prior to initiation of treatment with the medicament comprising the anti-CD40 antibody. It is not necessary that the subject was a responder to pretreatment with the prior therapy, or prior therapies. Thus, the subject that receives the medicament comprising the antagonist anti-CD40 antibody could have responded, or could have failed to respond (i.e. the disease was refractory), to pretreatment with the prior therapy, or to one or more of the prior therapies where pretreatment comprised multiple therapies. Examples of other therapies for which a subject can have received pretreatment prior to receiving the medicament comprising the anti-CD40 antibody include, but are not limited to the therapies for inflammatory or autoimmune diseases described elsewhere herein.

"Treatment" in the context of coordinated use of a medicament described herein with one or more other therapies is herein defined as the application or administration of the medicament or of the other therapy to a subject, or application or administration of the medicament or other therapy to an isolated tissue or cell line from a subject, where the subject has an inflammatory disease or autoimmune disease associated with CD40-expressing cells, a symptom associated with such a disease, or a predisposition toward development of such a disease, where the purpose is to cure, heal, alleviate, relieve, alter, remedy, ameliorate, improve, or affect the disease, any associated symptoms of the disease, or the predisposition toward the development of the disease.

In some embodiments, the combination therapy provides a synergistic improvement in therapeutic efficacy relative to the individual therapeutic agents when administered alone. The term "synergy" is used to describe a combined effect of two or more active agents that is greater than the sum of the individual effects of each respective active agent. Thus, where the combined effect of two or more agents results in "synergistic inhibition" of an activity or process, it is intended that the inhibition of the activity or process is greater than the sum of the inhibitory effects of each respective active agent. The term "synergistic therapeutic effect" refers to a therapeutic effect observed with a combination of two or more therapies wherein the therapeutic effect (as measured by any of a number of parameters) is greater than the sum of the individual therapeutic effects observed with the respective individual therapies.

Various aspects and embodiments of the present invention will now be described in more detail by way of example only. It will be appreciated that modification of detail may be made without departing from the scope of the invention.

### EXPERIMENTAL

The anti-CD40 antibody used in the examples below is CHIR-12.12. The production, sequencing and characterisation of the CHIR-12.12 antibody is described in detail in the international patent applications published as WO 2005/044854, WO 2005/044304, WO 2005/044305, WO 2005/044306, WO 2005/044855, WO 2005/044307, and WO 2005/044294. Hybridoma line 153.8E2.D10.D6.12.12 (CMCC#12056) expressing the CHIR-12.12 antibody has been deposited with the American Type Culture Collection [ATCC; 10801 University Blvd., Manassas, Virginia 20110-2209 (USA)] under Patent Deposit Number PTA-5543.

A number of the following examples are based on CHIR-12.12 binding to cancer cell lines and cancer patient cells. However, all of the following examples are directly relevant to the use of CHIR-12.12 to treat inflammatory diseases and/or autoimmune diseases, because they illustrate characteristics of the CHIR-12.12 antibody that are equally relevant to the treatment of inflammatory diseases and/or autoimmune diseases using anti-CD40 antibodies.

### Example 1: Analysis of ADCC in cell lines

CHIR-12.12 and rituximab were compared for their relative ADCC activity against a variety of malignant B-cell lines expressing both CD40 and CD20 antigens, including lymphoma cell lines (Daudi, Namalwa), multiple myeloma cell lines (ARH77, IM-9), a B-ALL cell line (CCRF-SB), and a B-CLL cell line (EHEB).

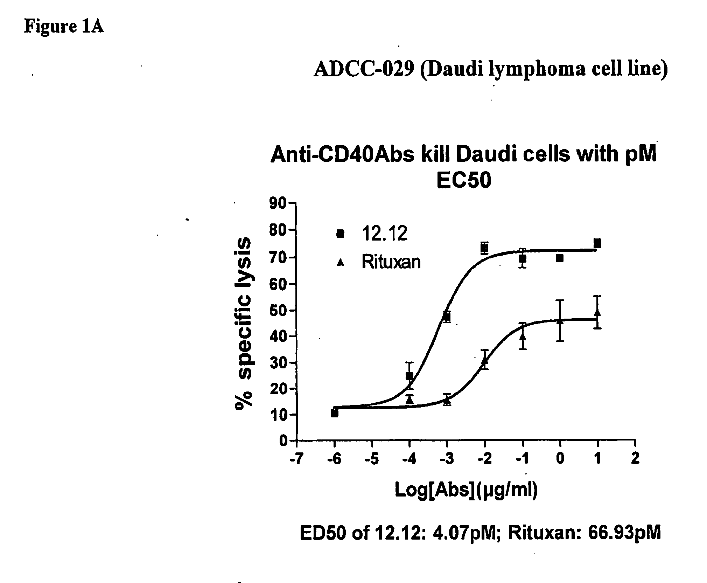

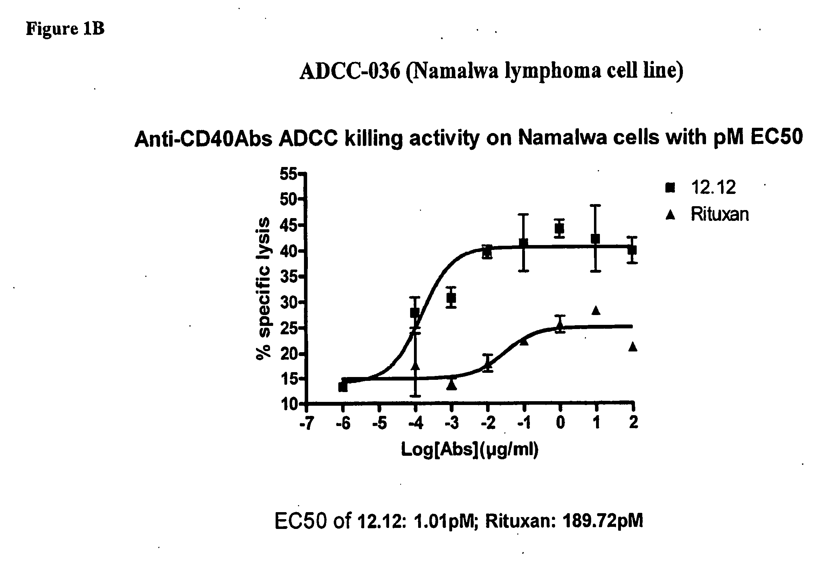

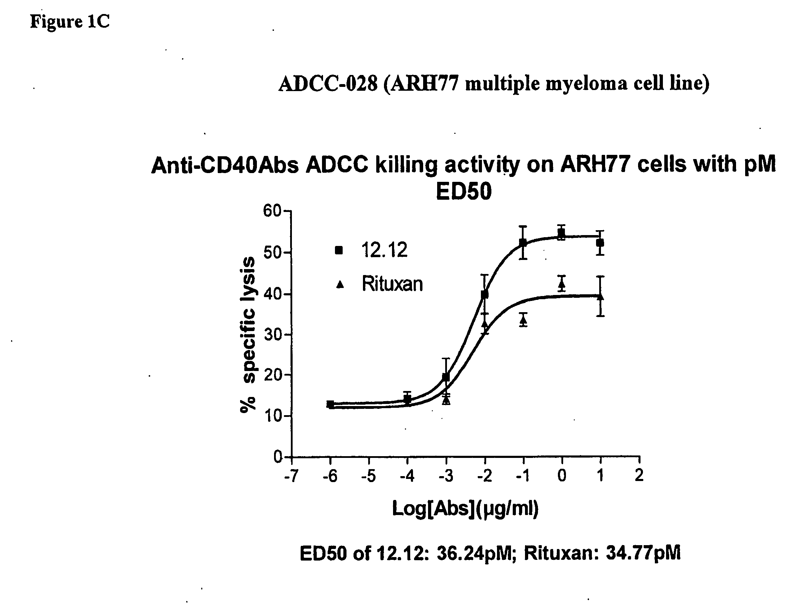

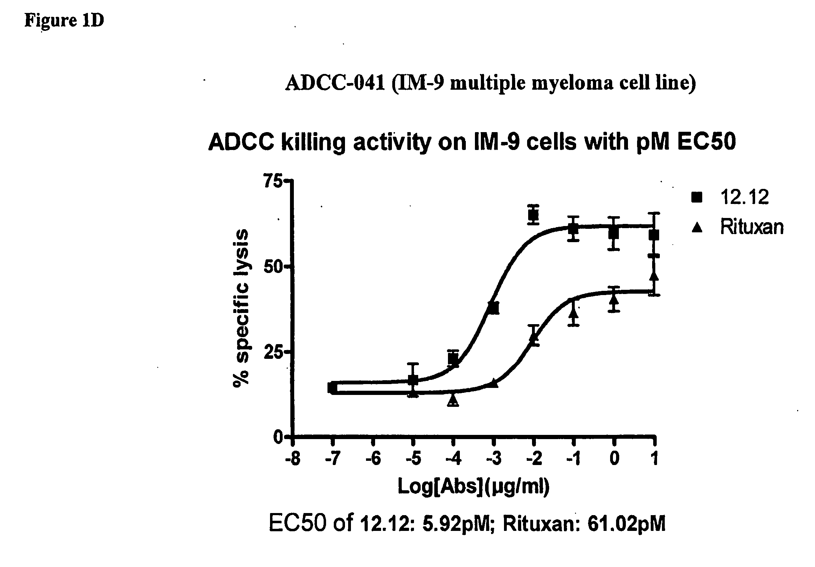

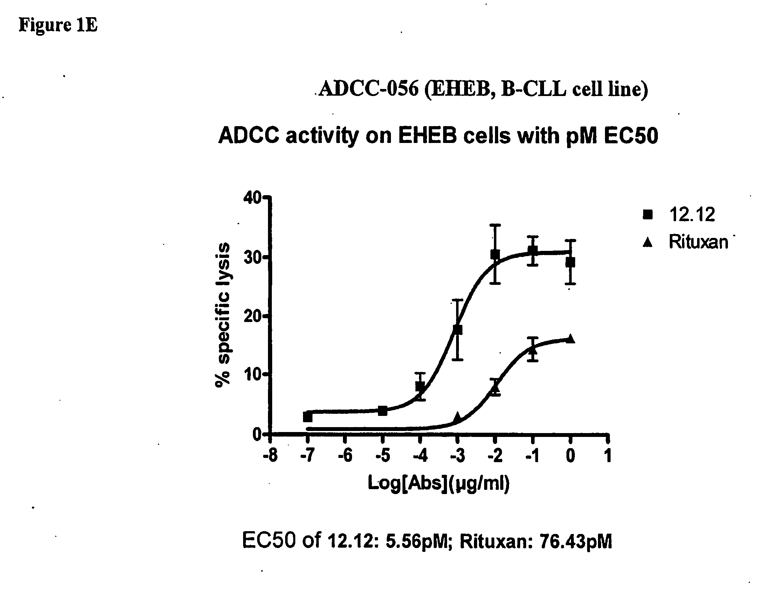

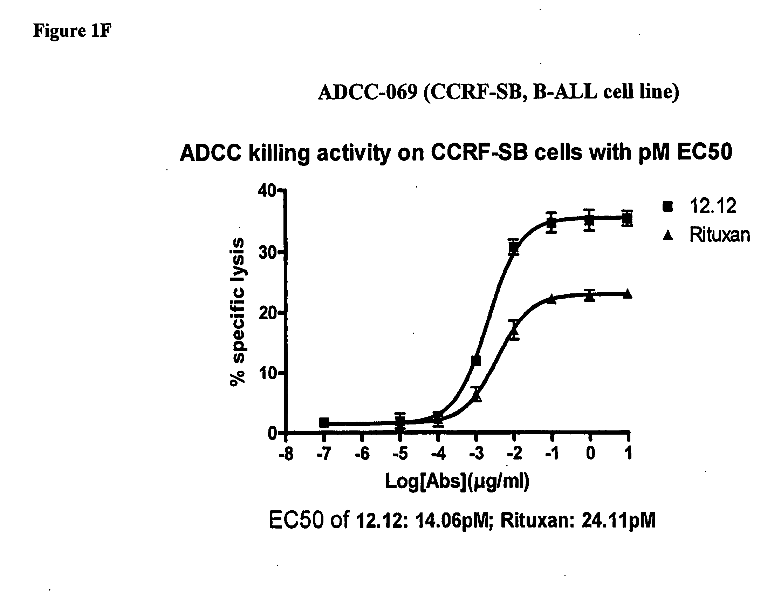

The ADCC efficacy and potency measured as maximum percent lysis and ED50, respectively, were compared for CHIR-12.12 and rituximab. The results of these experiments are shown in Figures 1A-1F. For all target cell lines, CHIR-12.12 was a more potent and efficacious mediator of ADCC than rituximab. In the six cell lines tested, the number of cell surface CD20 molecules per cell were 2.6 to 30.8-fold higher than CD40. These data show that despite displaying fewer CD40 molecules than CD20, malignant B-cell lines are more effectively lysed by CHIR-12.12 than rituximab.

### Example 2: Analysis of ADCC in CLL patient cells

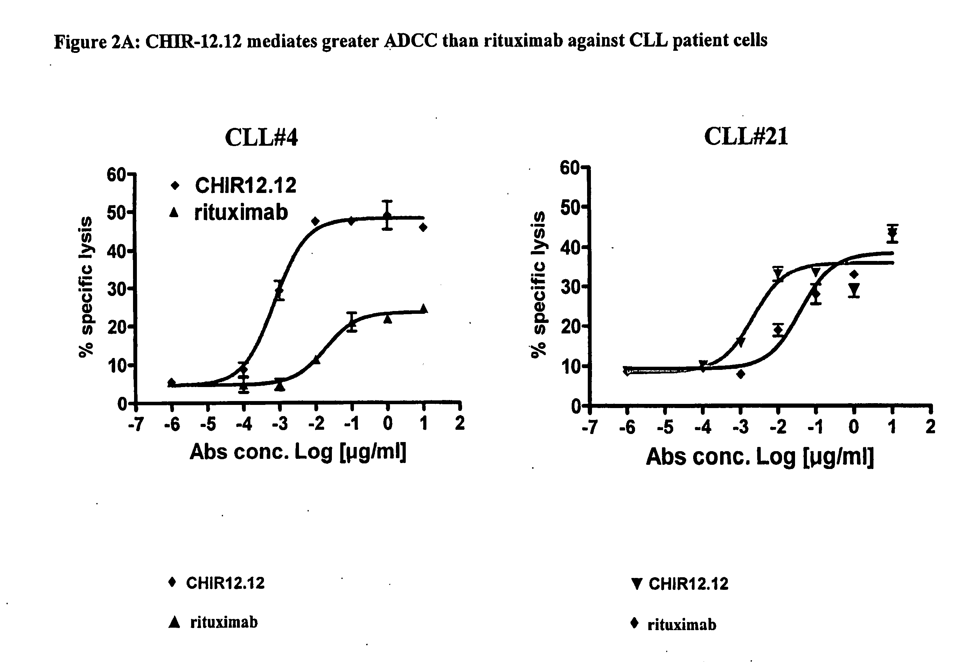

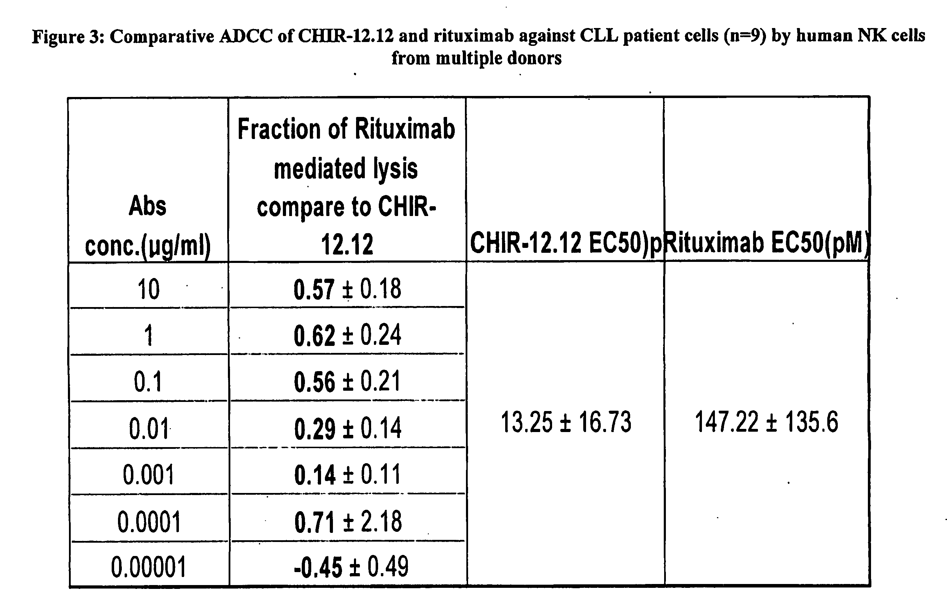

The relative ADCC activity of CHIR-12.12 and rituximab against *ex vivo* primary CLL cells from 8 patients was compared. CHIR-12.12 exhibited greater ADCC than rituximab against CLL from all patients (see Figure 2A-D and Figure 3). The results are summarized in Figure 3. CHIR-12.12 is more potent than rituximab.

### Antibody-Dependent Cellular Cytotoxicity (ADCC) Experiment Design

Target cells: CLL patient cells, 5000/well. Effector cells: purified normal human NK cells, 50,000/well. E:T ratio: 10. Abs concentration: 0.00001, 0.0001, 0.001, 0.01, 0.1, 1 and 10 µg/ml. Incubation time: 4 hrs. Culture medium: RPMI (w/o Phenol red) + 10% FBS + 1% P/S. Culture device: 96-well round bottom plate. Readout: Calcein AM release measured by Arbitrary Fluorescent Units (AFU) with 485nm excitation/535 nm emission. Calculation: % specific lysis = 100 x (AFU test - AFU spontaneous release 1) / (AFU maximal release2 - AFU spontaneous). Negative control: Calcein released by target cells in the absence of antibody or NK cell. Positive control: Calcein released by target cells upon lysis by detergent (1% NP40).

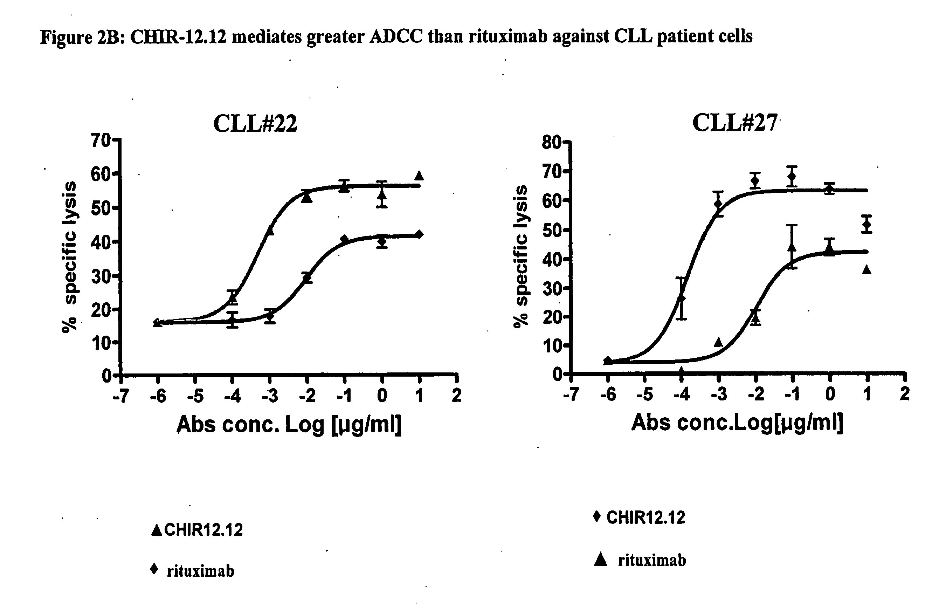

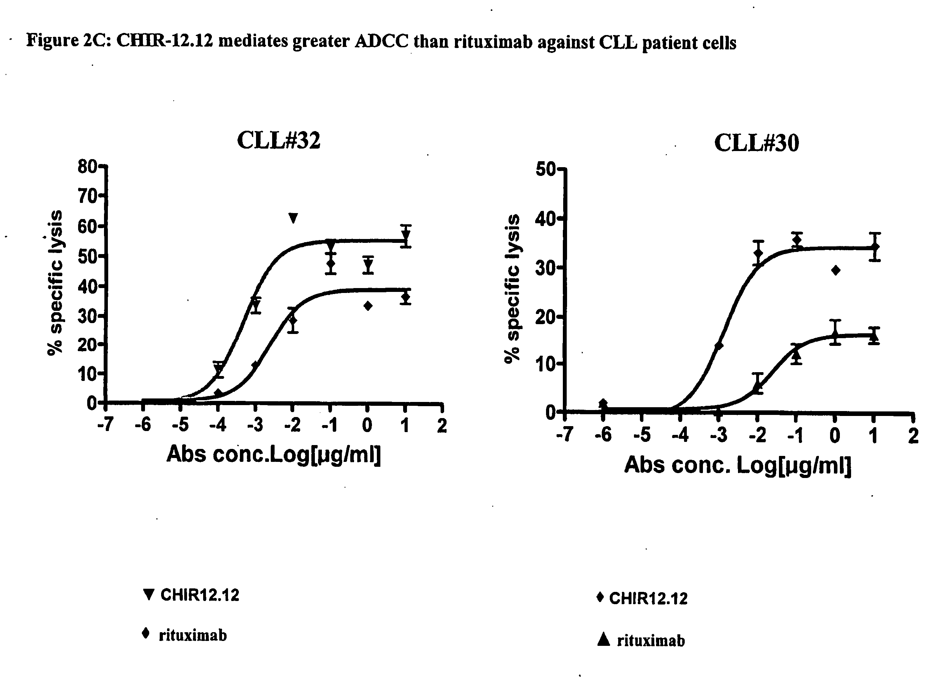

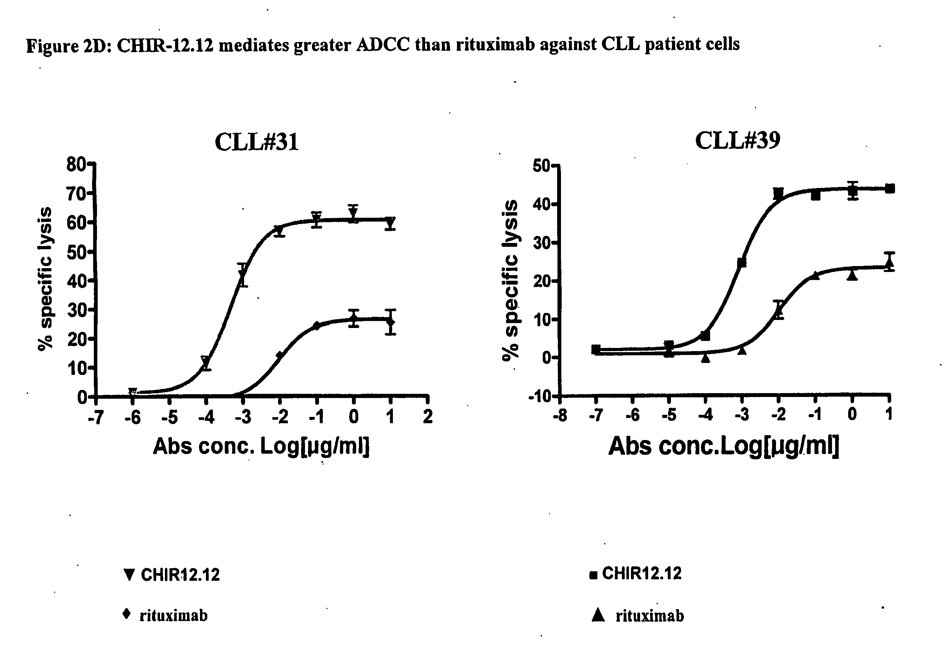

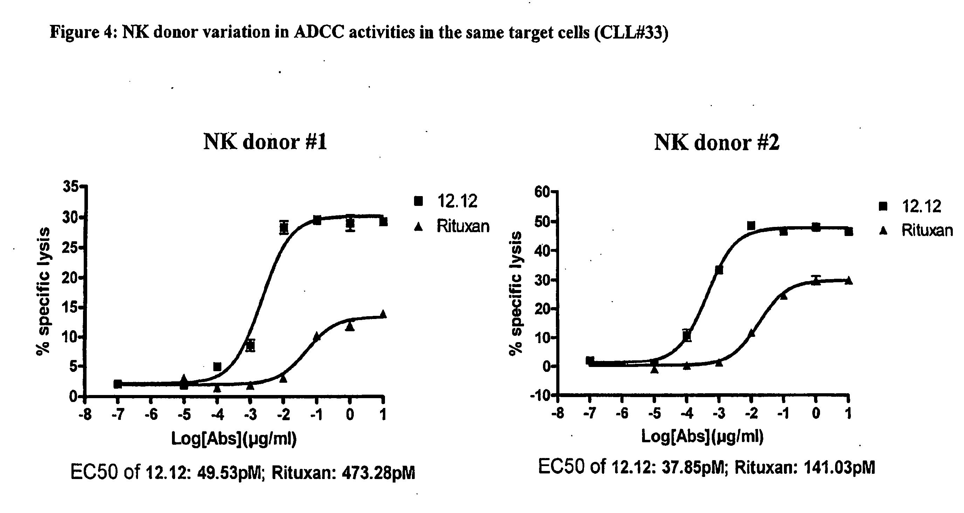

The results illustrated in Figures 2 and 3 show that CHIR-12.12 mediates greater ADCC than rituximab against CLL patient cells. The magnitude of the ADCC difference may depend on either the target cells or the NK donor cells but was observed against all patient samples. When CLL cells from single patient were tested with two different NK donors, CHIR-12.12 mediated greater ADCC than rituximab for both NK donor cells, although the magnitude of the differential ADCC was not identical (see Figure 4). The mechanistic basis for this superior ADCC might include the relative expression levels of the target antigens (CD20 and CD40), the extent of internalization of the antibody, and the affinity of the antibody for the FcγIIIa receptor on NK cells. Therefore the influence of these factors on the ADCC activity of CHIR-12.12 and rituximab. was investigated.

### Example 3: Quantitation of cell-surface CD40 and CD20 molecules

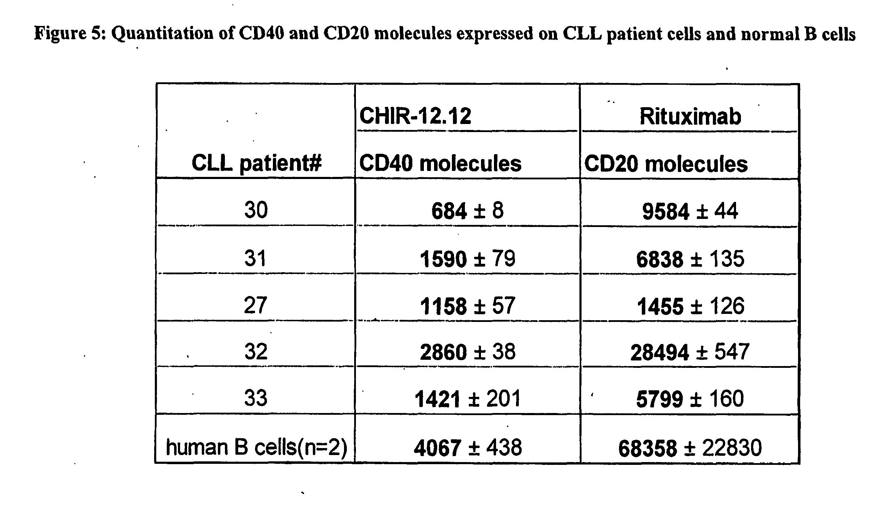

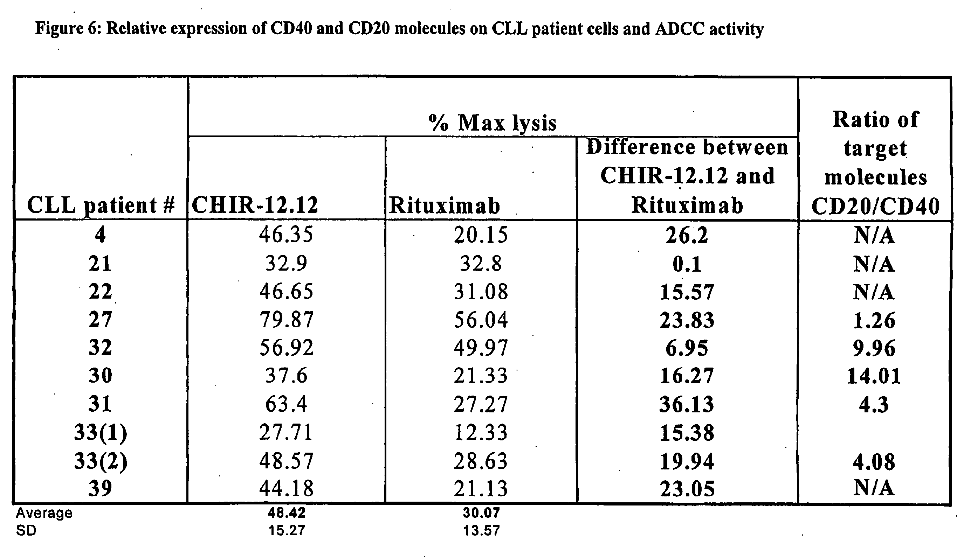

Quantitative CD20 and CD40 density on CLL cells (Example 3) and the degree of antibody internalization (Example 4) were investigated as potential reasons for the above-described difference in ADCC activity. The greater ADCC activity and efficacy of CHIR-12.12 was not dependent on a higher density of cell surface CD40 molecules, as there were 1.3- to 14-fold higher numbers of CD20 than CD40 molecules on the cell surface (see Figure 5 and Figure 6).

### Methods

Cells were preincubated with human IgG1 at 1 mg/ml in staining buffer (PBS contains 1% BSA, 0.1 % Na Azide) to block non-specific binding sites. They were incubated for 30 minutes at 4°C (on ice). Then FITC-conjugated human IgG1 isotype control, FITC-conjugated CHIR-12.12, or FITC-conjugated rituximab was added at 100, 10, 1, 0.1 µg/ml, and cells were incubated for 30 minutes at 4°C (on ice). Cells were washed with staining buffer (PBS+1%FBS+0.1% Sodium Azide), and analyzed by FACS Calibur.

Geometric mean fluorescence intensity was measured by FACS. Molecules of Equivalent Soluble Fluorchrome (MESF) were then calculated based on the standard curve established by calibrated FITC beads.

### Example 4: CHIR-12.12 does not induce internalization upon binding to CD40 on cell lines

Daudi, a lymphoma cell line, and ARH77, an MM cell line, were used to evaluate the effect of CHIR-12.12 binding on internalization. Cells were incubated with human IgG1 (control antibody) or CHIR-12.12 at 1 µg/mL on ice (with 0.1% sodium azide to block internalization) or 37°C (without sodium azide) for 3 hours. After a wash with cold staining buffer (PBS + 1% BSA + 0.1% sodium azide), cells were stained with goat anti-human IgG-FITC for 30 minutes on ice. Geometric mean fluorescent intensity (MFI) was recorded by FACS Calibur. No difference in MFI was observed between cells incubated with CHIR-12.12 on ice in the presence of sodium azide or at 37°C in the absence of sodium azide (Figure 7). These data show that CHIR-12.12, upon binding to CD40, is not internalized and continues to be displayed on the cell surface.

### Example 5: Internalization of CHIR-12.12 and Rituximab following binding to CLL patient cells: FACS and confocal microscope

### FACS Methodology

Cells were incubated with huIgG1, CHIR-12.12, or rituximab at 10 µg/ml for 3 hrs at 40°C (with 0.1% Na azide) or 37°C (w/o Na azide). Cells were washed with staining buffer (PBS + 1% FBS + 0.1% Na Azide), FITC-Goat-anti-human IgG was then added, and then cells were incubated for 30 minutes at 40°C, and analyzed by FACS Calibur.

### Confocal Microscope Methodology

Cells were incubated with Alexa 488 or FITC conjugated CHIR-12.12, rituximab, and IgG1 at 10 µg/ml, for 3 hrs at 40°C (with 0.1% Na azide) or 37°C (w/o Na azide). Cells were then washed and fixed with 2% formaldehyde, 5 min RT. Cells were then washed and placed on poly-L-lysine coated slides, mounted, and sealed, and then analyzed by confocal imaging.

### Results

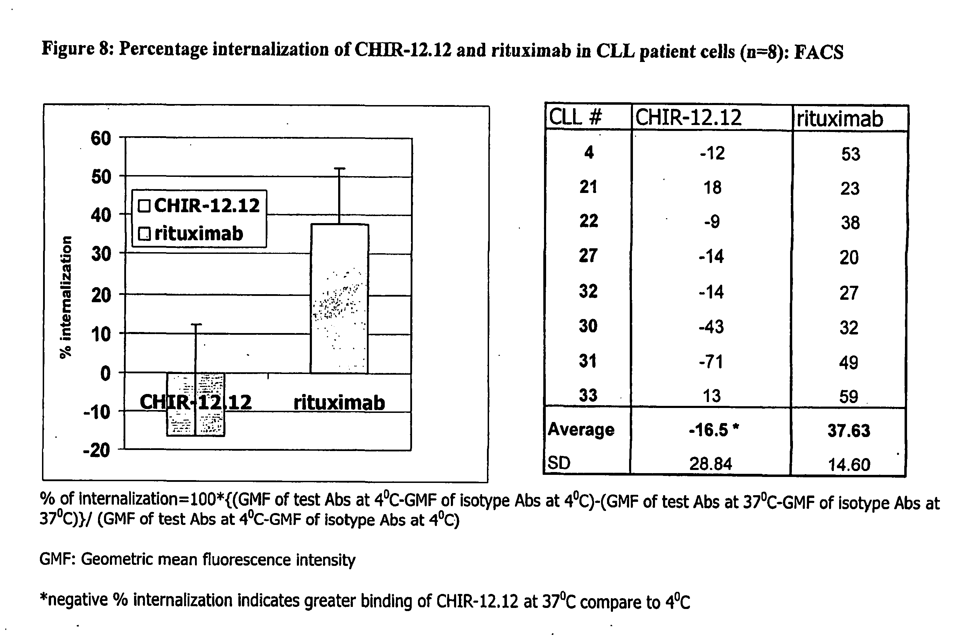

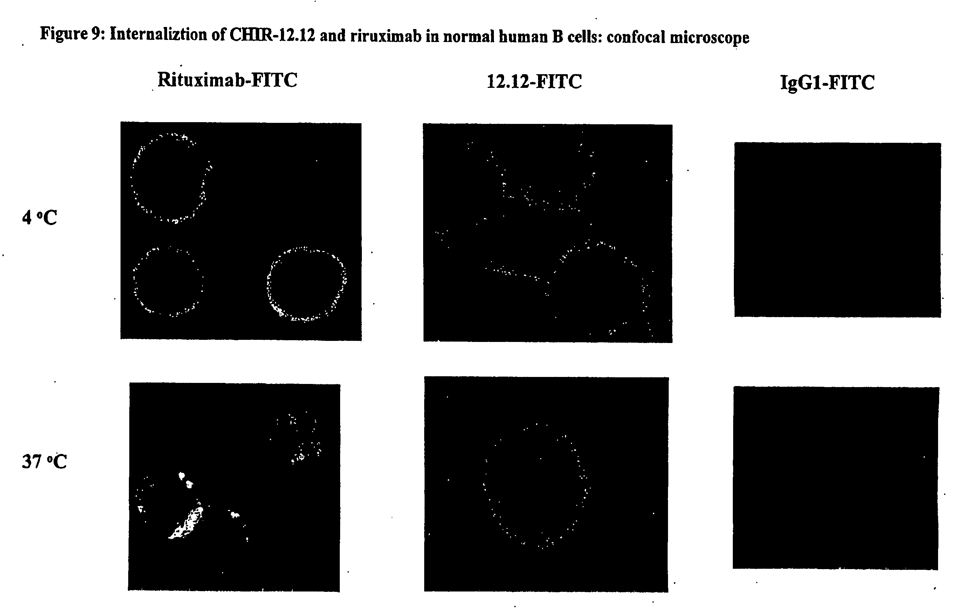

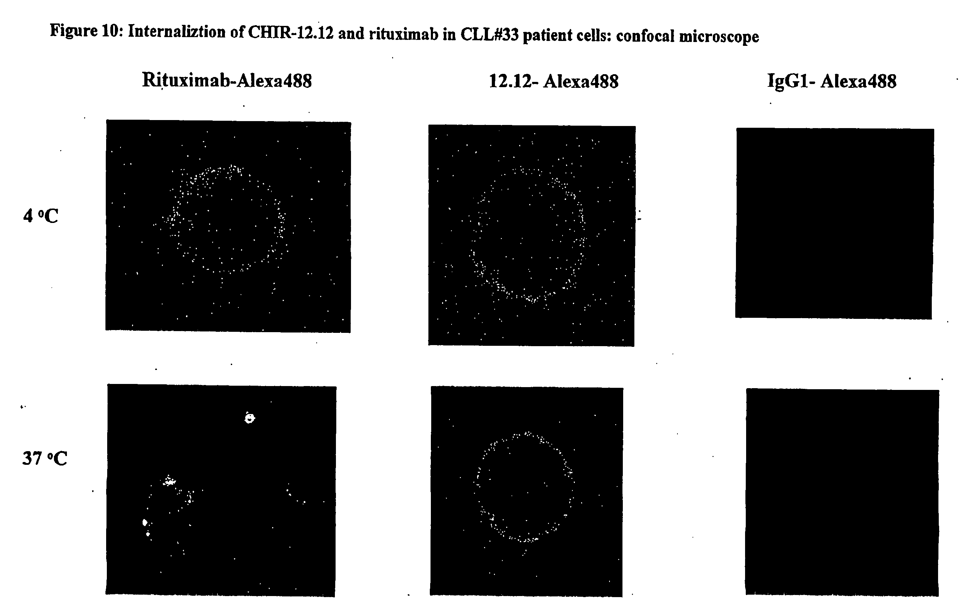

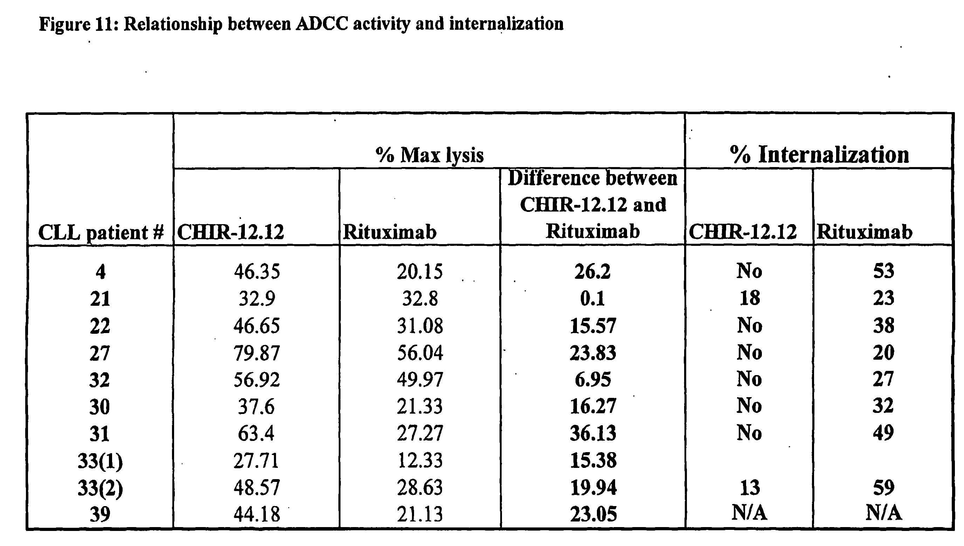

The results of these experiments are illustrated in Figure 8 (FACS) and Figures 9 and 10 (confocal microscope). The results from these experiments are summarized in Figure 11. These antibody internalization studies using primary CLL cells conducted by flow cytometry and confocal microscopy show that upon binding to CD40 at 37°C, CHIR-12.12 remains uniformly distributed on the cell surface, even after 3 hours. In contrast, after binding at 37°C, rituximab is redistributed into caps and internalized. These data suggest that the potent ADCC activity of CHIR-12.12 may be related to its ability to display itself uniformly on the surface of target cells, allowing optimal interaction with effector cells. These results suggest that CHIR-12.12 may be effective at mediating potent ADCC against CLL cells *in vivo.*

### Example 6: Biacore analysis of FcγRIIIa binding by Rituxan® and CHIR-12.12

The affinities of the FcγRIIIa aa158F and aa158V alleles for CHIR-12.12 and rituximab were compared by standard Biacore® analysis. CHIR-12.12 bound the aa158F allele with a 4.6-fold higher affinity when compared with rituximab (K_{D} of 2.8 µM versus 13 µM, respectively). The results of these experiments are summarised in the following table:

| | **K_{D} (nM)** | |
|---|---|---|
| | *CHIR-12.12* | *Rituximab* |
| FcγRIIIa 158V | 492 | 466 |
| FcγRIIIa 158F | 2800 | 13000 |

### Example 7: The effect of FcγRIIIa polymorphism on ADCC by NK effector cells

Antibody-dependent cellular cytotoxicity (ADCC) is a major mechanism of action for many marketed and investigational monoclonal antibodies. Rituximab (Rituxan®), marketed for the treatment of follicular non-Hodgkin's lymphoma (NHL) and active in other B-cell malignancies, is thought to have ADCC as one of its primary mechanisms of action. Notably, the clinical activity of rituximab in NHL has been shown to be correlated with the FcγRIIIa genotype. Patients with the FcγRIIIa 158aa polymorphism of V/V or V/F are more responsive to rituximab than those with F/F (for example, see Cartron et al. (2002) Blood 99(3):754-758 or Dall'Ozzo et al. (2004) Cancer Res. 64:4664-4669).

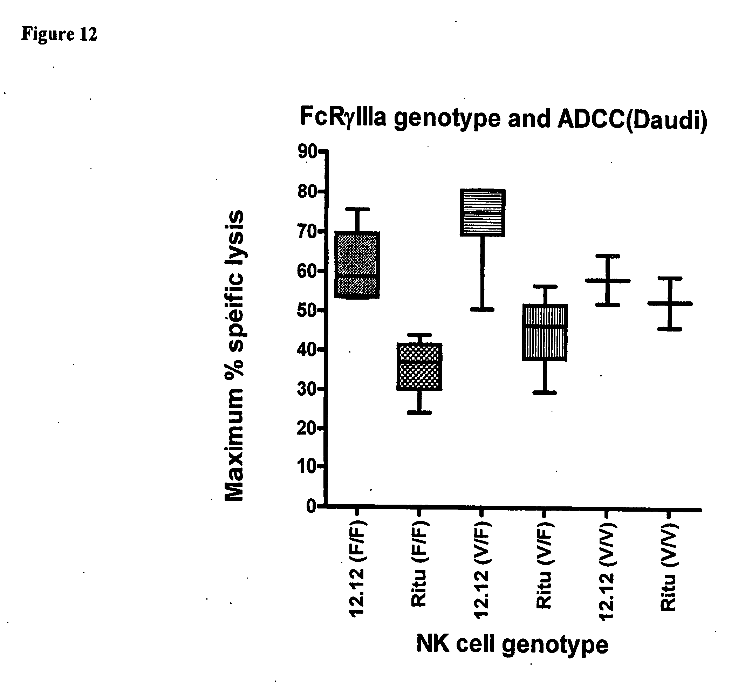

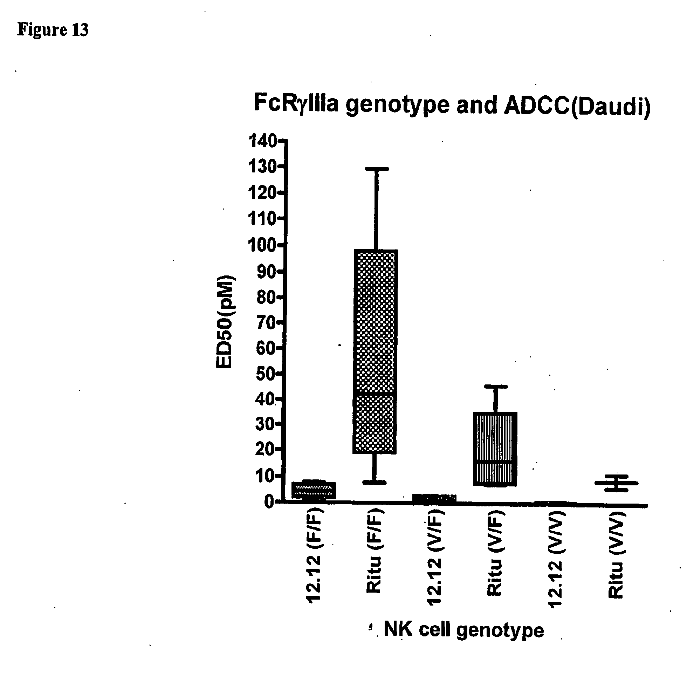

In these experiments, purified NK effector cells from multiple human donors expressing various FcγRIIIa aa158 polymorphisms were evaluated using the human lymphoma Daudi cell line as the target cells (see Figures 12 and 13). As illustrated by those figures, CHIR-12.12 induced potent ADCC with NK cells of all three genotypes. The CHIR-12.12 ED₅₀s for lysis of the Daudi cell line were 4, 2, and 0.4 pM for F/F, V/F and V/V, respectively (Figure 13). The rituximab ED₅₀s for lysis of the Daudi cell line were 53, 21, and 9 pM for F/F, V/F, and V/V, respectively (Figure 13).

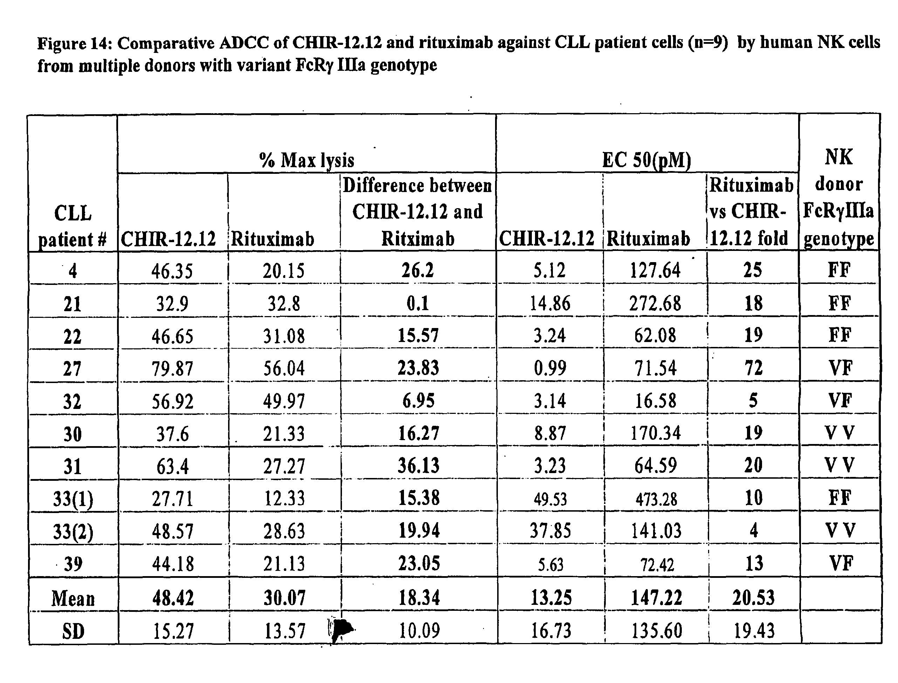

Purified NK effector cells from multiple human donors expressing various FcγRIIIa aa158 polymorphisms were also evaluated using the CLL patient cells as the target cells (see Figure 14). CHIR-12.12 was found to be a more potent mediator of ADCC than rituximab against all CLL patient cells tested (Figure 14). These data suggest that CHIR-12.12 is a more potent ADCC mediator than rituximab, even with NK cells of the aa158 V/F or F/F genotype.

These findings are surprising because it would have been expected that CHIR-12.12 would be significantly less potent in ADCC assays using NK cells with the FcγRIIIa 158aa polymorphism of F/F or V/F than those with V/V. Again, the clinical activity of rituximab in NHL has been shown to be correlated with the FcγRIIIa genotype. Patients with the FcγRIIIa 158aa polymorphism of V/V or V/F are more responsive to rituximab than those with F/F. Rituximab is also an IgG1 monoclonal antibody that binds to an antigen expressed on the surface of B cells, and so it would have been expected that CHIR-12.12 would display the same preference for the FxγRIIIa-158 V polymorphism. Instead, it was found that CHIR-12.12 induces potent ADCC with NK cells of all three genotypes.

## Claims

1. An anti-CD40 antibody, for use in the treatment of an inflammatory disease or autoimmune disease that is associated with CD40-expressing cells in a human patient heterozygous or homozygous for FcγRIIIa-158F (genotype V/F or F/F), wherein said antibody is selected from the group consisting of:
a) a monoclonal antibody that binds to an epitope capable of binding the monoclonal antibody CHIR-12.12 produced by the hybridoma cell line deposited with the ATCC as Patent Deposit No. PTA-5543;
b) a monoclonal antibody that binds to an epitope comprising residues 82-87 of the human CD40 sequence shown in SEQ ID NO:7 or SEQ ID NO:9; and
c) a monoclonal antibody that competes with the monoclonal antibody CHIR-12.12 produced by the hybridoma cell line deposited with the ATCC as Patent Deposit No. PTA-5543 in a competitive binding assay.

2. Use of a therapeutically or prophylactically effective amount of an anti-CD40 antibody in the manufacture of a medicament for the treatment of an inflammatory disease or autoimmune disease that is associated with CD40-expressing cells in a human patient heterozygous or homozygous for FcγRIIIa-158F (genotype V/F or F/F), wherein said antibody is selected from the group consisting of:
a) a monoclonal antibody that binds to an epitope capable of binding the monoclonal antibody CHIR-12.12 produced by the hybridoma cell line deposited with the ATCC as Patent Deposit No. PTA-5543;
b) a monoclonal antibody that binds to an epitope comprising residues 82-87 of the human CD40 sequence shown in SEQ ID NO:7 or SEQ ID NO:9; and
c) a monoclonal antibody that competes with the monoclonal antibody CHIR-12.12 produced by the hybridoma cell line deposited with the ATCC as Patent Deposit No. PTA-5543 in a competitive binding assay.

3. A method for selecting an antibody therapy for treatment of a human patient having an inflammatory disease or autoimmune disease which is refractory to treatment with rituximab (Rituxan®), comprising determining the FcγRIIIa-158 genotype (V/V, V/F or F/F) of a human patient having an inflammatory disease or autoimmune disease that is associated with CD40-expressing cells and which is refractory to treatment with rituximab (Rituxan®) using a biological sample obtained from the human patient;
wherein if said human patient is heterozygous or homozygous for FcγRIIIa-158F (genotype V/F or F/F), an anti-CD40 antibody is selected for treatment of said inflammatory disease or autoimmune disease,
wherein said antibody is selected from the group consisting of:
a) a monoclonal antibody that binds to an epitope capable of binding the monoclonal antibody CHIR-12.12 produced by the hybridoma cell line deposited with the ATCC as Patent Deposit No. PTA-5543;
b) a monoclonal antibody that binds to an epitope comprising residues 82-87 of the human CD40 sequence shown in SEQ ID NO:7 or SEQ ID NO:9; and
c) a monoclonal antibody that competes with the monoclonal antibody CHIR-12.12 produced by the hybridoma cell line deposited with the ATCC as Patent Deposit No. PTA-5543 in a competitive binding assay.

4. The antibody of claim 1, use of claim 2 or method of claim 3, wherein said inflammatory disease or autoimmune disease is selected from the group consisting of systemic lupus erythematosus (SLE), discoid lupus, lupus nephritis, sarcoidosis, inflammatory arthritis, including juvenile arthritis, rheumatoid arthritis, psoriatic arthritis, Reiter's syndrome, ankylosing spondylitis, and gouty arthritis, rejection of an organ or tissue transplant, hyperacute, acute, or chronic rejection and/or graft versus host disease, multiple sclerosis, hyper IgE syndrome, polyarteritis nodosa, primary biliary cirrhosis, inflammatory bowel disease, Crohn's disease, celiac's disease (gluten-sensitive enteropathy), autoimmune hepatitis, pernicious anemia, autoimmune hemolytic anemia, psoriasis, scleroderma, myasthenia gravis, autoimmune thrombocytopenic purpura, autoimmune thyroiditis, Grave's disease, Hasimoto's thyroiditis, immune complex disease, chronic fatigue immune dysfunction syndrome (CFIDS), polymyositis and dermatomyositis, cryoglobulinemia, thrombolysis, cardiomyopathy, pemphigus vulgaris, pulmonary interstitial fibrosis, Type I and Type II diabetes mellitus, type 1, 2, 3, and 4 delayed-type hypersensitivity, allergy or allergic disorders, unwanted/unintended immune responses to therapeutic proteins, asthma, Churg-Strauss syndrome (allergic granulomatosis), atopic dermatitis, allergic and irritant contact dermatitis, urtecaria, IgE-mediated allergy, atherosclerosis, vasculitis, idiopathic inflammatory myopathies, hemolytic disease, Alzheimer's disease, and chronic inflammatory demyelinating polyneuropathy, pulmonary inflammation including but not limited to lung graft rejection, asthma, sarcoidosis, emphysema, cystic fibrosis, idiopathic pulmonary fibrosis, chronic bronchitis, allergic rhinitis and allergic diseases of the lung such as hypersensitivity pneumonitis, eosinophilic pneumonia, bronchiolitis obliterans due to bone marrow and/or lung transplantation or other causes, graft atherosclerosis/graft phlebosclerosis, as well as pulmonary fibrosis resulting from collagen, vascular, and autoimmune diseases such as rheumatoid arthritis and lupus erythematosus.

5. The antibody of claim 1, use of claim 2 or method of claim 3, wherein said inflammatory disease or autoimmune disease is an inflammatory disease or autoimmune disease that is associated with cells expressing both CD40 and CD20.

6. The antibody, use or method of claim 5, wherein said inflammatory disease or autoimmune disease is rheumatoid arthritis, psoriasis, systemic lupus erythematosus, Crohn's disease, myasthenia gravis, idiopathic thrombocytopenia purpura, or Sjogren's syndrome.

7. The antibody, use or method of claim 5, wherein said inflammatory disease or autoimmune disease is multiple sclerosis, graft rejection, graft versus host disease, Alzheimer's disease, or diabetes.

8. The antibody of claim 1, use of claim 2 or method of claim 3, wherein said antibody is the monoclonal antibody CHIR-12.12 produced by the hybridoma cell line deposited with the ATCC as Patent Deposit No. PTA-5543.

9. The antibody, use or method according to any one of the preceding claims, wherein said anti-CD40 antibody is a human monoclonal antibody.

10. The antibody, use or method according to claim 9, wherein said human anti-CD40 monoclonal antibody comprises a human IgG1 heavy chain constant region.

11. The antibody, use or method according to claim 10, wherein said monoclonal antibody comprises the amino acid sequence recited in SEQ ID NO:4 or SEQ ID NO:5.

12. The antibody, use or method according to any one of the preceding claims, wherein said anti-CD40 antibody binds to human CD40 with an affinity (K_{D}) of at least about 10⁻⁶ M, or at least 10⁻⁸M.

13. The antibody, use or method according to any one of the preceding claims, wherein said anti-CD40 antibody binds to human FcγRIIIa-158V with an affinity (K_{D}) of at least about 0.5 µM and/or binds to human FcγRIIIa-158F with an affinity (K_{D}) of at least about 12 µM.

14. The antibody, use or method of any preceding claim, wherein said monoclonal antibody is an antigen-binding fragment of a monoclonal antibody, wherein said fragment retains the capability of specifically binding to said human CD40 antigen.

15. The antigen-binding fragment of claim 14, wherein said fragment is selected from the group consisting of a Fab fragment, an F(ab')₂ fragment, an Fv fragment, and a single-chain Fv fragment.

16. The antibody or use according to claim 1 or claim 2, wherein said use results in antibody dependent cellular cytotoxicity (ADCC) of CD40-expressing cells by a human patient's FcγRIIIa-expressing natural killer (NK) cells.

17. The antibody, use or method according to any of claims 1-15, wherein said anti-CD40 antibody is more potent than rituximab (Rituxan^{®}) in an assay of antibody-dependent cellular cytotoxicity (ADCC), wherein the assay comprises incubating CD40-expressing cells and CD20-expressing cells with isolated human natural killer (NK) cells in the presence of the relevant antibody.

18. The antibody, use or method according to any of claims 1-16, wherein said anti-CD40 antibody is more potent than rituximab (Rituxan^{®}) in a model of systemic lupus erythematosus (SLE), multiple sclerosis, inflammation and atherosclerosis, transplantation, or Alzheimer's disease.

19. The antibody, use or method according to any preceding claim, wherein said human patient is refractory to a therapy for an inflammatory or autoimmune disease.

20. The antibody, use or method according to claim 19, wherein said human patient is refractory to therapy with an anti-CD20 monoclonal antibody.

21. The antibody, use or method according to claim 20, wherein said anti-CD20 monoclonal antibody is rituximab (Rituxan^{®}).

## Patentansprüche

1. Anti-CD40-Antikörper für die Verwendung in der Behandlung einer entzündlichen Erkrankung oder Autoimmunerkrankung, die mit CD40 exprimierenden Zellen assoziiert ist, bei einem menschlichen Patienten, der für FcγRIIIa-158F (Genotyp V/F oder F/F) heterozygot oder homozygot ist, wobei der Antikörper aus der Gruppe bestehend aus den folgenden ausgewählt ist:
a) einem monoklonalen Antikörper, der an ein Epitop bindet, das fähig ist, den bei der ATCC unter der Patenthinterlegungsnummer PTA-5543 hinterlegten Hybridomzelllinie produzierten monoklonalen Antikörper CHIR-12.12 zu binden;
b) einem monoklonalen Antikörper, der an ein Epitop bindet, das die Reste 82-87 der in SEQ ID NO: 7 oder SEQ ID NO: 9 dargestellten humanen CD40-Sequenz umfasst; und
c) einem monoklonalen Antikörper, der mit dem bei der ATCC unter der Patenthinterlegungs-Nr. PTA-5543 hinterlegten Hybridomzelllinie produzierten monoklonalen Antikörper CHIR-12.12 in einem kompetitiven Bindungs-Assay konkurriert.

2. Verwendung einer therapeutisch oder prophylaktisch wirksamen Menge eines anti-CD40-Antikörpers in der Herstellung eines Arzneimittels für die Behandlung einer entzündlichen Erkrankung oder Autoimmunerkrankung, die mit CD40 exprimierenden Zellen assoziiert ist, bei einem menschlichen Patienten, der für FcγRIIIa-158F (Genotyp V/F oder F/F) heterozygot oder homozygot ist, wobei der Antikörper aus der Gruppe bestehend aus den folgenden ausgewählt ist:
a) einem monoklonalen Antikörper, der an ein Epitop bindet, das fähig ist, den bei der ATCC unter der Patenthinterlegungsnummer PTA-5543 hinterlegten Hybridomzelllinie produzierten monoklonalen Antikörper CHIR-12.12 zu binden;
b) einem monoklonalen Antikörper, der an ein Epitop bindet, das die Reste 82-87 der in SEQ ID NO: 7 oder SEQ ID NO: 9 dargestellten humanen CD40-Sequenz umfasst; und
c) einem monoklonalen Antikörper, der mit dem bei der ATCC unter der Patenthinterlegungs-Nr. PTA-5543 hinterlegten Hybridomzelllinie produzierten monoklonalen Antikörper CHIR-12.12 in einem kompetitiven Bindungs-Assay konkurriert.

3. Verfahren zum Auswählen einer Antikörpertherapie für die Behandlung eines menschlichen Patienten, der an einer entzündlichen Krankheit oder Autoimmunkrankheit, die auf Behandlung mit Rituximab (Rituxan®) refraktär ist, leidet, bei dem man den FcγRIIIa-158-Genotyp (V/V, V/F oder F/F) eines menschlichen Patienten, der an einer entzündlichen Krankheit oder Autoimmunkrankheit, die mit CD40-exprimierenden Zellen assoziiert ist und die auf Behandlung mit Rituximab (Rituxan®) refraktär ist, leidet, unter Einsatz einer von dem menschlichen Patienten stammenden biologischen Probe;
wobei, wenn der menschliche Patient auf FcγRIIIa-158F (Genotyp V/F oder F/F) heterozygot oder homozygot ist, ein anti-CD40-Antikörper für die Behandlung der entzündlichen Krankheit oder Autoimmunkrankheit ausgewählt wird, wobei der Antikörper aus der Gruppe bestehend aus den folgenden ausgewählt ist:
a) einem monoklonalen Antikörper, der an ein Epitop bindet, das fähig ist, den bei der ATCC unter der Patenthinterlegungsnummer PTA-5543 hinterlegten Hybridomzelllinie produzierten monoklonalen Antikörper CHIR-12.12 zu binden;
b) einem monoklonalen Antikörper, der an ein Epitop bindet, das die Reste 82-87 der in SEQ ID NO: 7 oder SEQ ID NO: 9 dargestellten humanen CD40-Sequenz umfasst; und
c) einem monoklonalen Antikörper, der mit dem bei der ATCC unter der Patenthinterlegungs-Nr. PTA-5543 hinterlegten Hybridomzelllinie produzierten monoklonalen Antikörper CHIR-12.12 in einem kompetitiven Bindungs-Assay konkurriert.

4. Antikörper nach Anspruch 1, Verwendung nach Anspruch 2 oder Verfahren nach Anspruch 3, wobei die entzündliche Krankheit oder Autoimmunkrankheit aus der folgenden Gruppe ausgewählt ist: systemischer Lupus erythematodes (SLE), diskoider Lupus, Lupus-Nephritis, Sarkoidose, entzündliche Arthritis, einschließlich juvenile Arthritis, rheumatoide Arthritis, Arthritis psoriatica, Reiter-Syndrom, ankylosierende Spondylitis, Gichtarthritis, Organ- oder Gewebetransplantatabstoßung, hyperakute, akute oder chronische Abstoßung und/oder Transplantat-Wirt-Reaktion, Multiple Sklerose, Hyper-IgE-Syndrom, Polyarteriitis nodosa, primär biliäre Zirrhose, Reizdarm, Morbus Crohn, Zöliakie (glutenempfindliche Enteropathie), Autoimmunhepatitis, perniziöse Anämie, autoimmunhämolytische Anämie, Schuppenflechte, Sklerodermie, Myasthenia gravis, immunbedingte thrombozytopenische Purpura, auto-immune Schilddrüsenentzündung, Morbus Basedow, Hasimoto-Schilddrüsenentzündung, Immunkomplexkrankheit, chronisches Müdigkeits- und Immundysfunktionssyndrom (CFIDS), Polymyositis und Dermatomyositis, Kryoglobulinämie, Thrombolyse, Kardiomyopathie, Pemphigus vulgaris, interstitielle Lungenfibrose, Diabetes mellitus Typ I und Typ II, DTH (Delayed-Type Hypersensitivity) Typ 1, 2, 3 und 4, Allergie oder allergische Erkrankungen, unerwünschte bzw. unbeabsichtigte Immunreaktionen auf therapeutische Proteine, Asthma, Churg-Strauss-Syndrom (allergische Granulomatose), atopische Dermatitis, allergische und reizende Kontaktdermatitis, Nesselsucht, IgEvermittelte Allergie, Atherosklerose, Vaskulitis, idiopathische entzündliche Myopathien, hämolytische Erkrankungen, Morbus Alzheimer und chronische entzündliche demyelinierende Polyneuropathie, Lungenentzündung, darunter, jedoch nicht ausschließlich, Lungentransplantatabstoßung, Asthma, Sarkoidose, Emphysem, Mukoviszidose, idiopathische Lungenfibrose, chronische Bronchitis, allergische Rhinitis und allergische Lungenerkrankungen, Hypersensitivitätspneumonitis, eosinophile Pneumonie, Bronchiolitis obliterans aufgrund von Knochenmarks- und/oder Lungentransplantation oder anderen Ursachen, Transplantat-Atherosklerose/Transplantat-Phlebosklerose, sowie Lungenfibrose aufgrund von Collagen-, Gefäß- und Autoimmunerkrankungen, wie rheumatoide Arthritis und Lupus erythematodes.

5. Antikörper nach Anspruch 1, Verwendung nach Anspruch 2 oder Verfahren nach Anspruch 3, wobei es sich bei der entzündlichen Krankheit oder Autoimmunkrankheit um eine entzündliche Krankheit oder Autoimmunkrankheit handelt, die mit Zellen die sowohl CD40 als auch CD20 exprimieren, assoziiert ist.

6. Antikörper, Verwendung oder Verfahren nach Anspruch 5, wobei es sich bei der entzündlichen Erkrankung oder Autoimmunerkrankung um rheumatoide Arthritis, Schuppenflechte, systemischen Lupus erythematodes, Morbus Crohn, Myasthenia gravis, idiopathische thrombozytopenische Purpura oder Sjogren-Syndrom handelt.

7. Antikörper, Verwendung oder Verfahren nach Anspruch 5, wobei es sich bei der entzündlichen Erkrankung oder Autoimmunerkrankung um Multiple Sklerose, Transplantatabstoßung, Transplantat-Wirt-Reaktion, Morbus Alzheimer oder Diabetes handelt.

8. Antikörper nach Anspruch 1, Verwendung nach Anspruch 2 oder Verfahren nach Anspruch 3, wobei es sich bei dem Antikörper um den von der Hybridomzelllinie, die bei der ATCC unter Patenthinterlegungs-Nr. PTA-5543 hinterlegt ist, produzierten monoklonalen Antikörper CHIR-12.12 handelt.

9. Antikörper, Verwendung oder Verfahren nach einem der vorhergehenden Ansprüche, wobei es sich bei dem anti-CD40-Antikörper um einen humanen monoklonalen Antikörper handelt.

10. Antikörper, Verwendung oder Verfahren nach Anspruch 9, wobei der humane monoklonale anti-CD40-Antikörper eine konstante Region der schweren Kette von Human-IgG1 umfasst.

11. Antikörper, Verwendung oder Verfahren nach Anspruch 10, wobei der monoklonale Antikörper die in SEQ ID NO: 4 oder SEQ ID NO: 5 genannte Aminosäuresequenz umfasst.

12. Antikörper, Verwendung oder Verfahren nach einem der vorhergehenden Ansprüche, wobei der anti-CD40-Antikörper an Human-CD40 mit einer Affinität (K_{D}) von mindestens ungefähr 10⁻⁶ M oder mindestens 10⁻⁸ M bindet.

13. Antikörper, Verwendung oder Verfahren nach einem der vorhergehenden Ansprüche, wobei der anti-CD40-Antikörper an Human-FcγRIIIa-158V mit einer Affinität (K_{D}) von mindestens ungefähr 0,5 µM bindet und/oder an Human-FcγRIIIa-158F mit einer Affinität (K_{D}) von mindestens ungefähr 12 µM bindet.

14. Antikörper, Verwendung oder Verfahren nach einem der vorhergehenden Ansprüche, wobei es sich bei dem monoklonalen Antikörper um ein antigenbindendes Fragment eines monoklonalen Antikörpers handelt, wobei das Fragment die Fähigkeit, spezifisch an das Human-CD40-Antigen zu binden, beibehält.

15. Antigenbindendes Fragment nach Anspruch 14, wobei das Fragment aus der Gruppe bestehend aus einem Fab-Fragment, einem F(ab')₂-Fragment, einem Fv-Fragment und einem Einzelketten-Fv-Fragment ausgewählt ist.

16. Antikörper oder Verwendung nach Anspruch 1 oder Anspruch 2, wobei die Verwendung zu einer antikörperabhängigen Zell-Zytotoxizität (ADCC) von CD40-exprimierenden Zellen durch FcγRIIIa exprimierende natürliche Killerzellen (NK-Zellen) eines menschlichen Patienten führt.

17. Antikörper, Verwendung oder Verfahren nach einem der Ansprüche 1-15, wobei der anti-CD40-Antikörper in einem Assay auf antikörperabhängige Zell-Zytotoxizität (ADCC) wirksamer als Rituximab (Rituxan®) ist, wobei in diesem Assay CD40 exprimierende Zellen und CD20 exprimierende Zellen in Gegenwart des entsprechenden Antikörpers mit isolierten menschlichen natürlichen Killerzellen (NK-Zellen) inkubiert werden.

18. Antikörper, Verwendung oder Verfahren nach einem der Ansprüche 1-16, wobei der anti-CD40-Antikörper in einem systemischen Lupus-erythematodes-Modell (SLE-Modell), Multiple-Sklerose-Modell, Entzündungs- und Atherosklerosemodell, Transplantationsmodell oder Morbus-Alzheimer-Modell wirksamer als Rituximab (Rituxan®) ist.

19. Antikörper, Verwendung oder Verfahren nach einem der vorhergehenden Ansprüche, wobei der menschliche Patient auf eine Therapie für eine entzündliche Erkrankung oder Autoimmunerkrankung refraktär ist.

20. Antikörper, Verwendung oder Verfahren nach Anspruch 19, wobei der menschliche Patient auf Therapie mit einem monoklonalen anti-CD20-Antikörper refraktär ist.

21. Antikörper, Verwendung oder Verfahren nach Anspruch 20, wobei es sich bei dem monoklonalen anti-CD20-Antikörper um Rituximab (Rituxan®) handelt.

## Revendications

1. Anticorps anti-CD40, destiné à être utilisé dans le traitement d'une maladie inflammatoire ou d'une maladie auto-immune qui est associée à des cellules exprimant le CD40 chez un patient humain hétérozygote ou homozygote pour FcγRIIIa-158F (génotype V/F ou F/F), ledit anticorps étant choisi dans le groupe constitué par :
a) un anticorps monoclonal qui se lie à un épitope capable de lier l'anticorps monoclonal CHIR-12.12 produit par la lignée cellulaire d'hybridome déposée auprès de ATCC sous le n° de dépôt de brevet PTA-5543 ;
b) un anticorps monoclonal qui se lie à un épitope comprenant les résidus 82-87 de la séquence de CD40 humain présentée dans SEQ ID NO:7 ou SEQ ID NO:9 ; et
c) un anticorps monoclonal qui entre en compétition avec l'anticorps monoclonal CHIR-12.12 produit par la lignée cellulaire d'hybridome déposée auprès de ATCC sous le n° de dépôt de brevet PTA-5543 dans un essai de liaison compétitive.

2. Utilisation d'une quantité thérapeutiquement ou prophylactiquement efficace d'un anticorps anti-CD40 dans la fabrication d'un médicament destiné au traitement d'une maladie inflammatoire ou d'une maladie auto-immune qui est associée à des cellules exprimant le CD40 chez un patient humain hétérozygote ou homozygote pour FcγRIIIa-158F (génotype V/F ou F/F), ledit anticorps étant choisi dans le groupe constitué par :
a) un anticorps monoclonal qui se lie à un épitope capable de lier l'anticorps monoclonal CHIR-12.12 produit par la lignée cellulaire d'hybridome déposée auprès de ATCC sous le n° de dépôt de brevet PTA-5543 ;
b) un anticorps monoclonal qui se lie à un épitope comprenant les résidus 82-87 de la séquence de CD40 humain présentée dans SEQ ID NO:7 ou SEQ ID NO:9 ; et
c) un anticorps monoclonal qui entre en compétition avec l'anticorps monoclonal CHIR-12.12 produit par la lignée cellulaire d'hybridome déposée auprès de ATCC sous le n° de dépôt de brevet PTA-5543 dans un essai de liaison compétitive.

3. Méthode de sélection d'une thérapie par anticorps pour le traitement d'un patient humain ayant une maladie inflammatoire ou une maladie auto-immune qui est réfractaire au traitement par le rituximab (Rituxan®), comprenant la détermination du génotype (V/V, V/F ou F/F) de FcγRIIIa-158 d'un patient humain ayant une maladie inflammatoire ou une maladie auto-immune qui est associée à des cellules exprimant le CD40 et qui est réfractaire au traitement par le rituximab (Rituxan®) en utilisant un échantillon biologique obtenu chez le patient humain ;
où si ledit patient humain est hétérozygote ou homozygote pour FcγRIIIa-158F (génotype V/F ou F/F), un anticorps anti-CD40 est choisi pour le traitement de ladite maladie inflammatoire ou maladie auto-immune,
ledit anticorps étant choisi dans le groupe constitué par :
a) un anticorps monoclonal qui se lie à un épitope capable de lier l'anticorps monoclonal CHIR-12.12 produit par la lignée cellulaire d'hybridome déposée auprès de ATCC sous le n° de dépôt de brevet PTA-5543 ;
b) un anticorps monoclonal qui se lie à un épitope comprenant les résidus 82-87 de la séquence de CD40 humain présentée dans SEQ ID NO:7 ou SEQ ID NO:9 ; et
c) un anticorps monoclonal qui entre en compétition avec l'anticorps monoclonal CHIR-12.12 produit par la lignée cellulaire d'hybridome déposée auprès de ATCC sous le n° de dépôt de brevet PTA-5543 dans un essai de liaison compétitive.

4. Anticorps selon la revendication 1, utilisation selon la revendication 2 ou méthode selon la revendication 3, **caractérisés en ce que** ladite maladie inflammatoire ou maladie auto-immune est choisie dans le groupe constitué par le lupus érythémateux disséminé (SLE), le lupus discoïde, la néphrite lupique, la sarcoïdose, l'arthrite inflammatoire, y compris l'arthrite juvénile, la polyarthrite rhumatoïde, l'arthrite psoriasique, le syndrome de Reiter, la spondylarthrite ankylosante et l'arthrite goutteuse, le rejet de greffe d'organe ou de tissu, le rejet hyper aigu, aigu ou chronique et/ou la maladie du greffon contre l'hôte, la sclérose en plaques, le syndrome hyper-IgE, la polyartérite noueuse, la cirrhose biliaire primaire, la maladie inflammatoire de l'intestin, la maladie de Crohn, la maladie coeliaque (entéropathie au gluten), l'hépatite auto-immune, l'anémie pernicieuse, l'anémie hémolytique auto-immune, le psoriasis, la sclérodermie, la myasthénie grave, le purpura thrombocytopénique auto-immun, la thyroïdite auto-immune, la maladie de Grave, la thyroïdite de Hashimoto, la maladie des complexes immuns, le syndrome de fatigue chronique lié à un dysfonctionnement immunitaire (CFIDS), la polymyosite et la dermatomyosite, la cryoglobulinémie, la thrombolyse, la cardiomyopathie, le pemphigus vulgaire, la fibrose interstitielle pulmonaire, le diabète sucré de type I et de type II, l'hypersensibilité retardée de type 1, 2, 3 et 4, les troubles d'allergie ou allergiques, les réponses immunitaires indésirables/accidentelles à des protéines thérapeutiques, l'asthme, le syndrome de Churg-Strauss (granulomatose allergique), la dermatite atopique, la dermatite de contact allergique et irritant, l'urticaire, l'allergie médiée par IgE, l'athérosclérose, la vascularite, les myopathies inflammatoires idiopathiques, la maladie hémolytique, la maladie d'Alzheimer, et la polyneuropathie démyélinisante inflammatoire chronique, l'inflammation pulmonaire y compris, mais sans s'y limiter, le rejet de greffe de poumon, l'asthme, la sarcoïdose, l'emphysème, la mucoviscidose, la fibrose pulmonaire idiopathique, la bronchite chronique, la rhinite allergique et les maladies allergiques du poumon telles que la pneumonite d'hypersensibilité, la pneumonie à éosinophiles, la bronchiolite oblitérante due à la greffe de moelle osseuse et/ou de poumon ou à d'autres causes, l'athérosclérose du greffon/phébosclérose du greffon, ainsi que la fibrose pulmonaire provenant des maladies du collagène, vasculaire et auto-immune telles que la polyarthrite rhumatoïde et le lupus érythémateux.

5. Anticorps selon la revendication 1, utilisation selon la revendication 2 ou méthode selon la revendication 3, **caractérisés en ce que** ladite maladie inflammatoire ou maladie auto-immune est une maladie inflammatoire ou une maladie auto-immune qui est associée à des cellules exprimant aussi bien le CD40 que le CD20.

6. Anticorps, utilisation ou méthode selon la revendication 5, **caractérisés en ce que** ladite maladie inflammatoire ou maladie auto-immune est la polyarthrite rhumatoïde, le psoriasis, le lupus érythémateux disséminé, la maladie de Crohn, la myasthénie grave, le purpura thrombocytopénique idiopathique ou le syndrome de Sjogren.

7. Anticorps, utilisation ou méthode selon la revendication 5, **caractérisés en ce que** ladite maladie inflammatoire ou maladie auto-immune est la sclérose en plaques, le rejet du greffon, la maladie du greffon contre l'hôte, la maladie d'Alzheimer ou le diabète.

8. Anticorps selon la revendication 1, utilisation selon la revendication 2 ou méthode selon la revendication 3, **caractérisés en ce que** ledit anticorps est l'anticorps monoclonal CHIR-12.12 produit par la lignée cellulaire d'hybridome déposée auprès de ATCC sous le n° de dépôt de brevet PTA-5543.

9. Anticorps, utilisation ou méthode selon l'une quelconque des revendications précédentes, **caractérisés en ce que** ledit anticorps anti-CD40 est un anticorps monoclonal humain.

10. Anticorps, utilisation ou méthode selon la revendication 9, **caractérisés en ce que** ledit anticorps monoclonal anti-CD40 humain comprend une région constante de chaîne lourde d'IgG1 humaine.

11. Anticorps, utilisation ou méthode selon la revendication 10, **caractérisés en ce que** ledit anticorps monoclonal comprend la séquence d'acides aminés présentée dans SEQ ID NO:4 ou SEQ ID NO:5.

12. Anticorps, utilisation ou méthode selon l'une quelconque des revendications précédentes, **caractérisés en ce que** ledit anticorps anti-CD40 se lie au CD40 humain avec une affinité (K_{D}) d'au moins environ 10⁻⁶ M, ou d'au moins 10⁻⁸ M.

13. Anticorps, utilisation ou méthode selon l'une quelconque des revendications précédentes, **caractérisés en ce que** ledit anticorps anti-CD40 se lie à FcγRIIIa-158V humain avec une affinité (K_{D}) d'au moins environ 0,5 µM et/ou se lie à FcγRIIIa-158F humain avec une affinité (K_{D}) d'au moins environ 12 µM.

14. Anticorps, utilisation ou méthode selon l'une quelconque des revendications précédentes, **caractérisés en ce que** ledit anticorps monoclonal est un fragment de liaison d'antigène d'un anticorps monoclonal, ledit fragment conservant la capacité de se lier de manière spécifique audit antigène CD40 humain.

15. Fragment de liaison d'antigène selon la revendication 14, **caractérisé en ce que** ledit fragment est choisi dans le groupe constitué par un fragment Fab, un fragment F(ab')₂, un fragment Fv, et un fragment Fv simple chaîne.

16. Anticorps ou utilisation selon la revendication 1 ou la revendication 2, **caractérisés en ce que** ladite utilisation conduit à la cytotoxicité cellulaire anticorps-dépendante (ADCC) antibody dependent cellular cytotoxicity de cellules exprimant le CD40 par les cellules tueuses naturelles (NK) exprimant le FcγRIIIa d'une patient humain.

17. Anticorps, utilisation ou méthode selon l'une quelconque des revendications 1 à 15, **caractérisés en ce que** ledit anticorps anti-CD40 est plus puissant que le rituximab (Rituxan®) dans un essai de cytotoxicité cellulaire anticorps-dépendante (ADCC), ledit essai comprenant l'incubation de cellules exprimant le CD40 et de cellules exprimant le CD20 avec des cellules tueuses naturelles (NK) humaines isolées en présence de l'anticorps pertinent.

18. Anticorps, utilisation ou méthode selon l'une quelconque des revendications 1 à 16, **caractérisés en ce que** ledit anticorps anti-CD40 est plus puissant que le rituximab (Rituxan®) dans un modèle de lupus érythémateux disséminé (SLE), de sclérose en plaques, d'inflammation et d'athérosclérose, de greffe ou de maladie d'Alzheimer.

19. Anticorps, utilisation ou méthode selon l'une quelconque des revendications précédentes, **caractérisés en ce que** ledit patient humain est réfractaire à une thérapie pour une maladie inflammatoire ou auto-immune.

20. Anticorps, utilisation ou méthode selon la revendication 19, **caractérisés en ce que** ledit patient humain est réfractaire à une thérapie par un anticorps monoclonal anti-CD20.

21. Anticorps, utilisation ou méthode selon la revendication 20, **caractérisés en ce que** ledit anticorps monoclonal anti-CD20 est le rituximab (Rituxan®).


### SEQUENCE LISTING

<110> Aukerman, Sharon Lea Luqman, Mohammad
<120> Uses of Anti-CD40 Antibodies
<130> PP028185.0002(319124)
<150> 60/732,580
   <151> 2005-11-01
<160> 9
<170> FastSEQ for Windows Version 4.0
<210> 1
   <211> 720
   <212> DNA
   <213> Artificial Sequence
<220>
   <223> Coding sequence for light chain of 12.12 human anti-CD40 antibody
<221> CDS
   <222> (1)...(720)
<400> 1
   atg gcg ctc cct gct cag ctc ctg ggg ctg cta atg ctc tgg gtc tct    48
   Met Ala Leu Pro Ala Gln Leu Leu Gly Leu Leu Met Leu Trp Val Ser
    1               5                  10                  15
   gga tcc agt ggg gat att gtg atg act cag tct cca ctc tcc ctg acc    96
   Gly Ser Ser Gly Asp Ile Val Met Thr Gln Ser Pro Leu Ser Leu Thr
               20                  25                  30
   gtc acc cct gga gag ccg gcc tcc atc tcc tgc agg tcc agt cag agc    144
   Val Thr Pro Gly Glu Pro Ala Ser Ile Ser Cys Arg Ser Ser Gln Ser
           35                  40                  45
   ctc ctg tat agt aat gga tac aac tat ttg gat tgg tac ctg cag aag    192
   Leu Leu Tyr Ser Asn Gly Tyr Asn Tyr Leu Asp Trp Tyr Leu Gln Lys
       50                  55                  60
   cca ggg cag tct cca cag gtc ctg atc tct ttg ggt tct aat cgg gcc    240
   Pro Gly Gln Ser Pro Gln Val Leu Ile Ser Leu Gly Ser Asn Arg Ala
    65                  70                  75                  80
   tcc ggg gtc cct gac agg ttc agt ggc agt gga tca ggc aca gat ttt    288
   Ser Gly Val Pro Asp Arg Phe Ser Gly Ser Gly Ser Gly Thr Asp Phe
                   85                  90                  95
   aca ctg aaa atc agc aga gtg gag gct gag gat gtt ggg gtt tat tac    336
   Thr Leu Lys Ile Ser Arg Val Glu Ala Glu Asp Val Gly Val Tyr Tyr
               100                 105                 110
   tgc atg caa gct cga caa act cca ttc act ttc ggc cct ggg acc aaa    384
   Cys Met Gln Ala Arg Gln Thr Pro Phe Thr Phe Gly Pro Gly Thr Lys
           115                 120                 125
   gtg gat atc aga cga act gtg gct gca cca tct gtc ttc atc ttc ccg    432
   Val Asp Ile Arg Arg Thr Val Ala Ala Pro Ser Val Phe Ile Phe Pro
       130                 135                 140
   cca tct gat gag cag ttg aaa tct gga act gcc tct gtt gtg tgc ctg    480
   Pro Ser Asp Glu Gln Leu Lys Ser Gly Thr Ala Ser Val Val Cys Leu
   145                 150                 155                 160
   ctg aat aac ttc tat ccc aga gag gcc aaa gta cag tgg aag gtg gat      528
   Leu Asn Asn Phe Tyr Pro Arg Glu Ala Lys Val Gln Trp Lys Val Asp
                   165                 170                 175
   aac gcc ctc caa tcg ggt aac tcc cag gag agt gtc aca gag cag gac      576
   Asn Ala Leu Gln Ser Gly Asn Ser Gln Glu Ser Val Thr Glu Gln Asp
           180                 185                 190
   agc aag gac agc acc tac agc ctc agc agc acc ctg acg ctg agc aaa      624
   Ser Lys Asp Ser Thr Tyr Ser Leu Ser Ser Thr Leu Thr Leu Ser Lys
               195                 200                 205
   gca gac tac gag aaa cac aaa gtc tac gcc tgc gaa gtc acc cat cag      672
   Ala Asp Tyr Glu Lys His Lys Val Tyr Ala Cys Glu Val Thr His Gln
       210                 215                 220
   ggc ctg agc tcg ccc gtc aca aag agc ttc aac agg gga gag tgt tag      720
   Gly Leu Ser Ser Pro Val Thr Lys Ser Phe Asn Arg Gly Glu Cys  *
   225                 230                 235
<210> 2
   <211> 239
   <212> PRT
   <213> Artificial Sequence
<220>
   <223> Light chain of 12.12 human anti-CD40 antibody
<400> 2
   Met Ala Leu Pro Ala Gln Leu Leu Gly Leu Leu Met Leu Trp Val Ser
   1               5                   10                  15
   Gly Ser Ser Gly Asp Ile Val Met Thr Gln Ser Pro Leu Ser Leu Thr
               20                  25                  30
   Val Thr Pro Gly Glu Pro Ala Ser Ile Ser Cys Arg Ser Ser Gln Ser
           35                  40                  45
   Leu Leu Tyr Ser Asn Gly Tyr Asn Tyr Leu Asp Trp Tyr Leu Gln Lys
       50                  55                  60
   Pro Gly Gln Ser Pro Gln Val Leu Ile Ser Leu Gly Ser Asn Arg Ala
   65                  70                  75                  80
   Ser Gly Val Pro Asp Arg Phe Ser Gly Ser Gly Ser Gly Thr Asp Phe
                   85                  90                  95
   Thr Leu Lys Ile Ser Arg Val Glu Ala Glu Asp Val Gly Val Tyr Tyr
               100                 105                 110
   Cys Met Gln Ala Arg Gln Thr Pro Phe Thr Phe Gly Pro Gly Thr Lys
           115                 120                 125
   Val Asp Ile Arg Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe Tyr Pro Arg Glu Ala Lys Val Gln Trp Lys Val Asp
                   165                 170                 175
   Asn Ala Leu Gln Ser Gly Asn Ser Gln Glu Ser Val Thr Glu Gln Asp
               180                 185                 190
   Ser Lys Asp Ser Thr Tyr Ser Leu Ser Ser Thr Leu Thr Leu Ser Lys
           195                 200                 205
   Ala Asp Tyr Glu Lys His Lys Val Tyr Ala Cys Glu Val Thr His Gln
       210                 215                 220
   Gly Leu Ser Ser Pro Val Thr Lys Ser Phe Asn Arg Gly Glu Cys
   225                 230                 235
<210> 3
   <211> 2016
   <212> DNA
   <213> Artificial Sequence
<220>
   <223> Coding sequence for heavy chain of 12.12 human anti-CD40 antibody (with introns)
<400> 3
   atg gag ttt ggg ctg agc tgg gtt ttc ctt gtt gct att tta aga ggt    48
   gtc cag tgt cag gtg cag ttg gtg gag tct ggg gga ggc gtg gtc cag    96
   cct ggg agg tcc ctg aga ctc tcc tgt gca gcc tct gga ttc acc ttc    144
   agt agc tat ggc atg cac tgg gtc cgc cag gct cca ggc aag ggg ctg    192
   gag tgg gtg gca gtt ata tca tat gag gaa agt aat aga tac cat gca    240
   gac tcc gtg aag ggc cga ttc acc atc tcc aga gac aat tcc aag atc    288
   acg ctg tat ctg caa atg aac agc ctc aga act gag gac acg gct gtg    336
   tat tac tgt gcg aga gat ggg ggt ata gca gca cct ggg cct gac tac    384
   tgg ggc cag gga acc ctg gtc acc gtc tcc tca gca agt acc aag ggc    432
   cca tcc gtc ttc ccc ctg gcg ccc gct agc aag agc acc tct ggg ggc    480
   aca gcg gcc ctg ggc tgc ctg gtc aag gac tac ttc ccc gaa ccg gtg    528
   acg gtg tcg tgg aac tca ggc gcc ctg acc agc ggc gtg cac acc ttc    576
   ccg gct gtc cta cag tcc tca gga ctc tac tcc ctc agc agc gtg gtg    624
   acc gtg ccc tcc agc agc ttg ggc acc cag acc tac atc tgc aac gtg    672
   aat cac aag ccc agc aac acc aag gtg gac aag aga gtt ggt gag agg    720
   cca gca cag gga ggg agg gtg tct gct gga agc cag gct cag cgc tcc    768
   tgc ctg gac gca tcc cgg cta tgc agt ccc agt cca ggg cag caa ggc    816
   agg ccc cgt ctg cct ctt cac ccg gag gcc tct gcc cgc ccc act cat    864
   gct cag gga gag ggt ctt ctg gct ttt tcc cca ggc tct ggg cag gca    912
   cag gct agg tgc ccc taa ccc agg ccc tgc aca caa agg ggc agg tgc    960
   tgg gct cag acc tgc caa gag cca tat ccg gga gga ccc tgc ccc tga    1008
   cct aag ccc acc cca aag gcc aaa ctc tcc act ccc tca gct cgg aca    1056
   cct tct ctc ctc cca gat tcc agt aac tcc caa tct tct ctc tgc aga    1104
   gcc caa atc ttg tga caa aac tca cac atg ccc acc gtg ccc agg taa    1152
   gcc agc cca ggc ctc gcc ctc cag ctc aag gcg gga cag gtg ccc tag    1200
   agt agc ctg cat cca ggg aca ggc ccc agc cgg gtg ctg aca cgt cca    1248
   cct cca tct ctt cct cag cac ctg aac tcc tgg ggg gac cgt cag tct    1296
   tcc tct tcc ccc caa aac cca agg aca ccc tca tga tct ccc gga ccc    1344
   ctg agg tca cat gcg tgg tgg tgg acg tga gcc acg aag acc ctg agg    1392
   tca agt tca act ggt acg tgg acg gcg tgg agg tgc ata atg cca aga    1440
   caa agc cgc ggg agg agc agt aca aca gca cgt acc gtg tgg tca gcg    1488
   tcc tca ccg tcc tgc acc agg act ggc tga atg gca agg agt aca agt    1536
   gca agg tct cca aca aag ccc tcc cag ccc cca tcg aga aaa cca tct    1584
   cca aag cca aag gtg gga ccc gtg ggg tgc gag ggc cac atg gac aga    1632
   ggc cgg ctc ggc cca ccc tct gcc ctg aga gtg acc gct gta cca acc    1680
   tct gtc cct aca ggg cag ccc cga gaa cca cag gtg tac acc ctg ccc    1728
   cca tcc cgg gag gag atg acc aag aac cag gtc agc ctg acc tgc ctg    1776
   gtc aaa ggc ttc tat ccc agc gac atc gcc gtg gag tgg gag agc aat    1824
   ggg cag ccg gag aac aac tac aag acc acg cct ccc gtg ctg gac tcc    1872
   gac ggc tcc ttc ttc ctc tat agc aag ctc acc gtg gac aag agc agg    1920
   tgg cag cag ggg aac gtc ttc tca tgc tcc gtg atg cat gag gct ctg    1968
   cac aac cac tac acg cag aag agc ctc tcc ctg tct ccg ggt aaa tga    2016
<210> 4
   <211> 469
   <212> PRT
   <213> Artificial Sequence
<220>
   <223> Heavy chain of 12.12 human anti-CD40 antibody
<400> 4
   Met Glu Phe Gly Leu Ser Trp Val Phe Leu Val Ala Ile Leu Arg Gly
   1 5 10 15
   Val Gln Cys Gln Val Gln Leu Val Glu Ser Gly Gly Gly Val Val Gln
   20 25 30
   Pro Gly Arg Ser Leu Arg Leu Ser Cys Ala Ala Ser Gly Phe Thr Phe
   35 40 45
   Ser Ser Tyr Gly Met His Trp Val Arg Gln Ala Pro Gly Lys Gly Leu
   50 55 60
   Glu Trp Val Ala Val Ile Ser Tyr Glu Glu Ser Asn Arg Tyr His Ala
   65 70 75 80
   Asp Ser Val Lys Gly Arg Phe Thr Ile Ser Arg Asp Asn Ser Lys Ile
   85 90 95
   Thr Leu Tyr Leu Gln Met Asn Ser Leu Arg Thr Glu Asp Thr Ala Val
   100 105 110
   Tyr Tyr Cys Ala Arg Asp Gly Gly Ile Ala Ala Pro Gly Pro Asp Tyr
   115 120 125
   Trp Gly Gln Gly Thr Leu Val Thr Val Ser Ser Ala Ser Thr Lys Gly
   130 135 140
   Pro Ser Val Phe Pro Leu Ala Pro Ala Ser Lys Ser Thr Ser Gly Gly
   145 150 155 160
   Thr Ala Ala Leu Gly Cys Leu Val Lys Asp Tyr Phe Pro Glu Pro Val
   165 170 175
   Thr Val Ser Trp Asn Ser Gly Ala Leu Thr Ser Gly Val His Thr Phe
   180 185 190
   Pro Ala Val Leu Gln Ser Ser Gly Leu Tyr Ser Leu Ser Ser Val Val
   195 200 205
   Thr Val Pro Ser Ser Ser Leu Gly Thr Gln Thr Tyr Ile Cys Asn Val
   210 215 220
   Asn His Lys Pro Ser Asn Thr Lys Val Asp Lys Arg Val Glu Pro Lys
   225 230 235 240
   Ser Cys Asp Lys Thr His Thr Cys Pro Pro Cys Pro Ala Pro Glu Leu
   245 250 255
   Leu Gly Gly Pro Ser Val Phe Leu Phe Pro Pro Lys Pro Lys Asp Thr
   260 265 270
   Leu Met Ile Ser Arg Thr Pro Glu Val Thr Cys Val Val Val Asp Val
   275 280 285
   Ser His Glu Asp Pro Glu Val Lys Phe Asn Trp Tyr Val Asp Gly Val
   290 295 300
   Glu Val His Asn Ala Lys Thr Lys Pro Arg Glu Glu Gln Tyr Asn Ser
   305 310 315 320
   Thr Tyr Arg Val Val Ser Val Leu Thr Val Leu His Gln Asp Trp Leu
   325 330 335
   Asn Gly Lys Glu Tyr Lys Cys Lys Val Ser Asn Lys Ala Leu Pro Ala
   340 345 350
   Pro Ile Glu Lys Thr Ile Ser Lys Ala Lys Gly Gln Pro Arg Glu Pro
   355 360 365
   Gln Val Tyr Thr Leu Pro Pro Ser Arg Glu Glu Met Thr Lys Asn Gln
   370 375 380
   Val Ser Leu Thr Cys Leu Val Lys Gly Phe Tyr Pro Ser Asp Ile Ala
   385 390 395 400
   Val Glu Trp Glu Ser Asn Gly Gln Pro Glu Asn Asn Tyr Lys Thr Thr
   405 410 415
   Pro Pro Val Leu Asp Ser Asp Gly Ser Phe Phe Leu Tyr Ser Lys Leu
   420 425 430
   Thr Val Asp Lys Ser Arg Trp Gln Gln Gly Asn Val Phe Ser Cys Ser
   435 440 445
   Val Met His Glu Ala Leu His Asn His Tyr Thr Gln Lys Ser Leu Ser
   450 455 460
   Leu Ser Pro Gly Lys
   465
<210> 5
   <211> 469
   <212> PRT
   <213> Artificial Sequence
<220>
   <223> Heavy chain of variant of 12.12 human anti-CD40 antibody
<400> 5
   Met Glu Phe Gly Leu Ser Trp Val Phe Leu Val Ala Ile Leu Arg Gly
   1               5                   10                  15
   Val Gln Cys Gln Val Gln Leu Val Glu Ser Gly Gly Gly Val Val Gln
                       20                  25                  30
   Pro Gly Arg Ser Leu Arg Leu Ser Cys Ala Ala Ser Gly Phe Thr Phe
               35                  40                  45
   Ser Ser Tyr Gly Met His Trp Val Arg Gln Ala Pro Gly Lys Gly Leu
           50                  55                  60
   Glu Trp Val Ala Val Ile Ser Tyr Glu Glu Ser Asn Arg Tyr His Ala
   65                  70                  75                  80
   Asp Ser Val Lys Gly Arg Phe Thr Ile Ser Arg Asp Asn Ser Lys Ile
                   85                  90                  95
   Thr Leu Tyr Leu Gln Met Asn Ser Leu Arg Thr Glu Asp Thr Ala Val
               100                 105                 110
   Tyr Tyr Cys Ala Arg Asp Gly Gly Ile Ala Ala Pro Gly Pro Asp Tyr
           115                 120                 125
   Trp Gly Gln Gly Thr Leu Val Thr Val Ser Ser Ala Ser Thr Lys Gly
       130                 135                 140
   Pro Ser Val Phe Pro Leu Ala Pro Ser Ser Lys Ser Thr Ser Gly Gly
   145                 150                 155                 160
   Thr Ala Ala Leu Gly Cys Leu Val Lys Asp Tyr Phe Pro Glu Pro Val
                   165                 170                 175
   Thr Val Ser Trp Asn Ser Gly Ala Leu Thr Ser Gly Val His Thr Phe
               180                 185                 190
   Pro Ala Val Leu Gln Ser Ser Gly Leu Tyr Ser Leu Ser Ser Val Val
           195                 200                 205
   Thr Val Pro Ser Ser Ser Leu Gly Thr Gln Thr Tyr Ile Cys Asn Val
       210                 215                 220
   Asn His Lys Pro Ser Asn Thr Lys Val Asp Lys Arg Val Glu Pro Lys
   225                 230                 235                 240
   Ser Cys Asp Lys Thr His Thr Cys Pro Pro Cys Pro Ala Pro Glu Leu
                   245                 250                 255
   Leu Gly Gly Pro Ser Val Phe Leu Phe Pro Pro Lys Pro Lys Asp Thr
               260                 265                 270
   Leu Met Ile Ser Arg Thr Pro Glu Val Thr Cys Val Val Val Asp Val
           275                 280                 285
   Ser His Glu Asp Pro Glu Val Lys Phe Asn Trp Tyr Val Asp Gly Val
       290                 295                 300
   Glu Val His Asn Ala Lys Thr Lys Pro Arg Glu Glu Gln Tyr Asn Ser
   305                 310                 315                 320
   Thr Tyr Arg Val Val Ser Val Leu Thr Val Leu His Gln Asp Trp Leu
                   325                 330                 335
   Asn Gly Lys Glu Tyr Lys Cys Lys Val Ser Asn Lys Ala Leu Pro Ala
               340                 345                 350
   Pro Ile Glu Lys Thr Ile Ser Lys Ala Lys Gly Gln Pro Arg Glu Pro
           355                 360                 365
   Gln Val Tyr Thr Leu Pro Pro Ser Arg Glu Glu Met Thr Lys Asn Gln
       370                 375                 380
   Val Ser Leu Thr Cys Leu Val Lys Gly Phe Tyr Pro Ser Asp Ile Ala
   385                 390                 395                 400
   Val Glu Trp Glu Ser Asn Gly Gln Pro Glu Asn Asn Tyr Lys Thr Thr
                   405                 410                 415
   Pro Pro Val Leu Asp Ser Asp Gly Ser Phe Phe Leu Tyr Ser Lys Leu
               420                 425                 430
   Thr Val Asp Lys Ser Arg Trp Gln Gln Gly Asn Val Phe Ser Cys Ser
           435                 440                 445
   Val Met His Glu Ala Leu His Asn His Tyr Thr Gln Lys Ser Leu Ser
       450                 455                 460
   Leu Ser Pro Gly Lys
   465
<210> 6
   <211> 612
   <212> DNA
   <213> Homo sapiens
<220>
   <221> CDS
   <222> (1)...(612)
<221> misc_feature
   <222> (0)..(0)
   <223> Coding sequence for short isoform of human CD40
<400> 6
   atg gtt cgt ctg cct ctg cag tgc gtc ctc tgg ggc tgc ttg ctg acc     48
   Met Val Arg Leu Pro Leu Gln Cys Val Leu Trp Gly Cys Leu Leu Thr
    1               5                  10                  15
   gct gtc cat cca gaa cca ccc act gca tgc aga gaa aaa cag tac cta     96
   Ala Val His Pro Glu Pro Pro Thr Ala Cys Arg Glu Lys Gln Tyr Leu
               20                  25                  30
   ata aac agt cag tgc tgt tct ttg tgc cag cca gga cag aaa ctg gtg    144
   Ile Asn Ser Gln Cys Cys Ser Leu Cys Gln Pro Gly Gln Lys Leu Val
           35                  40                  45
   agt gac tgc aca gag ttc act gaa acg gaa tgc ctt cct tgc ggt gaa    192
   Ser Asp Cys Thr Glu Phe Thr Glu Thr Glu Cys Leu Pro Cys Gly Glu
       50                  55                  60
   agc gaa ttc cta gac acc tgg aac aga gag aca cac tgc cac cag cac    240
   Ser Glu Phe Leu Asp Thr Trp Asn Arg Glu Thr His Cys His Gln His
   65                  70                  75                  80
   aaa tac tgc gac ccc aac cta ggg ctt cgg gtc cag cag aag ggc acc    288
   Lys Tyr Cys Asp Pro Asn Leu Gly Leu Arg Val Gln Gln Lys Gly Thr
                   85                  90                  95
   tca gaa aca gac acc atc tgc acc tgt gaa gaa ggc tgg cac tgt acg    336
   Ser Glu Thr Asp Thr Ile Cys Thr Cys Glu Glu Gly Trp His Cys Thr
               100                 105                 110
   agt gag gcc tgt gag agc tgt gtc ctg cac cgc tca tgc tcg ccc ggc    384
   Ser Glu Ala Cys Glu Ser Cys Val Leu His Arg Ser Cys Ser Pro Gly
           115                 120                 125
   ttt ggg gtc aag cag att gct aca ggg gtt tct gat acc atc tgc gag    432
   Phe Gly Val Lys Gln Ile Ala Thr Gly Val Ser Asp Thr Ile Cys Glu
       130                 135                 140
   ccc tgc cca gtc ggc ttc ttc tcc aat gtg tca tct gct ttc gaa aaa    480
   Pro Cys Pro Val Gly Phe Phe Ser Asn Val Ser Ser Ala Phe Glu Lys
   145                 150                 155                 160
   tgt cac cct tgg aca agg tcc cca gga tcg gct gag agc cct ggt ggt    528
   Cys His Pro Trp Thr Arg Ser Pro Gly Ser Ala Glu Ser Pro Gly Gly
                   165                 170                 175
   gat ccc cat cat ctt cgg gat cct gtt tgc cat cct ctt ggt gct ggt    576
   Asp Pro His His Leu Arg Asp Pro Val Cys His Pro Leu Gly Ala Gly
               180                 185                 190
   ctt tat caa aaa ggt ggc caa gaa gcc aac caa taa                    612
   Leu Tyr Gln Lys Gly Gly Gln Glu Ala Asn Gln  *
           195                 200
<210> 7
   <211> 203
   <212> PRT
   <213> Homo sapiens
<400> 7
   Met Val Arg Leu Pro Leu Gln Cys Val Leu Trp Gly Cys Leu Leu Thr
   1 5 10 15
   Ala Val His Pro Glu Pro Pro Thr Ala Cys Arg Glu Lys Gln Tyr Leu
   20 25 30
   Ile Asn Ser Gln Cys Cys Ser Leu Cys Gln Pro Gly Gln Lys Leu Val
   35 40 45
   Ser Asp Cys Thr Glu Phe Thr Glu Thr Glu Cys Leu Pro Cys Gly Glu
   50 55 60
   Ser Glu Phe Leu Asp Thr Trp Asn Arg Glu Thr His Cys His Gln His
   65 70 75 80
   Lys Tyr Cys Asp Pro Asn Leu Gly Leu Arg Val Gln Gln Lys Gly Thr
   85 90 95
   Ser Glu Thr Asp Thr Ile Cys Thr Cys Glu Glu Gly Trp His Cys Thr
   100 105 110
   Ser Glu Ala Cys Glu Ser Cys Val Leu His Arg Ser Cys Ser Pro Gly
   115 120 125
   Phe Gly Val Lys Gln Ile Ala Thr Gly Val Ser Asp Thr Ile Cys Glu
   130 135 140
   Pro Cys Pro Val Gly Phe Phe Ser Asn Val Ser Ser Ala Phe Glu Lys
   145 150 155 160
   Cys His Pro Trp Thr Arg Ser Pro Gly Ser Ala Glu Ser Pro Gly Gly
   165 170 175
   Asp Pro His His Leu Arg Asp Pro Val Cys His Pro Leu Gly Ala Gly
   180 185 190
   Leu Tyr Gln Lys Gly Gly Gln Glu Ala Asn Gln
   195 200
<210> 8
   <211> 834
   <212> DNA
   <213> Homo sapiens
<220>
   <221> CDS
   <222> (1)...(834)
<221> misc_feature
   <222> (0)...(0)
   <223> Coding sequence for long isoform of human CD40
<400> 8
   atg gtt cgt ctg cct ctg cag tgc gtc ctc tgg ggc tgc ttg ctg acc    48
   Met Val Arg Leu Pro Leu Gln Cys Val Leu Trp Gly Cys Leu Leu Thr
    1               5                  10                  15
   gct gtc cat cca gaa cca ccc act gca tgc aga gaa aaa cag tac cta    96
   Ala Val His Pro Glu Pro Pro Thr Ala Cys Arg Glu Lys Gln Tyr Leu
               20                  25                  30
   ata aac agt cag tgc tgt tct ttg tgc cag cca gga cag aaa ctg gtg    144
   Ile Asn Ser Gln Cys Cys Ser Leu Cys Gln Pro Gly Gln Lys Leu Val
           35                  40                  45
   agt gac tgc aca gag ttc act gaa acg gaa tgc ctt cct tgc ggt gaa    192
   Ser Asp Cys Thr Glu Phe Thr Glu Thr Glu Cys Leu Pro Cys Gly Glu
       50                  55                  60
   agc gaa ttc cta gac acc tgg aac aga gag aca cac tgc cac cag cac    240
   Ser Glu Phe Leu Asp Thr Trp Asn Arg Glu Thr His Cys His Gln His
   65                  70                  75                  80
   aaa tac tgc gac ccc aac cta ggg ctt cgg gtc cag cag aag ggc acc    288
   Lys Tyr Cys Asp Pro Asn Leu Gly Leu Arg Val Gln Gln Lys Gly Thr
                   85                  90                  95
   tca gaa aca gac acc atc tgc acc tgt gaa gaa ggc tgg cac tgt acg    336
   Ser Glu Thr Asp Thr Ile Cys Thr Cys Glu Glu Gly Trp His Cys Thr
               100                 105                 110
   agt gag gcc tgt gag agc tgt gtc ctg cac cgc tca tgc tcg ccc ggc    384
   Ser Glu Ala Cys Glu Ser Cys Val Leu His Arg Ser Cys Ser Pro Gly
               115                 120                 125
   ttt ggg gtc aag cag att gct aca ggg gtt tct gat acc atc tgc gag    432
   Phe Gly Val Lys Gln Ile Ala Thr Gly Val Ser Asp Thr Ile Cys Glu
       130                 135                 140
   ccc tgc cca gtc ggc ttc ttc tcc aat gtg tca tct gct ttc gaa aaa    480
   Pro Cys Pro Val Gly Phe Phe Ser Asn Val Ser Ser Ala Phe Glu Lys
   145                 150                 155                 160
   tgt cac cct tgg aca agc tgt gag acc aaa gac ctg gtt gtg caa cag    528
   Cys His Pro Trp Thr Ser Cys Glu Thr Lys Asp Leu Val Val Gln Gln
                   165                 170                 175
   gca ggc aca aac aag act gat gtt gtc tgt ggt ccc cag gat cgg ctg    576
   Ala Gly Thr Asn Lys Thr Asp Val Val Cys Gly Pro Gln Asp Arg Leu
               180                 185                 190
   aga gcc ctg gtg gtg atc ccc atc atc ttc ggg atc ctg ttt gcc atc    624
   Arg Ala Leu Val Val Ile Pro Ile Ile Phe Gly Ile Leu Phe Ala Ile
           195                 200                 205
   ctc ttg gtg ctg gtc ttt atc aaa aag gtg gcc aag aag cca acc aat    672
   Leu Leu Val Leu Val Phe Ile Lys Lys Val Ala Lys Lys Pro Thr Asn
       210                 215                 220
   aag gcc ccc cac ccc aag cag gaa ccc cag gag atc aat ttt ccc gac    720
   Lys Ala Pro His Pro Lys Gln Glu Pro Gln Glu Ile Asn Phe Pro Asp
   225                 230                 235                 240
   gat ctt cct ggc tcc aac act gct gct cca gtg cag gag act tta cat    768
   Asp Leu Pro Gly Ser Asn Thr Ala Ala Pro Val Gln Glu Thr Leu His
                   245                 250                 255
   gga tgc caa ccg gtc acc cag gag gat ggc aaa gag agt cgc atc tca    816
   Gly Cys Gln Pro Val Thr Gln Glu Asp Gly Lys Glu Ser Arg Ile Ser
               260                 265                 270
   gtg cag gag aga cag tga                                            834
   Val Gln Glu Arg Gln  *
           275
<210> 9
   <211> 277
   <212> PRT
   <213> Homo sapiens
<400> 9
   Met Val Arg Leu Pro Leu Gln Cys Val Leu Trp Gly Cys Leu Leu Thr
   1               5                   10                  15
   Ala Val His Pro Glu Pro Pro Thr Ala Cys Arg Glu Lys Gln Tyr Leu
               20                  25                  30
   Ile Asn Ser Gln Cys Cys Ser Leu Cys Gln Pro Gly Gln Lys Leu Val
           35                  40                  45
   Ser Asp Cys Thr Glu Phe Thr Glu Thr Glu Cys Leu Pro Cys Gly Glu
       50                  55                  60
   Ser Glu Phe Leu Asp Thr Trp Asn Arg Glu Thr His Cys His Gln His
   65                  70                  75                  80
   Lys Tyr Cys Asp Pro Asn Leu Gly Leu Arg Val Gln Gln Lys Gly Thr
                   85                  90                  95
   Ser Glu Thr Asp Thr Ile Cys Thr Cys Glu Glu Gly Trp His Cys Thr
               100                 105                 110
   Ser Glu Ala Cys Glu Ser Cys Val Leu His Arg Ser Cys Ser Pro Gly
           115                 120                 125
   Phe Gly Val Lys Gln Ile Ala Thr Gly Val Ser Asp Thr Ile Cys Glu
       130                 135                 140
   Pro Cys Pro Val Gly Phe Phe Ser Asn Val Ser Ser Ala Phe Glu Lys
   145                 150                 155                 160
   Cys His Pro Trp Thr Ser Cys Glu Thr Lys Asp Leu Val Val Gln Gln
                   165                 170                 175
   Ala Gly Thr Asn Lys Thr Asp Val Val Cys Gly Pro Gln Asp Arg Leu
               180                 185                 190
   Arg Ala Leu Val Val Ile Pro Ile Ile Phe Gly Ile Leu Phe Ala Ile
           195                 200                 205
   Leu Leu Val Leu Val Phe Ile Lys Lys Val Ala Lys Lys Pro Thr Asn
       210                 215                 220
   Lys Ala Pro His Pro Lys Gln Glu Pro Gln Glu Ile Asn Phe Pro Asp
   225                 230                 235                 240
   Asp Leu Pro Gly Ser Asn Thr Ala Ala Pro Val Gln Glu Thr Leu His
                   245                 250                 255
   Gly Cys Gln Pro Val Thr Gln Glu Asp Gly Lys Glu Ser Arg Ile Ser
               260                 265                 270
   Val Gln Glu Arg Gln
           275